# EUROPEAN PATENT APPLICATION

(11) **EP 2 269 515 A1**
(43) Date of publication of application: **05.01.2011**
(21) Application number: 10011891.8
(22) Date of filing: 30.05.2003
(51) Int. Cl.: A61B 8/00

(54) **Breast cancer screening with adjunctive ultra-sound mammography**

(30) Priority: 31.05.2002 US 160836; 27.11.2002 US 305661; 27.11.2002 US 305936; 09.01.2003 US 439437 P
(62) Divisional of application: 03734336.5
(71) Applicant: U-Systems, Inc., San Jose, CA 95134 (US); Karssemeijer, Nico, 6573 DZ Beek (NL)
(72) Inventor: Karssemeijer, Nico, 6573 DZ Beek (NL); Wang, Shih-Ping, Los Altos, CA 94022 (US); Rao, Fangyi, San Jose, CA 95120 (US); Chin, Donald, Palo Alto, CA 94303 (US); Zhang, Wei, Union City, CA 94587 (US); Yu, Zengpin, Palo Alto, CA 94306 (US)
(74) Representative: Brinck, David John Borchardt

(57) **Abstract**

An adjunctive ultrasound mammography system and associated methods are described, comprising a scanning apparatus facilitating standardized, repeatable breast ultrasound scans, and an adjunctive ultrasound display apparatus configured for fast, intuitive viewing of adjunctive ultrasound data concurrently with x-ray mammogram information. Thick-slice ultrasound images are displayed near x-ray mammogram images for assistance in interpreting the x-ray mammogram images. Concurrent acquisition and display of vibrational resonance image (VDI) data is described. Computer-aided diagnosis (CAD) algorithms incorporation acoustically-based feature vectors are described. Easy navigation among thick-slice image arrays, enlarged thick-slice images, and planar ultrasound views is described. Thick-slice images inverted prior to display are described. Algorithms for computing thick-slice images from volumetric scan data are described including feature enhancement algorithms, solutions for border-straddling lesions, and neighborhood-dependent integration algorithms for feature emphasis. Overlaying of thick-slice ultrasound images with x-ray mammogram images for facilitating comprehension of breast structures and detecting abnormalities is described.

## Description

### PRIORITY CLAIMS

This application claims the benefit of the filing date of each of the following applications: U.S. Ser. No. 10/160,836, filed May 31, 2002; U.S. Ser. No. 10/305,661, filed November 27, 2002; U.S. Ser. No. 10/305,936, filed November 27, 2002; and U.S. Ser. No. 60/439,437, filed January 9, 2003. Each of the above applications is incorporated by reference herein.

### ADDITIONAL INCORPORATIONS BY REFERENCE

In addition to the above applications, the following applications are incorporated by reference herein: U.S. Ser. No. 60/252,946, filed November 24, 2000; U.S. Ser. No. 60/326,715, filed October 3, 2001; International Application Ser. No. PCT/US01/43237, filed in the U.S. Receiving Office on November 19, 2001 and published on June 6, 2002 as WO2002/0043801; U.S. Ser. No. 60/415,385, filed October 1, 2002; and U.S. Ser. No. 60/429,728, filed November 27,2002.

### FIELD

This patent specification relates to medical imaging systems and processes. In particular, this patent specification relates to the acquisition and display of breast ultrasound information in a manner that complements traditional x-ray mammogram-based breast cancer screening methods.

### BACKGROUND

Breast cancer is the most common cancer among women other than skin cancer, and is the second leading cause of cancer death in women after lung cancer. The American Cancer Society currently estimates that there are about 203,500 new invasive cases of breast cancer per year among women in the United States and 39,600 deaths per year from the disease. Prevention and early diagnosis of breast cancer are of foremost importance. Because early breast cancer does not produce symptoms, the American Cancer Society recommends a screening mammogram and a clinical breast examination every year for women over the age of 40.

X-ray mammography is currently the only imaging method for mass screening of breast cancer. In health maintenance organizations (HMO's) and other medical organizations, specialized x-ray mammography clinics designed for high patient throughput are being increasingly used to screen as many women as possible in a time and cost efficient manner. Numerous studies have shown that early detection saves lives and increases treatment options. Recent declines in breast cancer mortality rates (*e.g*., 39,600 deaths in 2002 versus 41,200 in 2000) have been attributed, in large part, to the regular use of screening x-ray mammography.

Screening x-ray mammography practice in the United States has become largely standardized. For each x-ray mammogram screening of a patient, two standard x-ray mammogram views of each breast are commonly taken: a top (head-to-toe) view ordinarily called the craniocaudal view ("CC"), and a lateral view ordinarily called the mediolateral oblique view ("MLO"). Several efficiencies arise by virtue of this standardization. Importantly, the examinations can be conducted by an x-ray technician instead of a radiologist, with the radiologist later analyzing x-ray mammograms en masse for a large number of patients. An experienced radiologist can achieve a high throughput, *e.g*., on the order of 2 minutes per patient. This is a key advantage in today's cost-conscious health care environments, because additional radiologist time per patient means additional cost per patient. The efficacy of radiological procedures is today measured by the cost in dollars per quality adjusted life year (QALY), with procedures costing more than $100,000 per QALY being neither encouraged nor prescribed.

Other advantages of x-ray mammogram standardization include: the ability to compare and statistically track large numbers of x-ray mammograms taken from different facilities; the ability to track changes in a single patient over time even if the x-ray mammograms are taken at different facilities; the ability of radiologists to gain recursive expertise in analyzing the standard x-ray mammogram views; and the repeatability of results. The standardization of x-ray mammograms also yields benefits in the public health care area, including the ability for the U.S. government to provide a fixed and predictable per-mammogram reimbursement for Medicare patients. Additionally, health maintenance organizations (HMOs) and other medical insurers are provided with predictable outlays for breast cancer screening of their member patients using x-ray mammography.

A well-known shortcoming of x-ray mammography practice, however, is found in the case of dense-breasted women including patients with high content of fibroglandular tissues in their breasts. Because fibroglandular tissues have higher x-ray absorption than the surrounding fatty tissues, portions of breasts with high fibroglandular tissue content are not well penetrated by x-rays and thus the resulting mammograms would contain little or no information in areas where fibroglandular tissues reside. A study by Lehman et. al., entitled "Effect of Age and Breast Density on Screening Mammograms with False-Positive Findings," on 46,340 patients, published in the December 1999 issue of the American Journal of Reoentgenology (AJR), reports that the proportion of dense breasts (summing those with "heterogeneously dense" and "extremely dense" breasts) account for about 52% of women with age range of 35-39, 47% of age range 40-49, 32% of age range 50-59, 24% of age range 60-69, 23% of age range 70 or older, and 36% for all ages. For the estimated 36% of the female population who have dense breasts, this means that at least a portion of the breast area on the x-ray mammogram cannot be scrutinized for lesions by x-ray mammography alone. As a result, lesions camouflaged by dense breast tissue may go undetected.

Indeed, a study by Kolb et. al. on 18,005 consecutive patients, as reported by Jalali, entitled "Sound Combination: Ultrasound Paired With a Mammography Can Improve Cancer Detection for Dense-Breasted Women," published in the March 1999 issue of ADVANCE for Administrators In Radiology and Radiation Oncology, pages 68-70, states that x-ray mammography alone was able to detect only 70 percent of the cancers (56 of the 80 cancers) in 7,202 patients with dense breasts. Kopans, in a paper entitled "Breast cancer screening with ultrasonography," published in Lancet, Volume 357 (1999), pages 2096-2097, estimates that only 68% of the breast cancers of the screening population would be detected by x-ray mammography alone.

The study by Kolb, *supra*, revealed that by performing ultrasound examination, in the hand-held fashion by a physician and in real-time at a rate of 4 to 20 minutes per patient, on the 7,202 women with dense breasts, an additional 24 percent (19 of the 80 cancers) were detected. X-ray mammography alone detected 70 percent (56 of the 80 cancers), while combining x-ray mammography with ultrasound examination 94 percent of the cancers (75 of the 80 cancers) in dense breasts were detected.

Several other studies showing improved early breast cancer detection using independent ultrasound examination are reported in Jackson, "Controversies in Ultrasound Screening," Society of Breast Imaging 5th Postgraduate Course, San Diego, California, pp. 93-95 (May 2001).

A study by Richter et. al. evaluated an "automated ultrasound system" that generated two automatically reconstructed survey images of the breast based on an acquired set of three-dimensional B-mode scans, and was reported in Richter, K. et. al., "Detection of Malignant and Benign Breast Lesions with an Automated US System: Results in 120 Cases," Radiology 205:823-830 (December 1997). An experimental compression system compressed the breast as in mammography while a motor-driven transducer scanned the breast through an upper compression plate. The two survey images that were constructed from these three-dimensional B-mode scans were (i) a maximum intensity projection (MIP) image mapped from the three-dimensional B-mode scans onto a two-dimensional plane parallel to a lower compression plate, and (ii) a velocity map representing an average acoustic velocity in a direction perpendicular to the lower compression plate. For each patient among a set of patients having known malignant lesions, known benign lesions, or neither, four "blinded" radiologists not involved in the examination of the patients separately examined (i) the two survey images derived from the B-mode scans, with optional access to any of the original B-mode images, and (ii) a corresponding set of conventional x-ray mammograms. It was found that the rate of detection for malignant lesions was 100% (39 of 39 lesions) for combined mammography and ultrasound for all of the radiologists, with the condition that each lesion was identified on at least one of the medical images. The authors stated, "For both benign and malignant lesions, our results show that mammography and ultrasound are complementary modalities; as expected, this does not hold for those lesions that were objectively depicted by means of only one of the two modalities." Richter, *supra* at 830.

Despite strong evidence that use of independent ultrasound examination would improve early breast cancer detection and therefore save lives, substantial resistance against such use currently exists in the medical industry and among policymakers. Jackson, for example, in a paper entitled "The current role of ultrasonography in breast imaging," published in Radiologic Clinics of North America, Volume 33 (1995), pages 1161-1170, states, "The use of ultrasound for breast screening may, however, be harmful to patients." A standard textbook for breast imaging by Heywang-Kobrunner, Dershaw and Schreer, entitled: "Diagnostic Breast Imaging", published in 2001 by Thieme, states on page 88: "Only anecdotal evidence suggests that sonographic screening added to mammography may allow detection of additional carcinomas. However, the existing results suggest that the false positive rate (recommendation for biopsy for lesions that are benign) may be unacceptably high with sonography. The examination is also very operator dependent and time consuming. Feasibility of a quality assurance (technique and reporting), which, however, would be indispensable for any type of ultrasound screening, is not established."

Moreover, the Standards of the American College of Radiology specifically recommend against sonography for breast cancer screening. Heywang-Kobrunner et. al., *supra* at p. 88. The following interrelated factors are often cited against widespread use of ultrasound in breast cancer screening: (i) the false negative (missing) rate of independent ultrasound examination is unknown, (ii) the false positive rate of independent ultrasound examination is known to be very high, leading to an increase in unneeded patient callbacks and biopsies, (iii) lack of image acquisition standardization, leading to variability among different operators and radiologists, (iv) the additional time and equipment required to conduct the ultrasound examination, leading to an increase in cost, and (v) most if not all the breast physicians and radiologists are not trained to read screening ultrasound images, which contain features not found in current breast imaging textbooks or taught in current medical school courses, leading to a potential increase in false negative (missing) rate and in the additional radiologist time required to analyze the ultrasound images, and additional training and clinical experience required for the radiologist to properly analyze the ultrasound images.

Current ad hoc techniques for screening ultrasound examination, as reported by Kolb and others, are indeed not amenable to large-scale integration into the current breast cancer screening environment. For example, in the studies cited *supra* in support of breast screening ultrasound, many of the doctors simply performed the entire screening process themselves, scanning the breast with a hand-held ultrasound probe and viewing the ultrasound display monitor in real-time. Because this usually takes 4 to 20 minutes, such real-time analysis would be cost-prohibitive in today's mass screening environment. The ultrasound viewings are conducted independently on the monitor of the ultrasound machine in real-time without referring to any x-ray mammogram information that may exist for the patient. More importantly, if one pictures the breast as a book, the x-ray mammogram is a picture of the whole book with all the pages of the book superimposed on each another, while the ultrasound images each page independently. The ultrasound image contains many detailed features not observable in an x-ray mammogram, and is very different from a x-ray mammogram in appearance. In addition, the x-ray mammogram is fixed in orientation, either in CC or in MLO views, whereas, as reported by Kolb and others, for example when each breast is scanned in the radial and/or anti-radial fashion around the nipple, each ultrasound image has a different orientation and plane. Thus, even if one wants to view the ultrasound image with an x-ray mammogram, very little can be gained from such practice.

The problem of radiologist skill and training is a particularly important problem to overcome for any breast screening ultrasound scheme to gain acceptance. It has been estimated that only a small portion of today's radiologists would have the ability to effectively use today's ad hoc ultrasound techniques in a mass-screening environment without unacceptable increases in false positives or false negatives.

Vibrational Doppler imaging (VDI) and vibrational resonance techniques, such as those discussed in U.S. Pats. 5,919,139 and 6,068,597 have been proposed for analyzing suspect tumors. As discussed in Lowers, J., "Experimental Modes Abound For Detecting Breast Cancer: Vibrational Resonance Technique Among the Contenders," Women's Health Supplement to Diagnostic Imaging (April 2001) at pp. 15-17, an audio speaker is attached to the ultrasound probe to introduce audio-range vibrational tones (e.g., 69-247 Hz) into the patient during the acquisition of power Doppler ultrasound frames. Different tissue types often vibrate by different amounts responsive to the acoustic signals, and the different vibrations result in different power Doppler readings. Generally speaking, many types of suspect lesions tend to vibrate less than the surrounding breast tissue. It has been found that the absence of vibrations as compared to surrounding tissue can help to clearly differentiate suspect lesions, even those that appear isoechoic (*i*.*e*., less noticeable) with surrounding tissue on B-scan ultrasound frames. In some clinical practice, the patient is asked to produce her own acoustic vibration by humming at audio frequencies. This practice is called "fremitus." Unless otherwise specified herein, VDI refers generally to color or power Doppler images derived from a breast as it is vibrated at one or more audio frequencies, while vibrational resonance refers to VDI data taken at one or more particular sets of audio frequencies.

In view of the above discussions, it would be desirable to provide an adjunctive ultrasound mammography system that integrates ultrasound mammography into current breast cancer screening methodologies.

It would be further desirable to provide an adjunctive ultrasound mammography system in which the benefits of the many years of professional expertise developed in relation to current x-ray mammography, either the analog or the digital, methods are maintained.

It would be further desirable to provide an adjunctive ultrasound mammography system that takes little or no special familiarization or training from the radiologist in order to effectively view ultrasound information in combination with the x-ray mammogram information.

It would be further desirable to provide an adjunctive ultrasound mammography system in which technicians or assistants may perform the ultrasound scans for later en masse analysis by a physician, the physician's presence not being required during the scanning procedure.

It would be even further desirable to provide an adjunctive ultrasound mammography system in which per-patient image analysis time is not substantially increased as compared to x-ray mammogram analysis alone, or which may even reduce per-patient image analysis time.

It would be still further desirable to provide an adjunctive ultrasound mammography image acquisition system that assures standardization of techniques and minimizes operator variability.

It would be even further desirable to provide an adjunctive ultrasound mammography system that is easy to use, comfortable to the patient, and provides standardized and repeatable ultrasonic scans.

It would be still further desirable to provide an adjunctive ultrasound mammography system that is amenable to two and three dimensional computer-assisted diagnosis (CAD) techniques and to provide several such CAD techniques.

It would be still further desirable to provide an adjunctive ultrasound mammography system that is amenable to combined CAD analysis of ultrasound information with x-ray mammogram information for an enhanced CAD system and to provide several such CAD techniques.

It would be even further desirable to provide an adjunctive ultrasound mammography system for which, upon acquisition of the system, any increase in breast cancer screening costs is offset by savings brought about by an increased early breast cancer detection rate, whereby cost per patient QALY is ultimately reduced. ;;eol;;

Breast cancer is the most common cancer among women other than skin cancer, and is the second leading cause of cancer death in women after lung cancer. The American Cancer Society currently estimates that there are about 203,500 new invasive cases of breast cancer per year among women in the United States and 39,600 deaths per year from the disease. Prevention and early diagnosis of breast cancer are of foremost importance. Because early breast cancer does not produce symptoms, the American Cancer Society recommends a screening mammogram and a clinical breast examination every year for women over the age of 40.

X-ray mammography is currently the only imaging method for mass screening of breast cancer. In health maintenance organizations (HMOs) and other medical organizations, specialized x-ray mammography clinics designed for high patient throughput are being increasingly used to screen as many women as possible in a time and cost efficient manner. Numerous studies have shown that early detection saves lives and increases treatment options. Recent declines in breast cancer mortality rates (*e.g*., 39,600 deaths in 2002 versus 41,200 in 2000) have been attributed, in large part, to the regular use of screening x-ray mammography.

It has been found that the use of ultrasound mammography (sonomammography) in conjunction with conventional x-ray mammography can drastically increase the early breast cancer detection rate. Whereas x-ray mammograms only detect a summation of the x-ray opacity of individual slices over the entire breast, ultrasound can separately detect the acoustic impedance of individual slices of breast tissue, and therefore may allow detection of breast lesions where x-ray mammography alone fails.

However, as discussed in Ser. No. 10/160,836, *supra,* despite strong evidence that use of independent ultrasound examination would improve early breast cancer detection and therefore save lives, substantial resistance against such use currently exists in the medical industry, including the radiologists themselves, and among policymakers. As used herein, the term "radiologist" generically refers to a medical professional that analyzes medical images and makes clinical determinations therefrom, it being understood that such person might be titled differently, or might have differing qualifications, depending on the country or locality of their particular medical environment. Several interrelated factors are often cited, including: (i) the false negative (missing) rate of independent ultrasound examination is unknown, (ii) the false positive rate of independent ultrasound examination is known to be very high, leading to an increase in unneeded patient callbacks and biopsies, (iii) lack of image acquisition standardization, leading to variability among different operators and radiologists, (iv) the additional time and equipment required to conduct the ultrasound examination, leading to an increase in cost, (v) most if not all radiologists are not trained to read screening ultrasound images, which contain features not found in current breast imaging textbooks or taught in current medical school courses, leading to a potential increase in false negative (missing) rate and in the additional radiologist time required to analyze the ultrasound images, and (vi) the additional training and clinical experience that would be required for the radiologist to properly analyze the ultrasound images.

Various schemes have been proposed for processing and presenting breast ultrasound information in conjunction with x-ray mammogram information for use in breast cancer detection environments. In U.S. Pat. 5,938,613, which is incorporated by reference herein, a method and apparatus for performing sonomammography and enhanced x-ray imaging is discussed in which ultrasound equipment is integrated with mammography equipment to generate ultrasonic images of the breast that are in geometric registration with an x-ray mammogram. An x-ray mammogram image of an immobilized breast is acquired and, while the breast is still immobilized, an ultrasound scan is acquired using an automated ultrasound probe translation mechanism. Cross-sectional ultrasonic slices are summed across the entire breast to form a two-dimensional ultrasound image, which is then overlaid onto the digitized x-ray image for viewing by the radiologist. Precise geometric registration between the ultrasound image and the x-ray mammogram is automatically provided because the breast is immobilized between imaging procedures and because the coordinates of the ultrasound probe are known during each scan. The radiologist is permitted to instantiate certain algorithms such as digital subtraction between the registered medical images.

However, the '613 patent is deficient in several respects with respect to the practical, real-world factors associated with the current resistance against the use of ultrasound in mass breast cancer screening environments. For example, the large base of currently installed x-ray imaging systems would require substantial retooling to accommodate the mechanical apparatus of the '613 patent that keeps the breast immobilized between imaging procedures and that performs the automated ultrasound scans. As another example, by displaying a summation ultrasound image of all breast slices together, the '613 method deprives the radiologist of the ability to view individual planes inside the breast. More generally, the computer-registered, static overlay of the summation ultrasound image onto the x-ray image affords only a limited amount of ultrasonic information to the radiologist as compared to the actual amount of ultrasonic data actually acquired, and affords only limited perception by the radiologist of structures within the breast.

In U.S. Pat. 5,662,109, a method and system for multi-dimensional imaging and analysis for early detection of diseased tissue is discussed. Ultrasound scans of a breast are processed into multiple layers of two-dimensional images, thus yielding a three-dimensional data set. This data set and a two-dimensional x-ray mammogram are input to an enhancer that performs one or more "data fusion" algorithms to generate a three-dimensional representation of the breast for viewing. The enhancer includes a registration module that expands and/or reduces dimensions of the data to register and align the ultrasound and mammographic images.

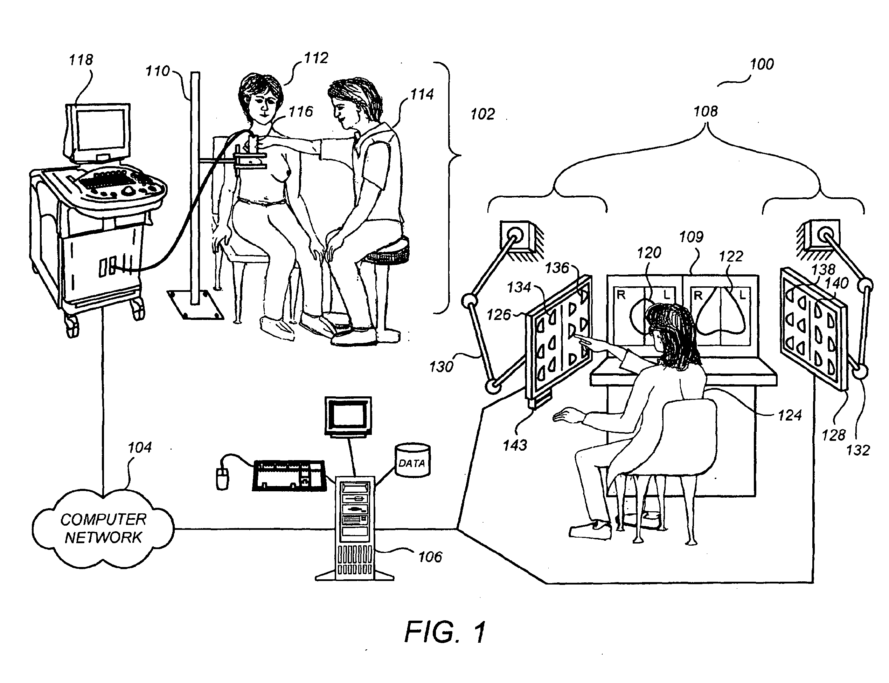

However, it is not believed that the various three-dimensional views of the "fused" data discussed in the '109 patent, such as the perspective view shown in FIG. 1 thereof, would be useful to a typical radiologist trained in conventional x-ray mammography methods. As described *supra,* radiologists typically spend many years developing expertise in analyzing a very particular set of two-dimensional x-ray mammographic data taken from standardized views, most commonly the craniocaudal (CC) and mediolateral oblique (MLO) views. It is believed that most radiologists would be reluctant to "start over again" with an entirely new, different way of viewing the complex structures of a breast, and that the medical industry would likewise be reluctant to force radiologists to accept these viewing methods.

In view of the above discussions, it would be desirable to provide an adjunctive ultrasound mammography system that integrates ultrasound mammography into current breast cancer screening methodologies.

It would be further desirable to provide an adjunctive ultrasound mammography system that displays breast ultrasound information in a manner that facilitates the radiologist's perception of internal breast structures that may not be readily apparent in an x-ray mammogram, while also being able to confirm the radiologist's perception of internal breast structures that are apparent in the x-ray mammogram.

It would be even further desirable to provide an adjunctive ultrasound mammography system that displays breast ultrasound information in a manner that supplements, rather than replaces, conventional x-ray mammogram viewing methods, thereby increasing the likelihood of adoption by both individual radiologists and the medical industry.

It would be even further desirable to provide an adjunctive ultrasound mammography system that takes little or no special familiarization or training from the radiologist in order to effectively view breast ultrasound information.

It would be still further desirable to provide an interactive user interface for an adjunctive ultrasound mammography system that allows the radiologist to quickly and intuitively navigate among different representations of the breast ultrasound information.

It would be even further desirable to display such breast ultrasound information in a manner that allows benign features to be more easily dismissed by the viewing radiologist.

Breast cancer is the most common cancer among women other than skin cancer, and is the second leading cause of cancer death in women after lung cancer. The American Cancer Society currently estimates that there are about 203,500 new invasive cases of breast cancer per year among women in the United States and 39,600 deaths per year from the disease. Prevention and early diagnosis of breast cancer are of foremost importance. Because early breast cancer does not produce symptoms, the American Cancer Society recommends a screening mammogram and a clinical breast examination every year for women over the age of 40.

X-ray mammography is currently the only imaging method for mass screening of breast cancer. In health maintenance organizations (HMOs) and other medical organizations, specialized x-ray mammography clinics designed for high patient throughput are being increasingly used to screen as many women as possible in a time and cost efficient manner. Numerous studies have shown that early detection saves lives and increases treatment options. Recent declines in breast cancer mortality rates (*e.g*., 39,600 deaths in 2002 versus 41,200 in 2000) have been attributed, in large part, to the regular use of screening x-ray mammography.

It has been found that the use of ultrasound mammography (sonomammography) in conjunction with conventional x-ray mammography can drastically increase the early breast cancer detection rate. Whereas x-ray mammograms only detect a summation of the x-ray opacity of individual slices over the entire breast, ultrasound can separately detect the acoustic impedance of individual slices of breast tissue, and therefore may allow detection of breast lesions where x-ray mammography alone fails.

However, as discussed in Ser. No. 10/160,836, *supra*, despite strong evidence that use of independent ultrasound examination would improve early breast cancer detection and therefore save lives, substantial resistance against such use currently exists in the medical industry, including the radiologists themselves, and among policymakers. As used herein, the term "radiologist" generically refers to a medical professional that analyzes medical images and makes clinical determinations therefrom, it being understood that such person might be titled differently, or might have differing qualifications, depending on the country or locality of their particular medical environment. Several interrelated factors are often cited, including: (i) the false negative (missing) rate of independent ultrasound examination is unknown, (ii) the false positive rate of independent ultrasound examination is known to be very high, leading to an increase in unneeded patient callbacks and biopsies, (iii) lack of image acquisition standardization, leading to variability among different operators and radiologists, (iv) the additional time and equipment required to conduct the ultrasound examination, leading to an increase in cost, (v) most if not all radiologists are not trained to read screening ultrasound images, which contain features not found in current breast imaging textbooks or taught in current medical school courses, leading to a potential increase in false negative (missing) rate and in the additional radiologist time required to analyze the ultrasound images, and (vi) the additional training and clinical experience that would be required for the radiologist to properly analyze the ultrasound images.

Various schemes have been proposed for processing and presenting breast ultrasound information in conjunction with x-ray mammogram information for use in breast cancer detection environments. In U.S. Pat. 5,938,613, which is incorporated by reference herein, a method and apparatus for performing sonomammography and enhanced x-ray imaging is discussed in which ultrasound equipment is integrated with mammography equipment to generate ultrasonic images of the breast that are in geometric registration with an x-ray mammogram. An x-ray mammogram image of an immobilized breast is acquired and, while the breast is still immobilized, an ultrasound scan is acquired using an automated ultrasound probe translation mechanism. Cross-sectional ultrasonic slices are summed across the entire breast to form a two-dimensional ultrasound image, which is then overlaid onto the digitized x-ray image for viewing by the radiologist. Precise geometric registration between the ultrasound image and the x-ray mammogram is automatically provided because the breast is immobilized between imaging procedures and because the coordinates of the ultrasound probe are known during each scan. The radiologist is permitted to instantiate certain algorithms such as digital subtraction between the registered medical images.

However, the '613 patent is deficient in several respects with respect to the practical, real-world factors associated with the current resistance against the use of ultrasound in mass breast cancer screening environments. For example, the large base of currently installed x-ray imaging systems would require substantial retooling to accommodate the mechanical apparatus of the '613 patent that keeps the breast immobilized between imaging procedures and that performs the automated ultrasound scans. As another example, by displaying a summation ultrasound image of all breast slices together, the '613 method deprives the radiologist of the ability to view individual planes inside the breast. More generally, the computer-registered, static overlay of the summation ultrasound image onto the x-ray image affords only a limited amount of ultrasonic information to the radiologist as compared to the actual amount of ultrasonic data actually acquired, and affords only limited perception by the radiologist of structures within the breast.

In U.S. Pat. 5,662,109, a method and system for multi-dimensional imaging and analysis for early detection of diseased tissue is discussed. Ultrasound scans of a breast are processed into multiple layers of two-dimensional images, thus yielding a three-dimensional data set. This data set and a two-dimensional x-ray mammogram are input to an enhancer that performs one or more "data fusion" algorithms to generate a three-dimensional representation of the breast for viewing. The enhancer includes a registration module that expands and/or reduces dimensions of the data to register and align the ultrasound and mammographic images.

However, it is not believed that the various three-dimensional views of the "fused" data discussed in the '109 patent, such as the perspective view shown in FIG. 1 thereof, would be useful to a typical radiologist trained in conventional x-ray mammography methods. As described *supra*, radiologists typically spend many years developing expertise in analyzing a very particular set of two-dimensional x-ray mammographic data taken from standardized views, most commonly the craniocaudal (CC) and mediolateral oblique (MLO) views. It is believed that most radiologists would be reluctant to "start over again" with an entirely new, different way of viewing the complex structures of a breast, and that the medical industry would likewise be reluctant to force radiologists to accept these viewing methods.

In view of the above discussions, it would be desirable to provide an adjunctive ultrasound mammography system that integrates ultrasound mammography into current breast cancer screening methodologies.

It would be further desirable to provide an adjunctive ultrasound mammography system that displays breast ultrasound information in a manner that facilitates the radiologist's perception of internal breast structures that may not be readily apparent in an x-ray mammogram, while also being able to confirm the radiologist's perception of internal breast structures that are apparent in the x-ray mammogram.

It would be even further desirable to provide an adjunctive ultrasound mammography system that displays breast ultrasound information in a manner that supplements, rather than replaces, conventional x-ray mammogram viewing methods, thereby increasing the likelihood of adoption by both individual radiologists and the medical industry.

It would be even further desirable to provide an adjunctive ultrasound mammography system that takes little or no special familiarization or training from the radiologist in order to effectively view breast ultrasound information.

It would be still further desirable to provide an interactive user interface for an adjunctive ultrasound mammography system that allows the radiologist to quickly and intuitively navigate among different representations of the breast ultrasound information.

It would be even further desirable to display such breast ultrasound information in a manner that allows benign features to be more easily dismissed by the viewing radiologist.

Breast cancer is the most common cancer among women other than skin cancer, and is the second leading cause of cancer death in women after lung cancer. The American Cancer Society currently estimates that there are about 203,500 new invasive cases of breast cancer per year among women in the United States and 39,600 deaths per year from the disease. Prevention and early diagnosis of breast cancer are of foremost importance. Because early breast cancer does not produce symptoms, the American Cancer Society recommends a screening mammogram and a clinical breast examination every year for women over the age of 40.

X-ray mammography is currently the only imaging method for mass screening of breast cancer. In health maintenance organizations (HMOs) and other medical organizations, specialized x-ray mammography clinics designed for high patient throughput are being increasingly used to screen as many women as possible in a time and cost efficient manner. Numerous studies have shown that early detection saves lives and increases treatment options. Recent declines in breast cancer mortality rates (*e.g*., 39,600 deaths in 2002 versus 41,200 in 2000) have been attributed, in large part, to the regular use of screening x-ray mammography.

It has been found that the use of ultrasound mammography (sonomammography) in conjunction with conventional x-ray mammography can drastically increase the early breast cancer detection rate. Whereas x-ray mammograms only detect a summation of the x-ray opacity of individual slices over the entire breast, ultrasound can separately detect the acoustic impedance of individual slices of breast tissue, and therefore may allow detection of breast lesions where x-ray mammography alone fails.

However, as discussed in Ser. No. 10/160,836, *supra*, despite strong evidence that use of independent ultrasound examination would improve early breast cancer detection and therefore save lives, substantial resistance against such use currently exists in the medical industry, including the radiologists themselves, and among policymakers. As used herein, the term "radiologist" generically refers to a medical professional that analyzes medical images and makes clinical determinations therefrom, it being understood that such person might be titled differently, or might have differing qualifications, depending on the country or locality of their particular medical environment. Several interrelated factors are often cited, including: (i) the false negative (missing) rate of independent ultrasound examination is unknown, (ii) the false positive rate of independent ultrasound examination is known to be very high, leading to an increase in unneeded patient callbacks and biopsies, (iii) lack of image acquisition standardization, leading to variability among different operators and radiologists, (iv) the additional time and equipment required to conduct the ultrasound examination, leading to an increase in cost, (v) most if not all radiologists are not trained to read screening ultrasound images, which contain features not found in current breast imaging textbooks or taught in current medical school courses, leading to a potential increase in false negative (missing) rate and in the additional radiologist time required to analyze the ultrasound images, and (vi) the additional training and clinical experience that would be required for the radiologist to properly analyze the ultrasound images.

Various schemes have been proposed for processing and presenting breast ultrasound information in conjunction with x-ray mammogram information for use in breast cancer detection environments. In U.S. Pat. 5,938,613, which is incorporated by reference herein, a method and apparatus for performing sonomammography and enhanced x-ray imaging is discussed in which ultrasound equipment is integrated with mammography equipment to generate ultrasonic images of the breast that are in geometric registration with an x-ray mammogram. An x-ray mammogram image of an immobilized breast is acquired and, while the breast is still immobilized, an ultrasound scan is acquired using an automated ultrasound probe translation mechanism. Cross-sectional ultrasonic slices are summed across the entire breast to form a two-dimensional ultrasound image, which is then overlaid onto the digitized x-ray image for viewing by the radiologist. Precise geometric registration between the ultrasound image and the x-ray mammogram is automatically provided because the breast is immobilized between imaging procedures and because the coordinates of the ultrasound probe are known during each scan. The radiologist is permitted to instantiate certain algorithms such as digital subtraction between the registered medical images.

However, the '613 patent is deficient in several respects with respect to the practical, real-world factors associated with the current resistance against the use of ultrasound in mass breast cancer screening environments. For example, the large base of currently installed x-ray imaging systems would require substantial retooling to accommodate the mechanical apparatus of the '613 patent that keeps the breast immobilized between imaging procedures and that performs the automated ultrasound scans. As another example, by displaying a summation ultrasound image of all breast slices together, the '613 method deprives the radiologist of the ability to view individual planes inside the breast. More generally, the computer-registered, static overlay of the summation ultrasound image onto the x-ray image affords only a limited amount of ultrasonic information to the radiologist as compared to the actual amount of ultrasonic data actually acquired, and affords only limited perception by the radiologist of structures within the breast.

In U.S. Pat. 5,662,109, a method and system for multi-dimensional imaging and analysis for early detection of diseased tissue is discussed. Ultrasound scans of a breast are processed into multiple layers of two-dimensional images, thus yielding a three-dimensional data set. This data set and a two-dimensional x-ray mammogram are input to an enhancer that performs one or more "data fusion" algorithms to generate a three-dimensional representation of the breast for viewing. The enhancer includes a registration module that expands and/or reduces dimensions of the data to register and align the ultrasound and mammographic images.

However, it is not believed that the various three-dimensional views of the "fused" data discussed in the '109 patent, such as the perspective view shown in FIG. 1 thereof, would be useful to a typical radiologist trained in conventional x-ray mammography methods. As described *supra*, radiologists typically spend many years developing expertise in analyzing a very particular set of two-dimensional x-ray mammographic data taken from standardized views, most commonly the craniocaudal (CC) and mediolateral oblique (MLO) views. It is believed that most radiologists would be reluctant to "start over again" with an entirely new, different way of viewing the complex structures of a breast, and that the medical industry would likewise be reluctant to force radiologists to accept these viewing methods.

In view of the above discussions, it would be desirable to provide an adjunctive ultrasound mammography system that integrates ultrasound mammography into current breast cancer screening methodologies.

It would be further desirable to provide an adjunctive ultrasound mammography system that displays breast ultrasound information in a manner that facilitates the radiologist's perception of internal breast structures that may not be readily apparent in an x-ray mammogram, while also being able to confirm the radiologist's perception of internal breast structures that are apparent in the x-ray mammogram.

It would be even further desirable to provide an adjunctive ultrasound mammography system that displays breast ultrasound information in a manner that supplements, rather than replaces, conventional x-ray mammogram viewing methods, thereby increasing the likelihood of adoption by both individual radiologists and the medical industry.

It would be even further desirable to provide an adjunctive ultrasound mammography system that takes little or no special familiarization or training from the radiologist in order to effectively view breast ultrasound information.

### SUMMARY

An adjunctive ultrasound mammography system and associated methods are provided, comprising a scanning apparatus for facilitating standardized, repeatable breast ultrasound scans, and further comprising an adjunctive ultrasound display apparatus configured for fast, intuitive viewing of ultrasound information concurrently with x-ray mammogram information. Many of the practical barriers to widespread integration of ultrasound mammography into existing mass breast cancer screening environments are mitigated. Additionally, many of the medical community acceptance barriers, economic barriers, and other political barriers to widespread integration of ultrasound mammography into existing mass breast cancer screening environments are mitigated.

The scanning apparatus of the preferred adjunctive ultrasound mammography system is configured to yield ultrasound slices from successive planes in a breast volume substantially parallel to a plane of a predetermined x-ray mammogram view of the breast. The scanning apparatus supports and maintains the breast during the ultrasound scan in a manner that promotes volumetric thoroughness of the scan, with the resulting ultrasound slices extending substantially all the way to the chest wall. The scanning apparatus is capable of partially flattening the breast according to a desired x-ray mammogram view plane while also maintaining patient comfort. Efficient patient throughput is facilitated, while at the same time the risk of inter-patient contamination and fomite propagation is minimized.

In one preferred embodiment, the scanning apparatus comprises a reservoir containing an acoustically conductive fluid for immersion of the breast therein, the scanning apparatus further comprising an ultrasound probe immersed within or positioned under the reservoir such that gap-free acoustic communication with the breast is established. A mechanical translation mechanism moves the ultrasound probe across the breast as ultrasound scans are taken, yielding a set of raw ultrasound slices. Preferably, the scanning apparatus further comprises two compression plates positioned on opposite sides of the breast, the compression plates being oriented parallel to a standard x-ray mammogram view plane such that information obtained from the raw ultrasound slices corresponds more closely to a standard x-ray mammogram view.

Preferably, the compression plates of the scanning apparatus are coupled to audio frequency transducers for accommodating a vibrational Doppler imaging (VDI) modality. As the ultrasound probe is moved across the breast, successive B-mode ultrasound slices are acquired at spatial intervals corresponding to a desired resolution, the audio frequency transducers being silent during these B-mode scans. However, at regular spatial intervals, acquisition of B-mode slices is temporarily suspended, the audio frequency transducers are activated, and VDI imaging information is obtained.

According to another preferred embodiment, the scanning apparatus comprises first and second compressive members that sandwich the breast along a plane that is near a standard x-ray mammogram view plane. The first compressive member is movable with respect to the second compressive member to allow entry of the breast therebetween, and preferably comprises a conformable sheet of acoustically transparent material in a taut state. An inner surface of the first compressive member compresses the breast while an outer surface accommodates an ultrasound probe that scans the compressed breast. Preferably, the first compressive member forms a first shallow angle with respect to the standard x-ray mammogram view plane such that interrogating ultrasonic waves from the ultrasound probe can penetrate through to the chest wall. This enhances image quality near the chest wall while still providing an overall mammogram-like view of the breast. Preferably, a nipple support element is provided on the second compressive member that urges the nipple into acoustic communication with the first compressive member. This enhances ultrasonic imaging of the breast nipple, which is a meaningful reference point in comparing the ultrasound mammography results to the x-ray mammography results. The second compressive member may comprise a substantially rigid surface and/or may comprise an air bag, a fluid bag, or a preformed sponge-like material designed to promote patient comfort while also providing a sufficient degree of breast compression.

According to a preferred embodiment, the adjunctive ultrasound display apparatus provides an array of thick-slice thumbnail images, each thick-slice thumbnail image comprising information integrated from a plurality of adjacent ultrasound slices and representing a thick-slice or slab-like portion of the breast volume substantially parallel to the standard x-ray mammogram view. The adjunctive ultrasound display apparatus comprises one or more adjunct display monitors positioned near a conventional x-ray mammogram display such that a screening radiologist can quickly turn their attention to the thick-slice thumbnail images to clarify questionable portions of the x-ray mammogram. In one preferred embodiment, each thick-slice thumbnail image is positioned not more than twenty inches from its corresponding x-ray mammogram view. Whereas the x-ray mammogram only shows the overall sum of breast tissue densities, the ultrasound thickslice thumbnail images permit a quick view of individual thick-slice portions of the breast tissue. This allows the radiologist, for example, to quickly investigate whether a suspicious-looking mass in the x-ray mammogram is truly a tumor or is simply a coincidental confluence of patterns from different breast planes.

According to a preferred embodiment, an intuitive, interactive user interface is provided that allows the radiologist to easily manipulate and examine the adjunctive ultrasound data in a manner that facilitates rapid screening. In one preferred embodiment, upon selection of a given thick-slice thumbnail image, a corresponding thick-slice image is expanded to a full-scale representation on the display that is comparable in size to the x-ray mammogram being viewed. In another preferred embodiment, the radiologist may examine each individual ultrasound slice used to form the thick-slice image. In another preferred embodiment, the radiologist may quickly jog through the individual ultrasound slices or may view a cine-loop presentation of them. In another preferred embodiment, side-by-side views of the individual ultrasound slices that form the thick-slice image are displayed. Advantageously, the individual ultrasound slices are of immediate and familiar significance to the radiologist because they correspond in orientation to standard x-ray mammogram views. In another preferred embodiment, the radiologist may directly view the raw, unprocessed ultrasound data from the ultrasound scans.

By way of analogy, the patient's breast may be thought of as a "book." An x-ray mammogram is a picture of the book with all of its pages clumped together. In contrast, each ultrasound slice is a picture of one page of the book. On the one hand, the x-ray mammogram alone often does not contain enough information, but on the other hand, separately screening each ultrasound slice would be both time-prohibitive and cost-prohibitive in today's mass screening environment. According to a preferred embodiment, if the x-ray mammogram alone is not sufficient, the radiologist may quickly glance at a simple array of thick-slice thumbnail images, which are analogous to "chapters" of the book. The radiologist only examines an individual page of the book if it lies within an interesting chapter. In this manner, the thoroughness afforded by volumetric ultrasound scans can be enjoyed in today's mass breast cancer screening environments without being time-prohibitive or cost-prohibitive.

According to another preferred embodiment, each thick-slice image is derived from B-mode ultrasound data, and vibrational Doppler imaging (VDI) data corresponding to each thick-slice image is superimposed thereon. The VDI data is superimposed only for those locations having at least a threshold amount of induced vibration resulting from the injected audio-frequency energy. For each such location, the B-mode data is displayed in ordinary fashion such as black-and-white, while the VDI data is superimposed thereon according to a color map that maps different amounts of induced vibration into different colors, lower amounts displayed in red, for example, up through higher amounts displayed in violet. In another preferred embodiment, the VDI data is displayed alone or in a separate image alongside the B-mode image. In another preferred embodiment, the radiologist may toggle among B-mode only, VDI only, superimposed B-mode/VDI, and side-by-side B-mode/VDI displays. For each of these options, the image may be expanded to a full-scale view comparable in size to the x-ray mammogram image, and may be jogged-through and/or cine-looped according to the desires of the radiologist.

According to a preferred embodiment, a method for mass breast cancer screening of a plurality of patients using adjunctive ultrasound mammography is provided comprising the steps of acquiring an x-ray mammogram for each of said plurality of patients, acquiring raw breast ultrasound scans for each of said plurality of patients, processing the raw breast ultrasound scans into adjunctive ultrasound data including viewable thick-slice images, and storing the adjunctive ultrasound data on an adjunctive ultrasound server. In one preferred embodiment in which the raw ultrasound slices are acquired in planes parallel or-nearly parallel to the desired standard x-ray mammogram view plane, the thick-slice images are directly computed from an integration of the raw ultrasound slices. In another preferred embodiment in which the raw ultrasound slices are acquired in planes perpendicular or substantially non-parallel to the desired standard x-ray mammogram view plane, the thick-slice images are indirectly computed by construction of a three-dimensional volumetric representation of the breast and extraction of the thick-slice images therefrom.

The method further comprises the steps of, for each patient, associating the adjunctive ultrasound data with the corresponding x-ray mammogram for that patient. In one preferred embodiment, an alphanumeric identifier (e.g., bar code) of the x-ray mammogram is directly assigned to the adjunctive ultrasound data. The method further comprises the steps of performing *en masse* screening of the x-ray mammograms for the plurality of patients. For each x-ray mammogram presented to the radiologist, the alphanumeric identifier thereof is used to retrieve the associated adjunctive ultrasound data from the adjunctive ultrasound server to facilitate screening of that x-ray mammogram. According to another preferred embodiment, the adjunctive ultrasound data is assigned a fixed patient ID for that patient and a date stamp. During *en masse* screening, a patient ID from the x-ray mammogram is used to query the adjunctive ultrasound server, which returns the most recent set of adjunctive ultrasound data for that patient ID.

Advantageously, because they generally exhibit improved signal-to-noise ratios as compared to their component individual ultrasound slices, the thick-slice images are more conducive to the use of two-dimensional computer-assisted diagnosis (CAD) algorithms to assist the radiologist. According to a preferred embodiment, two-dimensional CAD algorithms are applied to the thick-slice images similar to a manner in which conventional two-dimensional CAD algorithms are applied to digitized x-ray mammograms. Results from the two-dimensional CAD algorithms are superimposed on the thick-slice image display in a manner that highlights the location of possible tumors and their degree of suspiciousness. The preferred two-dimensional CAD algorithm comprises the steps of (i) determining region-of-interest (ROI) locations in the thick-slice image according to a two-dimensional ROI detection algorithm, (ii) segmenting the borders of candidate lesions at each ROI location, (iii) for each candidate lesion, extracting a first set of two-dimensional features, the first set of two-dimensional features being selected from known x-ray CAD methods, and (iv) applying a classifier algorithm to the first set of two-dimensional features to determine one or more metrics of suspiciousness therefrom. Examples of such known two-dimensional features include spiculation metrics, density metrics, eccentricity metrics, and sphericity metrics. In one preferred embodiment, a single scalar metric of suspiciousness, termed a score, is generated for each candidate lesion.

According to another preferred embodiment, for each candidate lesion a second set of two-dimensional features is extracted in addition to the first set of two-dimensional features, the second set of two-dimensional features being directly associated with acoustical characteristics of the breast. The second set of two-dimensional features may include any combination of (i) a lateral shadow metric, (ii) a VDI metric, (iii) a vertical shadow metric, and (iv) a posterior enhancement metric. For a given candidate lesion on a given thick-slice image, the lateral shadow metric relates to an amount of acoustic shadow cast by that lesion across that thick-slice image, and becomes relevant where the interrogating ultrasound beam is substantially parallel to that particular thick-slice portion of the breast volume. The vertical shadow metric relates to an amount of acoustic shadow cast by that lesion across other thick-slice images, and becomes relevant where the ultrasound probe is substantially non-parallel to the thick slices. In one preferred embodiment, the VDI metric is simply an average magnitude of measured velocity in a neighborhood of the lesion, wherein a lesser VDI metric indicates higher suspiciousness. The classifier algorithm then operates on both the first set and second set of two-dimensional features to determine a score, or other metric of lesion suspiciousness, for that candidate lesion.

According to another preferred embodiment, in conjunction with the determination of two-dimensional features from the thick-slice images, three-dimensional features are extracted from a three-dimensional thick-slice volume corresponding to each thick-slice image. In one preferred embodiment, the three-dimensional features are determined by (i) determining region-of-interest (ROI) locations in the thick-slice volume, (ii) segmenting the borders of candidate lesions at each ROI location, and (iii) extracting a set of three-dimensional features for each candidate lesion. Preferably, the ROI location step takes advantage of the known two-dimensional ROI locations computed for the thickslice images, for example, by using them as starting points in locating the three-dimensional ROI locations within the thick-slice volume. This can save computing time by reducing the ROI search to a one-dimensional search for each known two-dimensional ROI location. In an alternative preferred embodiment, a purely three-dimensional ROI location algorithm is used to locate the ROIs in the thick-slice volume. In one preferred embodiment, the three-dimensional features comprise a surface roughness metric for the candidate lesions, such as a surface area-to-volume ratio. The classifier algorithm then operates on the set of three-dimensional features, together with the first and second sets of two-dimensional features, to determine a score, or other metric of lesion suspiciousness, for each candidate lesion. The classifier algorithm may incorporate any of a variety of classification algorithms known in the art, including linear classifier algorithms, quadratic classifier algorithms, K-nearest-neighbor classifier algorithms, decision tree classifier algorithms, or neural network classifier algorithms.

According to another preferred embodiment, three-dimensional CAD algorithms are performed on a volumetric representation of the breast without regard to thick-slice boundaries, including the steps of the steps of (i) determining ROI locations in the breast volume according to a three-dimensional ROI detection algorithm, (ii) segmenting the borders of candidate lesions at each ROI location, (iii) for each candidate lesion, extracting three-dimensional features, and (iv) applying a classifier algorithm to the three-dimensional features to determine one or more metrics of suspiciousness therefrom. The locations of lesions found to require user attention are mapped from the breast volume into their corresponding thick-slice image, and a marker is placed on the two-dimensional thick-slice image on the user display. The marker may also be placed on the relevant individual ultrasound slices on the user display. The three-dimensional features may include three-dimensional spiculation metrics, three-dimensional density metrics, sphericity metrics, VDI metrics, shadow metrics, surface area-to-volume metrics, and other three-dimensional metrics.

According to another preferred embodiment, a computer-assisted microcalcification-highlighting algorithm is provided for assisting viewer perception of microcalcifications on the thick-slice images and/or individual ultrasound slices. The image is first thresholded on a pixel-by-pixel basis using a predetermined threshold value selected to separate the microcalcifications from the dense breast tissue and other breast tissue in a statistically reliable manner. For those pixels lying above the predetermined threshold, simple region-growing algorithms are performed in which neighboring above-threshold pixels are clustered together. An overlay display comprising those clusters having an average diameter less than a predetermined size, such as about 1 mm, are overlaid in a bright color on the otherwise black-and-white ultrasound image.

According to another preferred embodiment, a breast cancer screening CAD system is provided that performs a first set of CAD algorithms on a digitized x-ray mammogram view of the breast, performs a second set of CAD algorithms on a corresponding set of adjunctive ultrasound views, correlates regions of interest (ROIs) between the x-ray mammogram view and the adjunctive ultrasound views, and performs joint classification of the ROI using both the x-ray CAD results and the ultrasound CAD results. During the ROI correlation process, ROIs in the x-ray mammogram are matched to corresponding ROIs in the adjunctive ultrasound views in a manner that obviates the need for complex registration computations. Rather, a simplified but statistically reliable lesion-centric correlation process using nipple distance information, or using a combination of nipple distance information and nipple angle information, is used to match corresponding ROIs in the x-ray mammogram view and the adjunctive ultrasound views. In another preferred embodiment, the correlation process also uses lesion size as a factor in matching corresponding regions of interest in the x-ray mammogram view and the adjunctive ultrasound views. In still another preferred embodiment, the correlation process uses lesion distance from the chest wall as a factor in matching corresponding regions of interest in the x-ray mammogram view and the adjunctive ultrasound views. In one preferred embodiment, the joint classification algorithm comprises a direct addition of scalar suspiciousness metrics taken from the x-ray CAD results and the ultrasound CAD results.

An adjunctive ultrasound mammography system and associated methods are provided including an intuitive, interactive user interface for displaying breast ultrasound information to a user. According to a preferred embodiment, an array of thick-slice images derived from volumetric ultrasound scans of a breast is displayed, each thick-slice image representing a thick-slice or slab-like region of the breast volume substantially parallel to a standard x-ray mammogram view of the breast. Responsive to a first single-click or single-movement user selection of a first point on one of the thick-slice images, an enlarged view of that thick-slice image is displayed with a cursor positioned at a corresponding point. Responsive to a second single-movement user selection of a second point on the enlarged view, a first planar ultrasound image encompassing the second point is displayed, the first planar ultrasound image representing the volumetric ultrasound scans along a first plane substantially nonparallel to, and preferably perpendicular to, the orientation of the slab-like region for that thick-slice image.

According to another preferred embodiment, a second planar ultrasound image is shown concurrently with the first planar ultrasound image representing the volumetric scans along a second plane substantially orthogonal to both the first plane and to the orientation of the slab-like region. According to another preferred embodiment the first and second planar ultrasound images are displayed concurrently with the enlarged thickslice image or the array of thick-slice images. The first and second planes correspond to the current cursor position on an active one of the thick-slice images and are updated in real time as the cursor is moved. Range markers are provided on the planar ultrasound images corresponding to the current cursor position and to the borders of the slab-like region for the active thick-slice image.

According to another preferred embodiment, first and second plane indicators are displayed on the active thick-slice image, the plane indicators corresponding to the first and second planes and appearing as straight lines for a default configuration in which the first and second planes are orthogonal to each other and to the orientation of the slab-like region for the active thick-slice image. In the default configuration, the first and second plane indicators intersect the cursor on the active thick-slice image. The user is permitted to depart from the,default configuration if desired by moving the first and second plane indicators in a manner analogous to the way lines are moved in a computer-aided drawing system, while the first and second planes and the first and second planar ultrasound images are updated in real time to correspond to the orientations and locations of the first and second plane indicators.

A user interface according to the preferred embodiments is preferably provided in conjunction with an x-ray mammogram viewer such that the array of thick-slice ultrasound images is displayed in coordination with a corresponding x-ray mammogram image taken from the same standard x-ray mammogram view. The x-ray mammogram image, which is preferably provided on a backlighted film display but which can alternatively be provided on an electronic display, is displayed in close proximity to the array of thick-slice ultrasound images to allow easy back-and-forth viewing. Preferably, the thick-slice images are displayed at full scale on an LCD monitor positioned directly below the x-ray mammogram images, while the first and second planar ultrasound images are displayed on smaller CRT displays positioned to the sides of the LCD monitor. However, a variety of different configurations having differing advantages are within the scope of the preferred embodiments as described further *infra.*

According to one preferred embodiment, the displayed thick-slice images are inverted to represent high acoustic reflections as "dark" and low acoustic reflections as "bright," in distinction to a standard ultrasound display convention in which low acoustic reflections are displayed as "dark" and high acoustic reflections are displayed as "bright." Preferably, the breast area is digitally segmented from the surrounding area, and the surrounding area is reset to "dark" prior to display of the inverted thick-slice image. The inverted thick-slice images are of more familiar significance to radiologists having years of expertise in analyzing conventional x-ray mammograms. For example, the inverted thickslice images allow benign features to be more easily dismissed as compared to non-inverted thick-slice images..

According to another preferred embodiment, a method for computing the thickslice images from the volumetric ultrasound representation of the breast is provided, each thick-slice image pixel being computed based on the statistics of a voxel column passing through that location from a lower border to an upper border of the relevant slab-like region. In particular, the statistical properties of interest are ones that incur changes across different pixel locations in mass localities that are more significant for masses greater than a preselected size of interest and that are less significant for smaller masses. Accordingly, mass lesions greater than the preselected size of interest are emphasized while smaller mass lesions are de-emphasized in the resulting thick-slice image. In one preferred embodiment, the thick-slice pixel value is selected as that value for which a cumulative distribution function (CDF) of the voxel column becomes equal to a threshold value, the threshold value being a predetermined fraction of a ratio of the preselected size of interest to the distance between the first and second border planes. A method for ensuring the visibility of lesions straddling the borders between adjacent thick-slice regions is also provided in which (i) an actual result for the actual thick-slice region is computed, (ii) a hypothetical result is computed for a hypothetical thick-slice region that is partially elevated into the adjacent thick-slice region, and (iii) resetting the actual result to the hypothetical result if the hypothetical result is more indicative of lesser ultrasound reflections.

An adjunctive ultrasound mammography system and associated methods are provided including an adjunctive ultrasound display system configured to allow flexible, intuitive, and interactive viewing of breast ultrasound information in a manner that complements x-ray mammogram viewing. An ultrasound image of a breast is displayed near an x-ray mammogram image of the breast, the adjunctive ultrasound display system allowing for superposition of the ultrasound image over the x-ray mammogram image, or vice-versa, for facilitating rapid comprehension of breast structures and detection of abnormalities therein. In one preferred embodiment, the x-ray mammogram image corresponds to a standard x-ray mammogram view, and the ultrasound image is a thickslice image representing a thick-slice or slab-like portion of the breast volume substantially parallel to that standard x-ray mammogram view. In another preferred embodiment, the user is permitted to perform manual vernier adjustments of the registration of the ultrasound image with the x-ray mammogram image.

Advantageously, thick-slice ultrasound images corresponding to standard x-ray mammogram view planes are of immediate and familiar significance to the radiologist, both as stand-alone images and as components of bimodal ultrasound/x-ray images. Moreover, it has been found that the manual vernier registration adjustment process itself, in which the radiologist shifts the relative positions of the component images responsive to perceived registration differences, can rapidly increase the radiologist's perception and appreciation of the breast structures being displayed by both component images. Even though ultrasound images tend to have substantially different textures and feature emphases than x-ray images, the resulting bimodal image, alone or in combination with the manual vernier registration adjustment process, can often expose or clarify tissue structures that may be hidden or less apparent in the separate x-ray mammogram and/or ultrasound images, and can often obviate or explain certain noticed structures in the separate x-ray mammogram and/or ultrasound images.

Preferably, an array of thick-slice images is displayed to the radiologist representing different thick-slice portions of the breast, and the radiologist can manually superimpose any one of them over the x-ray mammogram image, or can manually superimpose the x-ray mammogram image over any one of them. In one preferred embodiment, both the x-ray mammogram image and the ultrasound image are displayed on the same screen of a high-resolution monitor, and a pixelwise digital mixing algorithm is used to achieve image superposition. Preferably, a mixing algorithm is selected that approximates the visual effect of (i) placing a conventional x-ray mammogram film on a light box, and (ii) superimposing a second transparency thereon containing a printed version of the thick-slice ultrasound image. However, the incorporation of any of a variety of digital mixing algorithms is within the scope of the preferred embodiments, including those that permit dynamic adjustment of one or more mixing parameters by the radiologist, and including both pixelwise and neighborhood-based mixing algorithms.

In other preferred embodiments, the component medical images are presented in any of a variety of physical configurations that permit the user to overlay them to form a bimodal image and to perform fine registration adjustments. The component medical images may exist on x-ray film, as lightbox backprojections, on high-brightness computer monitors, on transparent or opaque hardcopies, on subtractive liquid crystal displays, and/or on other types of image displays, and in different combinations thereof, provided that image superposition is possible. Any of a variety of mechanisms may be used to physically move/overlay the medical images and to provide manual vernier adjustment capability, ranging from hand manipulation of hardcopy images to computerized click-and-drag techniques.

In one preferred embodiment in which the ultrasound image is displayed in electronic format, user inputs are provided for allowing dynamic adjustment of (i) the thickness of the thick-slice image, *i.e*., the thickness of the slab-like region of the breast that is integrated into a single two-dimensional thick-slice image, and (ii) thick-slice image elevation, *i.e*., the vertical elevation of the slab-like region within the breast volume. In one preferred embodiment, the positioning of the overlying image onto the underlying image is an entirely manual process, the radiologist manually performing both (i) preliminary or coarse registration, *i*.*e*., moving the images from their initial positions onto each other, and (ii) vernier registration, *i.e*., perfecting the registration of the images. In an alternative preferred embodiment, preliminary registration is provided automatically by the display system using any of a variety of known methods, such that the radiologist only needs to perform the vernier registration step.

Preferably, in an adjunctive ultrasound system using ultrasound overlays according to a preferred embodiment, the ultrasound image information is provided to the radiologist on a supplementary, as-needed basis, without interfering with the radiologist's primary task of analyzing x-ray mammograms. This is believed to be advantageous from a strategic medical-community acceptance viewpoint, because entrenched radiologists will not be "forced" to use the ultrasound information. Once called upon, however, it is expected that the convenient, easy-to-use, intuitive ultrasound information viewing system according to the preferred embodiments will attract many radiologists to its use in everyday mass breast cancer screening activities.

An adjunctive ultrasound mammography system and associated methods are provided in which an array of thick-slice images derived from volumetric ultrasound scans of a breast is displayed, each thick-slice image representing a thick-slice or slab-like region of the breast volume substantially parallel to a standard x-ray mammogram view of the breast. Each thick-slice image pixel represents an integration of values along a voxel column of a three-dimensional array containing the volumetric ultrasound scan readings for the associated thick-slice volume. According to a preferred embodiment, each thickslice image is generated using a neighborhood-dependent integration algorithm. The neighborhood-dependent integration algorithm varies its methods and/or parameters according to neighborhood characteristics of the three-dimensional array around the voxel column corresponding to the associated thick-slice image pixel. The neighborhood-dependent integration algorithm is designed to result in thick-slice images that emphasize mass lesions of a predetermined size range. Thick-slice images generated using the preferred neighborhood-dependent integration algorithms result in easier spotting of mass lesions as compared to thick-slice images generated by spatially-invariant or voxel-column-specific integration algorithms.

### BRIEF DESCRIPTION OF THE DRAWINGS

FIG. 1 illustrates a conceptual diagram of a system and method for breast cancer screening using adjunctive ultrasound mammography according to a preferred embodiment;

FIG. 2 illustrates a perspective view of an ultrasound scanning apparatus according to a preferred embodiment;

FIG. 3 illustrates a conceptual perspective view of a reservoir portion of an ultrasound scanning apparatus according to a preferred embodiment;

FIG. 4 illustrates a conceptual side view of a reservoir portion of an ultrasound scanning apparatus according to a preferred embodiment;

FIG. 5 illustrates a perspective view of an ultrasound scanning apparatus according to a preferred embodiment;

FIG. 6 illustrates side views of an ultrasound scanning apparatus according to a preferred embodiment in different patient positions;

FIG. 7 illustrates a simplified perspective view of an ultrasound scanning apparatus according to a preferred embodiment;

FIG. 8A illustrates a side view of a breast compressed within the ultrasound scanning apparatus of FIG. 7;

FIG. 8B illustrates a simplified perspective view of a portion of an ultrasound scanning apparatus according to a preferred embodiment;

FIG. 8C illustrates a side view of a breast compressed within the ultrasound scanning apparatus of FIG. 8B;

FIG. 8D illustrates a conceptual top view of an ultrasound scanning apparatus according to a preferred embodiment that facilitates an arcuate scanning trajectory;

.

FIG. 9A illustrates a conceptual view of thick-slice portions of a breast as mapped upon the breast itself and as appears on an adjunct ultrasound display according to a preferred embodiment;

FIG. 9B illustrates an adjunct ultrasound display according to a preferred embodiment corresponding to an arcuate ultrasound scanning trajectory;

FIG. 10A illustrates an adjunct ultrasound display according to a preferred embodiment;

FIG. 10B illustrates an adjunct ultrasound display according to a preferred embodiment corresponding to an arcuate ultrasound scanning trajectory;

FIG. 11 illustrates a control panel of an adjunct ultrasound display according to a preferred embodiment;

FIG. 12 illustrates an adjunct ultrasound display according to a preferred embodiment, along with an explanatory conceptual view of a breast with a cancerous tumor and a cyst;

FIG. 13 illustrates an adjunct ultrasound display according to a preferred embodiment;

FIG. 14 illustrates steps for mass breast cancer screening using adjunctive ultrasound mammography according to a preferred embodiment;

FIG. 15 illustrates steps for mass breast cancer screening using adjunctive ultrasound mammography according to a preferred embodiment;

FIG. 16 illustrates steps for computer-aided diagnosis of adjunctive ultrasound data according to a preferred embodiment;

FIGS. 17A and 17B illustrate an adjunct ultrasound display according to a preferred embodiment in which a lesion casts lateral shadows across a thick-slice image, along with explanatory conceptual views of interrogating ultrasonic waves impinging upon the breast;

FIG. 18 illustrates an adjunct ultrasound display according to a preferred embodiment in which a lesion casts a vertical shadow across other thick-slice images, along with an explanatory conceptual view of a breast with a suspect lesion;

FIG. 19 illustrates steps for computer-aided diagnosis of adjunctive ultrasound data according to a preferred embodiment;

FIG. 20A illustrates steps for computer-aided diagnosis of adjunctive ultrasound data according to a preferred embodiment;

FIG. 20B illustrates steps for computer-aided diagnosis of adjunctive ultrasound data according to a preferred embodiment;

FIGS. 21-24 illustrates x-ray mammogram viewing stations with adjunctive ultrasound displays according to the preferred embodiments;

FIG. 25A illustrates an adjunct ultrasound display according to a preferred embodiment showing thumbnail thick slice views;

FIG. 25B illustrates an adjunct ultrasound display according to a preferred embodiment showing a single thick slice image;

FIG. 25C illustrates an adjunct ultrasound display according to a preferred embodiment showing a paused cine presentation of individual ultrasound slices corresponding to the thick slice image of FIG. 25B;

FIG. 25D illustrates an adjunct ultrasound display according to a preferred embodiment showing a paused cine presentation of raw B-mode ultrasound slices corresponding to the ultrasound data of FIGS. 25A-25C;

FIG. 25E illustrates an adjunct ultrasound display according to a preferred embodiment showing thumbnail thick slice views with superimposed CAD markers;

FIG. 25F illustrates an adjunct ultrasound display according to a preferred embodiment showing a single thick slice image with superimposed CAD markers;

FIG. 26 illustrates a plot of a conventional contrast enhancing remapping curve;

FIGS. 27-31 illustrate conceptual diagrams of thick-slice regions as superimposed onto a front view of a breast;

FIG. 32 illustrates steps for joint computer-aided diagnosis of digitized x-ray mammogram data and adjunctive ultrasound data according to a preferred embodiment;

FIG. 33 illustrates a conceptual diagram of a digitized x-ray mammogram and regions of interest therein;

FIG. 34 illustrates a conceptual diagram of an adjunctive ultrasound view and regions of interest therein;

FIG. 35 illustrates a plot of nipple distance in an x-ray mammogram view versus nipple distance in an adjunctive ultrasound view for registering regions of interest between the x-ray mammogram view and the adjunctive ultrasound view;

FIG. 36 illustrates a plot of nipple angle in an x-ray mammogram view versus nipple angle in an adjunctive ultrasound view for registering regions of interest between the x-ray mammogram view and the adjunctive ultrasound view;

FIG. 37 illustrates a conceptual diagram of a system and method for breast cancer screening using adjunctive ultrasound mammography according to a preferred embodiment;

FIG. 38 illustrates steps for breast cancer screening using adjunctive ultrasound mammography according to a preferred embodiment;

FIG. 39 illustrates steps for interactively displaying adjunctive ultrasound mammography information to a user according to a preferred embodiment;

FIG. 40 illustrates an adjunct ultrasound display according to a preferred embodiment presenting an array of inverted thick-slice images;

FIG. 41 illustrates an adjunct ultrasound display according to a preferred embodiment presenting an enlarged inverted thick-slice image;

FIG. 42 illustrates an adjunct ultrasound display according to a preferred embodiment presenting a planar ultrasound image;

FIG. 43 illustrates an adjunct ultrasound display according to a preferred embodiment presenting an array of non-inverted thick-slice images for the same breast illustrated in FIG. 40;

FIG. 44 illustrates an adjunct ultrasound display according to a preferred embodiment presenting an array of inverted but non-segmented thick-slice images for the same breast as FIGS. 40 and 43;

FIG. 45 illustrates an adjunct ultrasound display according to a preferred embodiment presenting an array of inverted thick-slice images;

FIG. 46 illustrates an adjunct ultrasound display according to a preferred embodiment presenting an enlarged inverted thick-slice image;

FIG. 47 illustrates an adjunct ultrasound display according to a preferred embodiment presenting a raw ultrasound image;

FIG. 48A illustrates a conceptual view of a thick-slice region and lesions contained therein, along with related histograms and cumulative distribution functions;

FIG. 48B illustrates a conceptual view of a thick-slice region and lesions contained therein, along with plots of thick-slice image values along a single line in the thick-slice image resulting from thick-slice image computation algorithms according to preferred embodiments;

FIG. 49 illustrates a conceptual frontal view of a set of neighboring thick-slice region and lesions contained therein, along with conceptual thick-slice images;

FIG. 50 illustrates steps for interactively displaying adjunctive ultrasound mammography information to a user according to a preferred embodiment;

FIG. 51 illustrates an adjunct ultrasound display according to a preferred embodiment presenting an array of inverted thick-slice images and two planar ultrasound images;

FIG. 52 illustrates the adjunct ultrasound display of FIG. 51 when a user moves the cursor on a selected thick-slice image;

FIG. 53 illustrates an adjunct ultrasound display according to a preferred embodiment comprising an enlarged inverted thick-slice image and two planar ultrasound images;

FIG. 54 illustrates an exterior view of an adjunctive ultrasound mammography display unit according to a preferred embodiment;

FIG. 55 illustrates a keypad for the adjunctive ultrasound mammography display unit of FIG. 54;

FIG. 56 illustrates a closer view of display portions of the adjunctive ultrasound mammography display unit of FIG. 54;

FIG. 57 illustrates a conceptual diagram of a system and method for breast cancer screening using adjunctive ultrasound mammography according to a preferred embodiment;

FIG. 58 illustrates a conceptual diagram of a first medical image of a breast;

FIG. 59 illustrates a conceptual diagram of a second medical image of a breast;

FIG. 60 illustrates a conceptual diagram of a bimodal medical image formed from the medical images of FIGS. 58 and 59;

FIG. 61 illustrates an adjunct ultrasound display at different intervals during an overlay of an ultrasound image onto an x-ray mammogram image;

FIG. 62 illustrates steps corresponding to a method for breast cancer screening using an x-ray mammogram with adjunct ultrasound overlays according to a preferred embodiment;

FIG. 63 illustrates a conceptual diagram of varying thick-slice elevation and thick-slice thickness using a dynamic control mechanism according to a preferred embodiment;

FIG. 64 illustrates an adjunct ultrasound display according to a preferred embodiment displaying an ultrasound thick-slice image;

FIG. 65 illustrates an adjunct ultrasound display according to a preferred embodiment displaying a remapped version of the ultrasound thick-slice image of FIG. 64;

FIG. 66 illustrates an adjunct ultrasound display according to a preferred embodiment displaying a digital x-ray mammogram adjacent to the ultrasound thick-slice image of FIG. 65;

FIG. 67 illustrates an adjunct ultrasound display according to a preferred embodiment displaying the digital x-ray mammogram of FIGS. 65-66 as it is moved toward the ultrasound thick-slice image of FIGS. 65-66 for mixing therewith;

FIG. 68 illustrates an adjunct ultrasound display according to a preferred embodiment displaying a bimodal image of a digital x-ray mammogram superimposed upon an ultrasound thick-slice image and in approximate registration therewith;

FIG. 69 illustrates an adjunct ultrasound display according to a preferred embodiment displaying the bimodal image of FIG. 68 as adjusted to allow its digital x-ray mammogram component to predominate over the ultrasound thick-slice image component;

FIG. 70 illustrates an adjunct ultrasound display according to a preferred embodiment displaying the bimodal image of FIG. 68 as adjusted to allow its ultrasound thick-slice image component to predominate over its digital x-ray mammogram component.

FIG. 71 illustrates a conceptual diagram of a system and associated methods for breast cancer screening using adjunctive ultrasound mammography;

FIG. 72 illustrates a conceptual diagram of a three-dimensional array comprising ultrasound scan values for a particular thick-slice volume within a breast substantially parallel to the CC view plane, and a corresponding thick-slice image computed therefrom;

FIG. 73 illustrates a conceptual example of a single thick-slice image along with planar ultrasound images;

FIG. 74 illustrates a user display according to a preferred embodiment;

FIG. 75 conceptually illustrates the dependence of a weighting vector on neighborhood characteristics around a voxel column; and

FIG. 76 illustrates a cut-away side view of a scanning apparatus during a breast scan.

### DETAILED DESCRIPTION

FIG. 1 illustrates a conceptual diagram of a system 100 and associated methods for breast cancer screening using adjunctive ultrasound mammography according to a preferred embodiment. Adjunctive ultrasound mammography refers to the acquisition and display of breast ultrasound information during the breast cancer screening process in a manner that supplements x-ray mammogram information. System 100 comprises an ultrasound scanning station 102, a computer network 104, an adjunctive ultrasound server 106, and an adjunctive ultrasound screening station 108. Ultrasound scanning station 102 comprises an ultrasound scanning apparatus 110 for facilitating breast ultrasound scans of the patient 112 by an ultrasound technician 114. An ultrasound probe 116 is used to scan a breast of the patient 112, with reflected acoustic interrogation signals being processed by an ultrasound machine 118.

The ultrasound scanning apparatus 110 supports and maintains the breast during the ultrasound scanning process. According to a preferred embodiment, the ultrasound scanning apparatus 110 also flattens the breast along a plane parallel to a standard x-ray mammogram view plane such that resulting ultrasound images correspond more closely to standard x-ray mammogram images. In the example of FIG. 1, the standard x-ray mammogram view is the craniocaudal (CC) view. While described herein with respect to the CC view for simplicity and clarity of explanation, it is to be appreciated that the preferred embodiments are readily applied to the mediolateral oblique (MLO) view or to standard or custom x-ray mammogram views.

Although not shown in FIG. 1, the patient 112 also undergoes a standard x-ray mammography procedure in addition to the ultrasound mammography procedure. The x-ray mammogram is usually taken during the same office visit as the ultrasonic mammography scans, although the scope of the preferred embodiments is not so limited. The ultrasound technician 114 may be the same person or a different person as the x-ray technician who performs the x-ray mammography procedure.

If the ultrasound probe 116 is manipulated by hand, as in the embodiment of FIG. 1, a position sensing system (not shown) is used to track the probe position such that the acquired ultrasound frames may be processed into a three-dimensional volumetric representation of the breast. It is generally preferable, however, that the ultrasound probe 116 be machine-manipulated and controlled so as to provide reliable, consistent ultrasound scans. The ultrasound scans should be of sufficient resolution and taken at small enough intervals such that the three-dimensional volumetric representation has sufficient resolution to enable computer-aided diagnosis (CAD) algorithms to perform effectively, and such that both individual ultrasound slices and thick-slice images are of sufficient resolution to enable meaningful screening assistance to the radiologist.

As will be described further infra, the raw ultrasound scans may be taken directly in the standard x-ray mammogram view plane, or may alternatively be taken from a different orientation. When the raw ultrasound scans are taken directly in the standard x-ray mammogram view plane, each individual ultrasound slice is computed directly from an acquired two-dimensional ultrasound image or ultrasound frame. When the raw ultrasound scans are taken from a different orientation, each individual ultrasound slice corresponds to a plane of voxels (volume elements) in a three-dimensional volumetric representation of the breast, the plane of voxels being oriented in a direction parallel to the standard x-ray mammogram view plane. Most commonly, the three-dimensional volumetric representation of the breast is computed from the raw ultrasound scans, and then the individual ultrasound slice is extracted therefrom. However, in other preferred embodiments such as those described in Ser. No. 60/326,715, supra, it is not always necessary to reconstruct the entire three-dimensional volumetric representation to compute the individual ultrasound slices. Stated more generally, if the raw ultrasound scans are taken in planes directly parallel to a plane of interest (CC, MLO, or a different "custom" plane of importance), each individual ultrasound slice is computed directly from an acquired two-dimensional ultrasound image or ultrasound frame, whereas if the raw ultrasound scans are taken from directions different than the plane of interest, each individual ultrasound slice corresponds to a plane of voxels in a three-dimensional volumetric representation of the breast in a direction parallel to the plane of interest.

Ultrasound machine 118 may generally comprise any commercially available ultrasound machine having sufficient resolution, speed, and network connectivity to achieve the functionalities described herein. In one preferred embodiment, ultrasound machine 118 comprises a system available from U-Systems, Inc. of San Jose, California. Preferably, ultrasound probe 116 is of sufficient width (e.g., 15 cm) to scan the entire flattened breast surface in a single sweep. If a smaller probe (e.g., 7.5 cm) is used for cost-containment reasons or other practical reasons, the ultrasound probe 116 is swept across two or more times until the entire breast surface is scanned.

During or after the ultrasound scanning process, the raw ultrasound data is provided across the computer network 104 to the adjunctive ultrasound server 106, where the raw ultrasound data is processed into adjunctive ultrasound data that will be made available to the screening radiologist, the adjunctive ultrasound data including ultrasound slices, thick-slice images, vibrational Doppler imaging (VDI) images, CAD outputs, and other useful information. It is to be appreciated that the processing of the raw ultrasound data into the adjunctive ultrasound data may be performed by any of a variety of different computing devices coupled to the computer network 104 and then transferred to the adjunctive ultrasound server 106.

In current mass breast cancer screening environments based on x-ray mammography, a screening radiologist 124 examines x-ray mammograms for many patients en masse in a single session using an x-ray viewing station 109. The x-ray viewing station 109 may range from a simple light box, as in FIG. 1, to more complex x-ray CAD workstations that automatically move the x-ray mammograms past the radiologist 124 on a conveyor belt as a nearby CAD display highlights suspicious areas of the mammogram. Almost universally, left and right CC x-ray views 120 are positioned on one side of the x-ray viewing station 109, and left and right MLO x-ray views 122 are positioned on the other side. The radiologist 124 quickly examines the x-ray mammograms. For some x-ray mammograms the radiologist needs only a few seconds, while for other x-ray mammograms the radiologist needs up to five minutes, with an average being about two minutes per mammogram.

According to a preferred embodiment, this existing arrangements remains substantially undisturbed, but is augmented with equipment and data that facilitates fast and thorough x-ray mammogram screening by giving the radiologist a quick ultrasonic "second look" at the internal breast structure. Adjunctive ultrasound screening station 108 comprises an adjunct display 126 conveniently positioned near the x-ray viewing station 109 such that the radiologist 124 can easily look back and forth between the adjunct display 126 and the x-ray mammograms 120 and 122. Thumbnail representations 134 and 136 of thick-slice images are displayed. Generally speaking, a thick-slice image is an integration of a plurality of substantially parallel individual ultrasound slices used to represent a slab-like or thick-slice volume of the breast. The thickness of the slab-like or thick-slice volume may lie, for example, in the range of 2 mm to 20 mm, although the scope of the preferred embodiments is not so limited. Techniques for integrating the component ultrasound slices into thick-slice images according to the preferred embodiments include arithmetic averaging, geometric averaging, reciprocal averaging, exponential averaging, and other averaging methods, in each case including both weighted and unweighted averaging techniques. Other suitable integration methods may be based on statistical properties of the population of component ultrasound slices at common locations, such as maximum value, minimum value, mean, variance, or other statistical algorithms.

Preferably, the thick-slice images correspond to slab-like or thick-slice volumes of the breast having a thickness related to the size of the lesions to be detected. At an upper end, a larger thickness of 20 mm, for example, may be used if it is desirable to overlook most of the breast details and direct the user's attention to larger features on the order 10 mm in size. At a lower end, a smaller thickness of 3 mm, for example, may be used if it is desirable to view small, low-contrast lesions on the order of 2 mm in size. Thicknesses in the range of 7 mm - 12 mm are likely to be suitable for most breast cancer screening purposes.

For even quicker reference, a second adjunct display 128 is provided to form an adjunct display pair, each side corresponding to the nearest mammogram view being displayed at x-ray viewing station 109. A bar code reader 143 reads a bar code of the x-ray mammogram, wherein the associated adjunctive ultrasound data for that breast is automatically retrieved from the ultrasound server 106 and displayed on the adjunct displays 126 and 128.

By way of example and not by way of limitation, where the x-ray mammogram 120 represents the craniocaudal (CC) x-ray view for right and left breasts, respectively, the thumbnail thick-slice images 134 and 136 represent thick-slice portions of the right and left breast volumes, respectively, oriented parallel to the CC view plane. Where the x-ray mammogram 122 represents the mediolateral oblique (MLO) x-ray view for right and left breasts, respectively, thumbnail thick-slice images 138 and 140 displayed on adjunct display 128 represent thick-slice portions of the right and left breast volumes, respectively, oriented parallel to the MLO view plane.

Each thick-slice image usually represents between 0.5 cm to 1.0 cm of breast thickness. It has been found that this thickness range yields good results in assisting in the recognition of tumors that are of concern in the breast cancer screening process, which are about 0.5 cm or greater in diameter with an average diameter of about 1.2 cm. Also, because the flattened breast is usually somewhere between 4 cm and 6 cm thick, it has been found that the preferred 0.5 cm-1.0 cm thickness dimension facilitates an easy "single-glance" view of the entire breast, with a simple display of six to eight thick-slice images covering the entire breast volume. This is clearly advantageous over ultrasonic screening methods that examine one ultrasound slice at a time, in which case it would take up to 500 ultrasound slices or more to cover the entire breast volume. Although a 0.5-1.0 cm slab thickness and a 6-8 image layout has been found to yield good results, it is to be appreciated that the scope of the preferred embodiments is not so limited, and that a wide range of slab thicknesses and images-per-layout are within the scope of the preferred embodiments.

Each adjunct display 126 and 128 is flexibly mounted using adjustable mountings 130 and 132 such that the radiologist 124 may freely move them into different positions near the x-ray mammogram views to facilitate optimal back-and-forth viewing.
It has been found that ideal back and forth viewing is facilitated where each thick-slice image is not greater than 20 inches from its corresponding x-ray mammogram image.

Preferably, the adjunct displays 126 and 128 comprise touchscreen monitors. When the radiologist 124 presses one of the thumbnail thick-slice images, a larger thickslice image is displayed. In one preferred embodiment, the larger thick-slice image has a size identical to that of the x-ray mammogram image for further facilitating back-and-forth image comparisons. A control panel (not shown in FIG. 1 but described further infra) is positioned near or integrated with each adjunct display 126 and 128 that provides a user interface for the radiologist 124. In conjunction with the touchscreen display, the control panel permits quick manipulation and examination of the thick-slice images in a manner that facilitates rapid screening. By way of example, the radiologist 124 is permitted to examine each individual component slice of any thick-slice image, jogging through the individual ultrasound slices or viewing a cine-loop representation. Also, as will be described further infra, the radiologist may flexibly overlay useful information onto the thick-slice images such as vibrational Doppler image (VDI) outputs and computer-aided diagnosis (CAD) outputs.

Thus, an adjunctive ultrasound system according to the preferred embodiments does not supplant existing x-ray mammogram screening methods. Indeed, reference to the adjunctive ultrasound data is optional depending on the contents of the x-ray mammogram image, and for many patients it may not even be used at all. Rather, the adjunctive ultrasound system is there to assist the radiologist in performing their pre-existing professional duties with respect to "difficult" or "marginal" mammograms. As such, a medical establishment faces little risk of failure in acquiring an adjunctive ultrasound system according to the preferred embodiments. In a worst-case scenario, the adjunctive ultrasound system would be met with indifference by the entrenched "x-ray-only" believers, because it would not disturb their pre-existing routines. However, the adjunctive ultrasound system will be there standing by to assist in the "difficult" cases, and it is expected that even the "x-ray-only" believers will eventually find the system useful and will increasingly rely on it to increase their sensitivity and specificity performance.

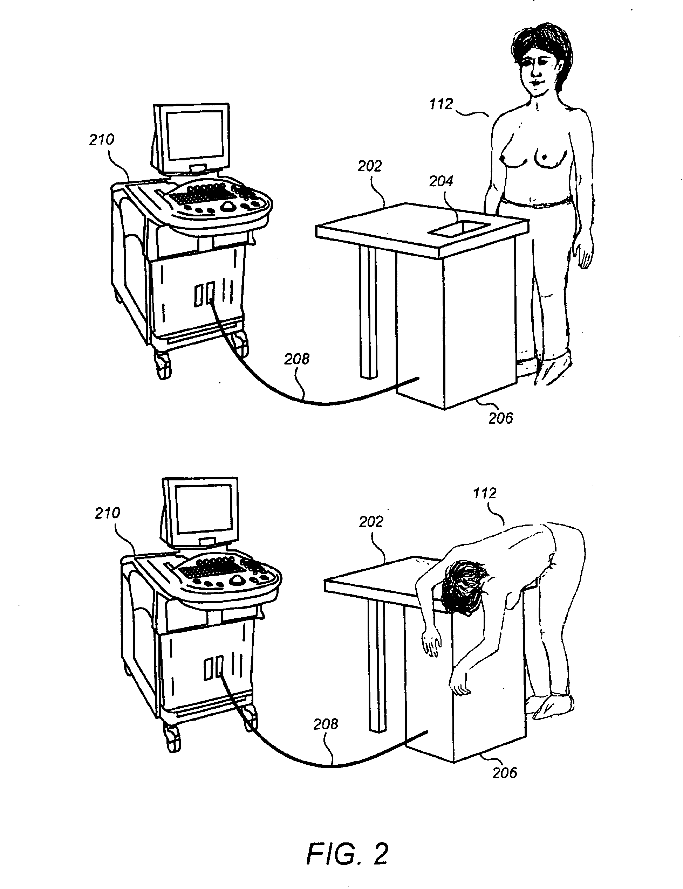

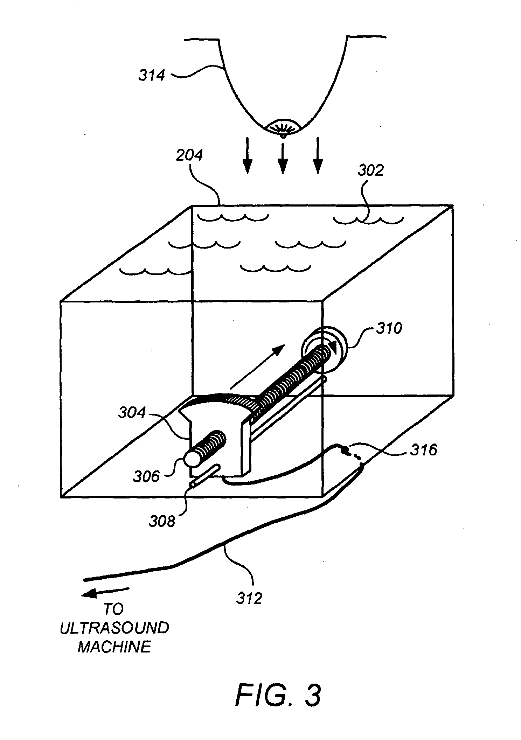

FIG. 2 illustrates an ultrasound scanning apparatus 202 according to a preferred embodiment, comprising a chamber 204 contained in a housing 206. The chamber 204 is configured to receive a breast at an open end, and further contains an acoustically conductive liquid such as water or an ultrasonic gel for facilitating acoustic coupling between an ultrasound probe (not shown) contained in the housing 206 and the breast. The ultrasound probe is connected by a lead 208 through housing 206 to an ultrasound system 210. A mechanical apparatus within housing 206, which is described further with respect to FIG. 3 below, translates the ultrasound probe during the scanning process. As shown in FIG. 2, the chamber 204 is positioned at approximately the waist level of the patient 112, who bends over to position the breast therein.

FIG. 3 illustrates a conceptual perspective view of the chamber 204 of FIG. 2. An ultrasound probe 304 is submerged beneath the surface of a liquid 302 and scans a breast 314 after the breast is immersed. A worm gear 306 is driven by a motor 310 to increment the position of the probe 304 as the ultrasound scans are taken. A probe lead 312 connects the ultrasound probe 304 to an ultrasound machine through a sealed hole 316. If the patient is in a first relative orientation with respect to the chamber 204 that yields a CC view, the relative orientation is simply shifted by roughly 45-80 degrees (e.g., by shifting the patient and/or the chamber) to obtain the MLO view, and vice-versa. To obtain another standardized x-ray mammogram view termed the lateral (LAT) view the relative orientation is shifted by about 90 degrees. The use of chamber 204 provides an advantage that the raw ultrasound slices are inherently parallel to a standard x-ray mammogram view, which obviates the need for three-dimensional volumetric reconstruction in generating the thick-slice images. Although less processing is needed, this "direct" approach requires a substantial penetration depth to penetrate from the front of the breast to the chest wall, and so a lower frequency probe (e.g., 3.5 MHz or less) is required. The raw ultrasound slices therefore have less spatial resolution as compared to a scenario in which higher frequency probes are used.

By way of example and not by way of limitation, B-mode ultrasound frames may be acquired at a rate of about 10 frames per second (fps) as the ultrasound probe is translated at a constant rate of about 1.5 mm/sec across the breast. For an uncompressed breast, the ultrasound probe 304 requires a translation distance of about 7 cm - 9 cm in order to capture the entire breast volume, which takes between 45-60 seconds. This yields from 450 to 600 raw ultrasound slices which, when processed into 1.0 cm thick-slice portions, results in 7-9 thick-slice images for display.

According to another preferred embodiment, the scanning apparatus 202 is similar to one or more scanning apparatuses described in the following references, each of which is hereby incorporated by reference: U.S. Pat. 4,167,180; U.S. Pat. 4,298,009; U.S. Pat. 4,485,819; and WO02/17792A1. Between patients, the fluid 302 should be drained and replaced to prevent inter-patient contamination and fomite propagation. In one preferred embodiment, the fluid drainage and replacement system is similar to that described in U.S. Pat. 4,282,880, which is incorporated by reference herein.

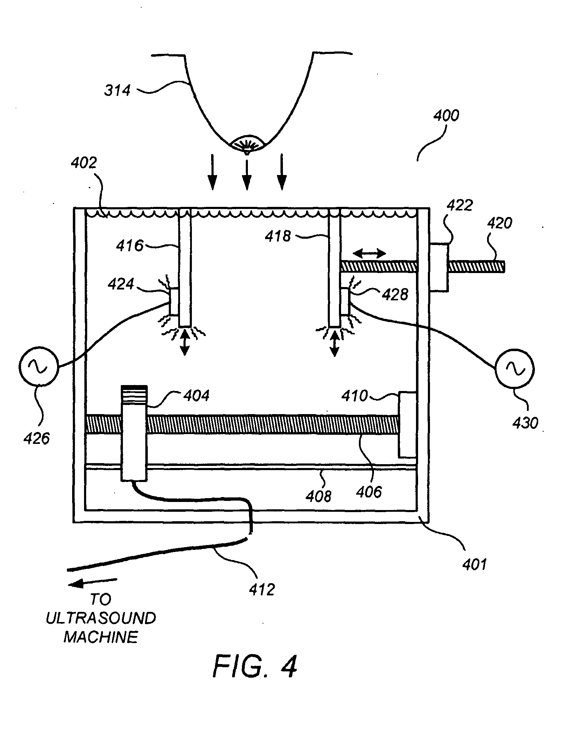

FIG. 4 illustrates a conceptual perspective view of a chamber 400 of an ultrasound scanning apparatus in accordance with a preferred embodiment. The chamber 400 may be incorporated into the ultrasound scanning apparatus of FIG. 2 in place of the chamber 204. Generally speaking, the chamber 400 is similar to the chamber 204 but has (i) the additional ability to compress the breast along an x-ray mammogram view plane, and (ii) the additional ability to facilitate vibrational Doppler imaging (VDI).

Chamber 400 comprises a sealed inner housing 401 that contains a fluid 402, an ultrasound probe 404, a worm gear 406, a stabilizer bar 408, a motor 410, and probe lead 412 similar to elements 302-310 of FIG. 3, respectively. Additionally, however, chamber 400 comprises a fixed plate 416 and a movable plate 418 that compress the breast along an x-ray mammogram view plane. If the patient is in a first orientation with respect to the compression plates 416 and 418 that yields CC compression, the relative orientation can be changed by 45-80 degrees to achieve MLO compression, and vice-versa. This can be done by rotating the patient, rotating the chamber 400, or both. Movable plate 418 is actuated by a motor 422 via a worm gear 420. It is to be appreciated that the diagram of FIG. 4 is conceptual only, and that many different mechanical configurations to achieve such breast compression functionality may be used.

Chamber 400 further comprises audio frequency transducers 424 and 428 affixed to the compression plates 416 and 418, respectively. The audio frequency transducers 424 and 428 are shown as being separately driven by audio frequency sources 426 and 430, but may alternatively be driven by a common audio frequency source. In operation, the audio frequency transducers 424 and 428 cause the compression plates 416 and 418 to vibrate at one or more audio frequencies consistent with vibrational Doppler imaging (VDI) methods. Generally speaking, the audio frequencies of interest are in the range of 100 Hz to 1000 Hz, although other frequencies may be used. To obtain the VDI data, the ultrasound system is switched into power Doppler mode as the breast volume is being vibrated. In one preferred embodiment, the breast is vibrated simultaneously at audio frequencies of 100 Hz, 200 Hz, 300 Hz, and 400 Hz. A plurality of power Doppler frames are taken sufficient to compute an average magnitude of reflector velocity. Generally speaking, this average magnitude will be lower for hard, tumor-like tissue and higher for liquid or soft tissue.

Several different sequencing techniques for acquiring B-mode frames and VDI frames are within the scope of the preferred embodiments. In one preferred embodiment, a separate-sweep method is used in which the ultrasound probe acquires B-mode data during a first sweep of the breast, and acquires VDI data during a separate second sweep across the breast. Again by way of example and not by way of limitation, the B-mode frames may be acquired at a rate of about 10 fps as the ultrasound probe is translated at 1.5 mm/sec across the breast. For a compressed breast, the required translation distance is about 4-6 cm to capture the entire breast volume, which takes between 25-40 seconds. This yields from 250 to 400 raw ultrasound slices which, when processed into 0.5-1.0 cm thick-slice portions, results in 8-12 thick-slice images. The audio frequency vibrations are turned off during the B-mode sweep and are turned on for the VDI sweep. For the VDI sweep, the ultrasound probe is preferably moved across the breast in a start-stop fashion in fixed increments of distance. It has been found that only a few VDI frames are needed per thick-slice image to meaningfully complement the B-mode scans, and so these fixed increments may be up to about 3 mm apart. At each increment, the probe is stopped for about one second to capture a plurality of power Doppler frames sufficient to measure an average reflector velocity magnitude. The probe may be quickly moved between increments, e.g., at a rate of about 6 mm per second or faster, because there are no measurements being taken during this interval. The net translation rate of the probe is therefore about 2 mm/sec, and so the VDI sweep takes on the order of 20-30 seconds to cover the breast volume. Accordingly, a combined time of between 45-70 seconds is required for the combined B-mode and the VDI sweeps. This calculation, of course, assumes that a wide probe (15 cm) is used. The required time interval is doubled if a narrower (7.5 cm) probe is used. If a conventional 4 cm probe is used, four sweeps would be required, and so on.

It is to be appreciated that the above numerical examples are presented by way of example only and not by way of limitation. For example, the raw B-mode ultrasound scans correspond to planes that are about 0.15 mm apart in the above example, and there are about 33 individual ultrasound slices per 0.5 cm thick-slice volume that are integrated into a single thick-slice image, or about 66 individual ultrasound slices per 1.0 cm thickslice volume. The above example presumes that the raw B-mode ultrasound scans are taken parallel to the desired x-ray mammogram view plane, and therefore correspond directly to the individual component ultrasound slices used to generate the thick-slice images. In a more general scenario, the raw B-mode ultrasound scans are taken along planes different than the desired x-ray mammogram view plane, in which case it is necessary to construct a three-dimensional volumetric representation of the breast and to extract the individual component ultrasound slices therefrom. In the above example, the 0.15 mm spacing of the raw ultrasound scans corresponds generally to both the axial and lateral resolution of commercially available ultrasound probes/systems, which are commonly around 0.17 mm. In this exemplary preferred embodiment, the voxels of a three-dimensional volumetric representation of the breast derived from the raw ultrasound scans will have roughly the same resolution in all three directions. Accordingly, an individual ultrasound slice extracted from the three-dimensional volumetric representation will have roughly this same resolution in both directions. However, the scope of the preferred embodiments is not so limited, and the voxels of the three-dimensional volumetric representation can generally have different resolutions in different directions. In another example, the raw B-mode ultrasound scans correspond to planes that are about 0.75 mm apart (e.g., by translating the probe at 7.5 mm/sec for 10 fps B-mode acquisition). In this case, the voxels of the three-dimensional volumetric representation of the breast will have a larger resolution in one direction than the other, and accordingly the individual ultrasound slices will have different resolutions in different directions depending on their angle relative to the scan angle, ranging from a maximum of about 0.17 mm/pixel and about 30 individual ultrasound slices per 0.5 cm thick-slice volume to a minimum of 0.75 mm/pixel and about 7 individual ultrasound slices per 0.5 cm thick-slice volume. These variations are readily compensated prior to display using any of a variety of known methods. In still another preferred embodiment, when the distance between scan planes is larger than the axial/lateral resolution limitations of the ultrasound system, the operating parameters of the ultrasound system are adjusted such that these axial/lateral resolutions correspond to the distance between scan planes, in which case the voxels of the derived from the raw ultrasound scans will again have roughly the same resolution in all three directions.

According to another preferred embodiment, an interlaced single-sweep method is used in which the B-mode frames and the VDI frames are taken in a single sweep of the probe across the breast, and in which the audio frequency vibrations are turned on at all times. The B-mode scans are taken as the ultrasound probe is moved between the discrete VDI scanning locations. By way of example, the B-mode frames may be acquired at a rate of about 10 fps as the ultrasound probe is translated at 1.5 mm/sec between VDI sampling increments that are about 3 mm apart, the probe being stopped for about one second to capture the Doppler data. This yields a net probe translation rate of about 1 mm/sec, and therefore the total required time for the scan is about 40-60 seconds, which is doubled if a narrow probe is used.

According to still another preferred embodiment, an interlaced single-sweep method is used in which the audio frequency vibrations are applied in a start-stop fashion, such that these vibrations are turned off during the B-mode frames and are turned on during the VDI frames. This technique is used where a single-sweep method is desired that avoids audio vibrations during B-scans, which may be necessary if excessive tissue movement occurs during the audio vibrations. In this preferred embodiment, sufficient time is needed at each VDI scanning location to (i) turn on the audio-frequency transducers, (ii) allow about 0.5 seconds for the tissues to begin vibrating, (ii) take the power Doppler measurements, (iv) turn off the audio- frequency transducers, and (v) allow about 0.5 seconds for the tissues to stop vibrating before resuming B-mode scans. Using a 1.5 mm/sec translation speed for the B-mode portions, this results in a net velocity of (3 mm)/(2+2 sec) = 0.75 mm/sec. For a 4-6 cm translation distance, this results in an overall scanning time between 53-80 seconds, which is doubled if a narrow probe is used.

In the above the non-limiting example, each thick-slice image is associated with a plurality of corresponding VDI frames of data. These VDI frames may be arithmetically averaged together or otherwise integrated into a single thick-slice VDI frame for subsequent optional superposition onto the thick-slice images as described infra.

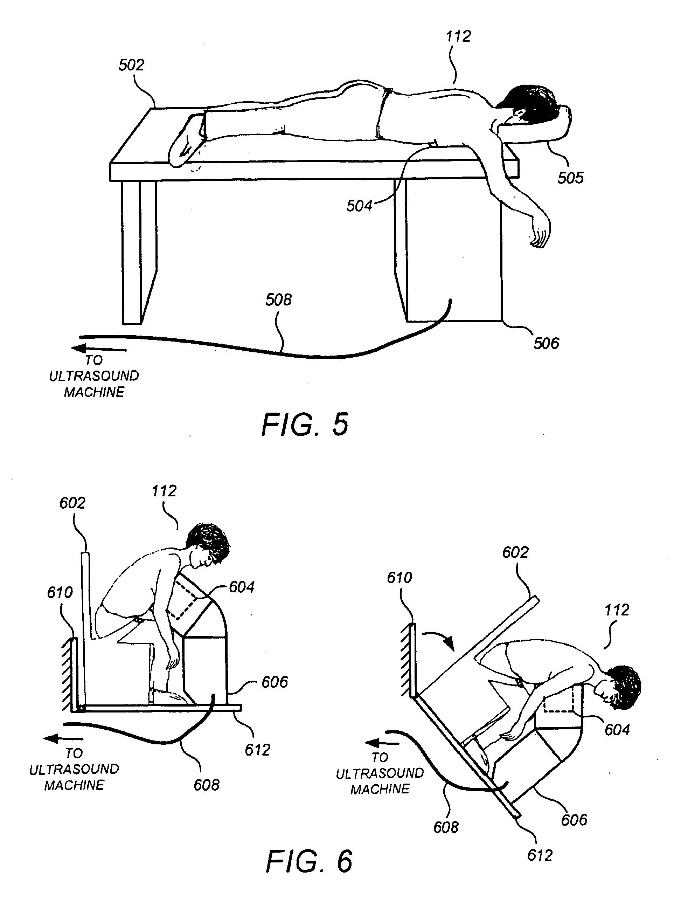

FIG. 5 illustrates a perspective view of an ultrasound scanning apparatus 502 according to another preferred embodiment. Ultrasound scanning apparatus 502 comprises a chamber 504 similar to the chamber 204 of FIG. 2 or the chamber 400 of FIG. 4, a chamber housing 506, and a probe lead 508 for coupling to an ultrasound machine. However, a table-like structure is provided that allows the patient 112 to lie face down during the procedure, their head resting comfortably in a headrest 505 similar to a headrest provided on a conventional massage table. Using means within housing 506 not shown in FIG. 5, the reservoir 504 is preferably rotatable around a central vertical axis such that compression along different standard x-ray mammogram view planes (CC/MLO) is allowed.

FIG. 6 illustrates a perspective view of an ultrasound scanning apparatus 602 according to another preferred embodiment. Ultrasound scanning apparatus 602 comprises a chamber 604 similar to the chamber 204 of FIG. 2 or the chamber 400 of FIG. 4, a chamber housing 606, and a probe lead 608 for coupling to an ultrasound machine. However, a chair-based structure is provided that is mounted upon a forward-tiltable surface 612 that tilts forward respect to a base 610. In operation, the patient 112 is seated in the scanning apparatus 602 while the chamber 604 is devoid of fluid, and the patient gently leans against the housing 606. The entire scanning apparatus 602 is then tilted forward until the patient's chest wall is substantially horizontal and the breast is hanging down into the chamber 604. The chamber 604 is then filled with fluid, the breast is compressed by compression plates (see FIG. 4), and the scanning operation is initiated. After the scanning operation is complete, the chamber 604 is emptied of fluid and the scanning apparatus 602 is returned to its initial horizontal position.

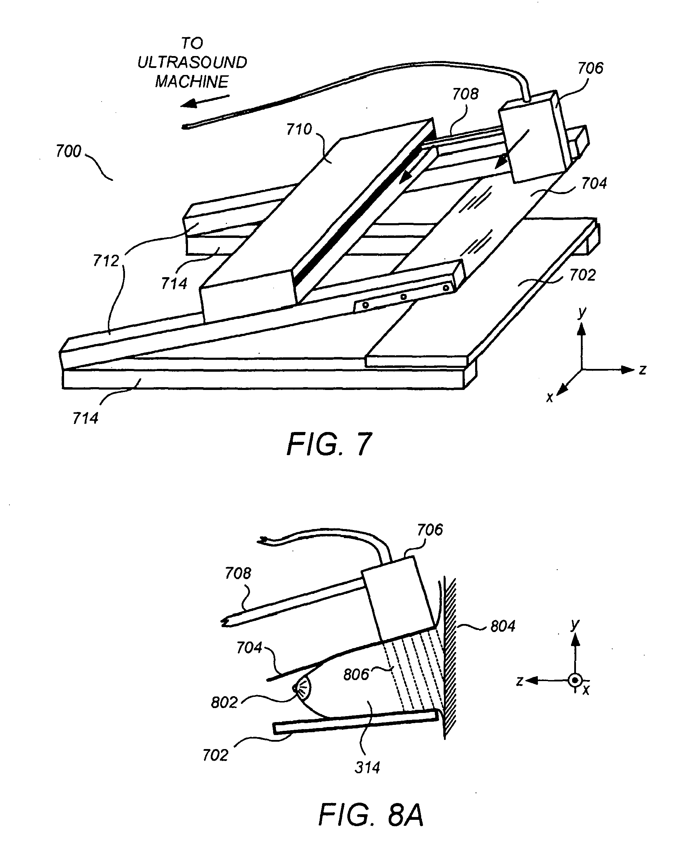

FIG. 7 illustrates a perspective view of an ultrasound scanning apparatus 700 according to another preferred embodiment. Scanning apparatus 700 comprises an upper support frame 712 that is movable toward a lower support frame 714. It is not required that the upper and lower support frames be hingeably connected to each other, as implied in the configuration of FIG. 7, provided that they can ultimately be brought together to so as to compress the breast. A lower compressive member 702, comprising for example a stiff Buna-N rubber material, is supported by the lower support frame 714. An upper compressive member 704 comprising a taut, partially conformable sheet of acoustically transparent material such as Mylar® polyester film is stretched across, and supported by, the upper support frame 712. An ultrasound probe 706 coupled to an ultrasound machine is positioned over the polyester film 704 and is affixed to a rigid member 708, which is in turn coupled to an actuator 710 as shown. Preferably, the polyester film 704 is optically transparent so that the medical technical can see the breast from above as it is being compressed. Preferably, as is detailed further with respect to FIG. 8A below, ultrasound scanning apparatus 700 is configured and dimensioned such that lower compressive member 702 is at a shallow angle of about 10 degrees with respect to the horizontal. Also, the polyester film 704 preferably forms about a 5 degree angle with respect to the lower compressive member 702 when lowered to a distance of about 6 cm therefrom. While being shown between two lateral frame members in the embodiment of FIG. 7, the polyester film 704 can be framed on all sides in an alternative preferred embodiment.

The lower compressive member 702 is preferably maintained at a height that allows a standing patient to lay their breast thereon without crouching and such that their chest wall is substantially vertical. In operation, the polyester film 704 is generously coated on both sides with an ultrasonic gel, mineral oil, and/or water and lowered onto the breast. Although the polyester film 704 deforms by a small amount, a substantially horizontal surface is still provided for mechanical translation of the ultrasound probe 706 across the top surface thereof. Preferably, an overall compressive force similar to that applied during x-ray mammography, e.g. about 20-25 pounds, is applied by the polyester film 704. Between patients, the polyester film 704 is replaced and the lower compressive member 702 is sterilized or replaced to prevent inter-patient contamination and fomite propagation. As is common in the field, a small metallic object such as a small ball bearing or BB is taped to the breast at a location corresponding to the nipple so that the nipple location is detectable in the acquired ultrasound images.

According to another preferred embodiment, the ultrasound probe 706 actually comprises two mechanically affixed side-by-side ultrasound probe heads, including a lower-frequency probe head and a higher-frequency probe head. Each probe head is connected to a different probe input of the ultrasound machine, which is capable of being controlled by the ultrasound technician so as to selected one probe head or the other. According to a preferred embodiment, the lower-frequency probe is used for patients with large breasts, while the higher-frequency probe is used for patients with medium or small breasts. The lower-frequency probe operates at lower frequencies, e.g. 5-7 MHz, for enabling deeper scans for the larger breast, e.g. as deep as 10 cm, albeit at a somewhat reduced resolution. The higher-frequency probe operates at higher frequencies, e.g. 8-12 MHz, which penetrates only to the shallower depths of 4-6 cm, but which provides higher resolution than the lower-frequency probe. In an alternative preferred embodiment, large breasts are scanned in a two-sweep process, the first sweep being high-frequency and the second sweep being low-frequency, and the near field portions of the images resulting from the first sweep are stitched to the far field portions of the images resulting from the second sweep.

In still another alternative embodiment, the lower compressive member 702 is replaced by a lower film/probe/actuator assembly ("lower assembly") that mirrors the upper film/probe/actuator assembly 704/706/708/710 ("upper assembly"). The lower assembly is similar to the upper assembly, and both are equipped with higher-frequency ultrasound probes. When small or medium breasts are presented, only the upper assembly actively scans the breast, and the lower assembly remains dormant, that is, it serves the function of a lower compressive member but does not actively scan the breast. However, when a large breast is presented, a first sweep of the upper assembly and a second sweep of the lower assembly are executed. The near-field portions of the images resulting from the first and second sweeps, which collectively scan the entire large breast volume, are then stitched together.

According to another preferred embodiment, the ultrasound probe 706 actually comprises two mechanically affixed end-to-end ultrasound probe heads, each having a shorter length individually (e.g., 7.5 cm) but, when placed end-to-end, forming a larger effective probe head (e.g., 15 cm). In one preferred embodiment, the first probe head is operative on a first sweep across the breast, while the second probe is operative on a second sweep. An A-B switch is provided in the ultrasound machine for automatically switching between the probe heads. In another preferred embodiment in which only a single sweep is required, the probe heads operate sequentially at non-overlapping time intervals for each ultrasound slice during the sweep. The resulting separate ultrasound frames are stitched together using known methods. Generally speaking, acquiring two shorter end-to-end probe heads is less expensive than acquiring a single long probe head. This can be appreciated with respect to transducer element yields during probe manufacture. Other factors being equal, if a single 7.5 cm transducer can be made with a 90% element yield, a 15 cm transducer would only have a 90% x 90% or 81% element yield, with associated higher manufacturing costs.

In still another preferred embodiment, an irrigation mechanism (not shown) is provided that maintains a stream of nonviscous, acoustically transparent fluid such as water at an interface between the thin film 704 and the ultrasound probe 706 during the ultrasound scans, thereby enhancing the acoustic coupling between said ultrasound probe 706 and the breast. In still another preferred embodiment, one or more audio frequency transducers (not shown) are affixed to one or more of the lower compressive member 702 and the thin film 704 for inducing audio frequency vibrations in the breast during the ultrasound scans according to a vibrational Doppler imaging (VD1) modality.

In yet another preferred embodiment, the upper support frame 712 is springably and hingeably mounted with respect to the lower support frame 714 such that the polyester film 704 may adaptably tilt forward or backward in the anterior/posterior direction relative to the patient as it compresses the breast to obtain a best compression angle. Visualized with respect to FIG. 7, the springable, hingeable tilting would take place around an axis parallel to the "x" axis, and that axis of rotation would itself be movable in the "y" direction and, to a lesser extent, in the "z" direction. Such a device would provide enhanced compression for a wider variety of breasts that have differing profiles in the y-z plane of FIG. 7. In one preferred embodiment, the device is similar to a commercially available conforming compression device used for x-ray mammography, termed the FAST Paddle (Fully Automatic Self-adjusting Tilt Compression Paddle), available from LORAD, a Hologic Company, of Danbury, Connecticut, and described on their public web site.

In still another preferred embodiment, a gap-filling gel bag or other flexible bag containing an acoustically transparent fluid such as water, gel, mineral oil, or the like is positioned above the breast prior to compression by the polyester film 704. This enhances imaging of those breast portions corresponding to upper skin locations that may not come directly into contact with the polyester film 704 during compression. The gap-filling bag surface may comprise latex, Nitrile, Saran Wrap, cellophane, or other suitable material. Alternatively, the gap filling material may be a highly conformable cross-linked polymer gel (or a "slime" material often used in children's toys) having appreciable acoustic transmissivity but requiring an outer bag.

In yet another preferred embodiment, the polyester film 704 is replaced by a stiffer plastic material such as PETG. The stiffer plastic material does not require taut placement across frame members, but rather is vacuum-formed into a structure having a flat bottom surrounded by elevated outer ridges. The elevated outer ridges are affixed to the frame members. When the structure is pressed down onto the breast, a lower surface of the flat bottom compresses the breast, and the ultrasound probe is swept across an upper surface of the flat bottom. Substantial force is applied to the breast by virtue of the stiffness of the flat bottom material as strengthened by the elevated outer ridges, and the flat bottom only deforms by a small amount.

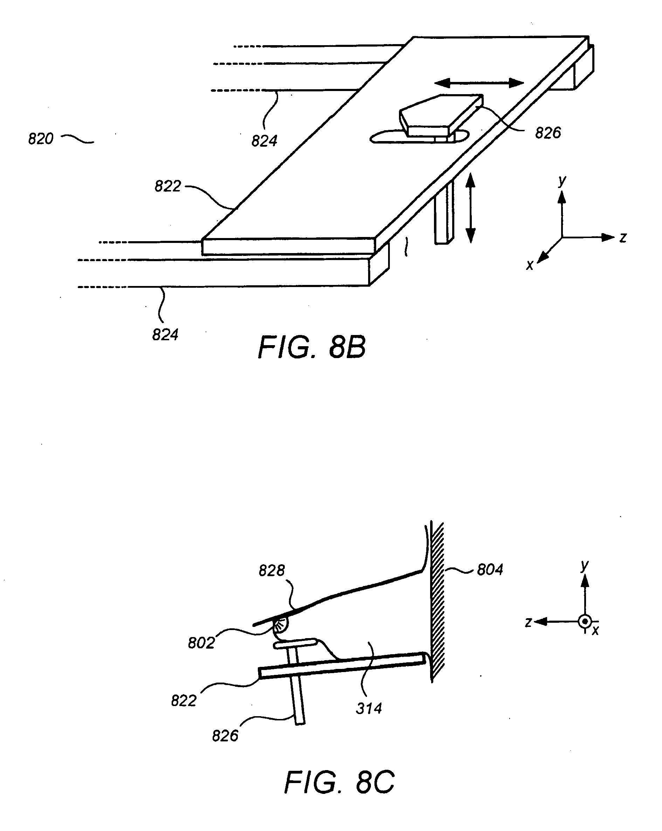

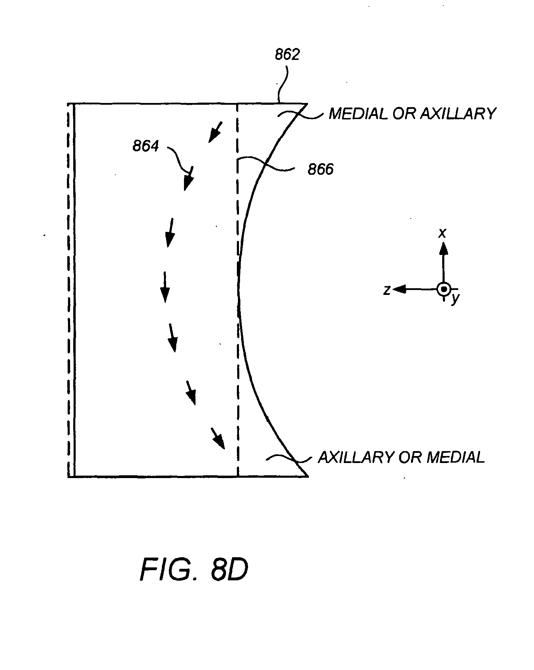

FIG. 8A illustrates a side cutaway view of a breast 314 as it is being compressed and scanned by the ultrasonic scanning apparatus 700. One advantage of the ultrasound scanning apparatus 700 is illustrated in FIG. 8, which depicts a conceptual view of the substantially vertical chest wall 804 of the patient. Because the angle of the polyester film 704 is less than 90 degrees (preferably about 75 degrees) with respect to the chest wall 804, the acoustic interrogation field 806 can penetrate all the way to the chest wall 804 for most of the breast volume. This penetration can be achieved even where a high frequency ultrasound probe (e.g., 7.5 MHz or greater), which has a greater resolution but a lesser acoustic penetration depth, is used. This feature is especially important in light of the fact that a substantial percentage of breast cancers are formed within 2 cm of the chest wall. Yet another advantage of the ultrasound scanning apparatus 700 is its physical profile as a "stand-up" apparatus that takes up minimal floor space, which is a valuable and scarce resource at many medical clinics.

FIG. 8B illustrates a lower portion 820 of an ultrasound scanning apparatus according to another preferred embodiment in which nipple imaging is enhanced. The ultrasound scanning apparatus is similar to that of FIG. 7, but only an area around the lower compressive member is drawn for clarity of description. A lower compressive member 822 is positioned on a lower support member 824 as in the embodiment of FIG. 7. Slidably coupled to the lower compressive member is a nipple support platform 826 for urging the breast nipple upward toward the polyester film. The nipple support platform 826 comprises a rigid frame that is covered by a soft, pliable silicone rubber sheet. The nipple support platform 826 is adjustable in two directions as indicated in FIG. 8B in order to be operative on a variety of different breast types. A locking mechanism (not shown) locks the nipple support platform 826 into place after being adjusted to a particular breast. The nipple support platform 826 allows the area of the breast near the nipple, as well as the nipple itself, to appear more completely in the acquired adjunctive ultrasound data.

FIG. 8C illustrates a side view of the breast 314 as it is being compressed and scanned by the ultrasonic scanning apparatus of FIG. 8B. The nipple support platform 826 gently urges the nipple 802 against the polyester film 828 such that it makes sound acoustic contact therewith. This enhances imaging of the nipple 802, which is a useful reference point for the radiologist in comparing the ultrasonic images to the x-ray mammogram images. By acoustic contact, it is meant that the nipple 802 either directly touches the polyester film 828 or is close enough to it such that any gaps between the nipple 802 and the polyester film 828 are occupied by ultrasonic gel or other acoustically conductive material.

FIG. 8D illustrates a simplified top view of a lower portion of an ultrasound scanning apparatus according to another preferred embodiment in which a more thorough scan of the breast is provided toward the medial and axillary regions of the breast near the chest wall. A lower compressive member 862 is curved so as to be conformal with the chest wall all the way from the center-facing side of the breast to the axillary side of the breast. It has been found that a radius of curvature of about 9 inches provides improved chest wall and axillary imaging of the breast for a broad range of breast sizes. Optionally, different lower compressive members with different shapes and/or radii of curvature are used for different patients, e.g., different lower compressive members for small, medium, and large breasts. During the scanning process, the ultrasound probe is moved in a curved or arcuate trajectory by an actuator mechanism, for example, by adapting the actuator 710 of FIG. 7 to have an inward/outward motion along with its side-to-side motion. The increased-area region 864 is shown in FIG. 8D for comparison with a rectangular-area scan area 866. Preferably, the associated x-ray mammograms are taken using a similarly-conformal x-ray detector mechanism, such as that described in U.S. Pat. 6,181,769, which is incorporated by reference herein. This allows meaningful comparison of the increasedarea adjunctive ultrasound data with corresponding increased-area x-ray mammogram data.

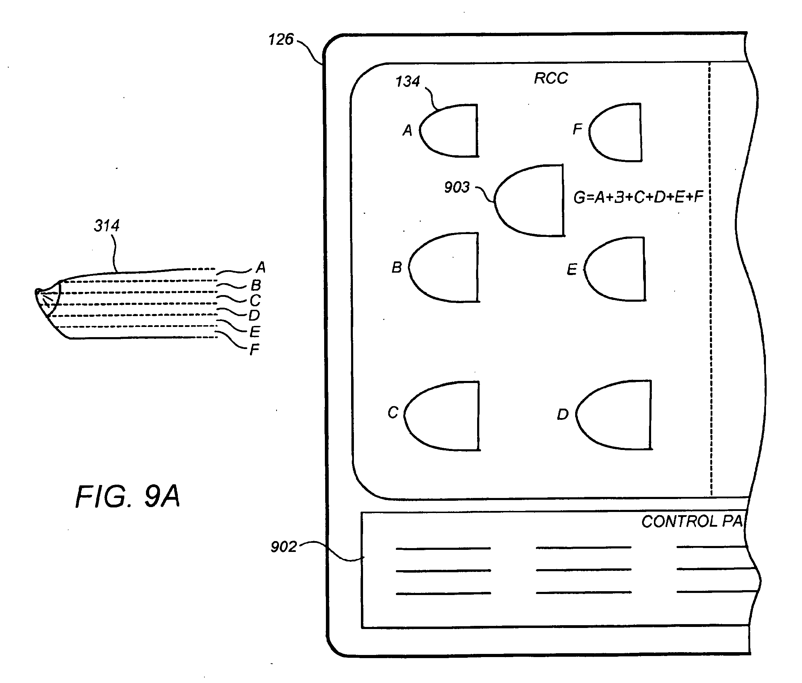

FIG. 9A illustrates a view of thick-slice portions A-F of a breast 314 as presented to the radiologist by the adjunct display 126 according to a preferred embodiment. It is to be appreciated that while the examples herein are presented in terms the right CC view ("RCC"), they are readily extended to other views and combinations of views. Adjunct display 126 comprises a control panel 902 (described further infra) for use by the radiologist in causing the thumbnail thick-slice images 134 to be displayed as shown. According to one preferred embodiment, the thumbnail thick-slice images 134 represent 10:1 subsamplings of the full-scale thick-slice images. However, it is to be appreciated that the thumbnail thick-slice images 134 can vary in size from very small to very large, even full-scale or bigger, depending on the number of thick-slice regions in the breast and the available space on the adjunct display 126. Thus, the term thumbnail image is used primarily to denote their information summarizing and linkage functionalities, and is not to be construed as limiting their size. As indicated in FIG. 9A, the thick-slice thumbnail images 134 are preferably displayed in a quasi-counterclockwise manner ("A" downward to "C" on the left side, "D upward to "F" on the right side) to approximate a visual "migration" for the radiologist's eye from the top layer to the bottom layer of the breast 314. In an alternative preferred embodiment, the sequence can be quasi-clockwise.

According to another preferred embodiment, as illustrated in FIG. 9A, an image 903 showing a composite view G is also displayed to the user. The composite view G represents an integration of all of the thick-slice images (A+B+C+D+E+F) and/or their component slices such that it represents information from the entire breast volume. Preferably, the composite view G of the breast has its gray-scale polarity toggled and/or remapped such that it is reminiscent of an x-ray mammogram taken from the standardized x-ray mammogram direction. In another preferred embodiment, each of the thick-slice thumbnail images 134 has its gray-scale polarity toggled and/or remapped such that it is reminiscent of an x-ray mammogram.

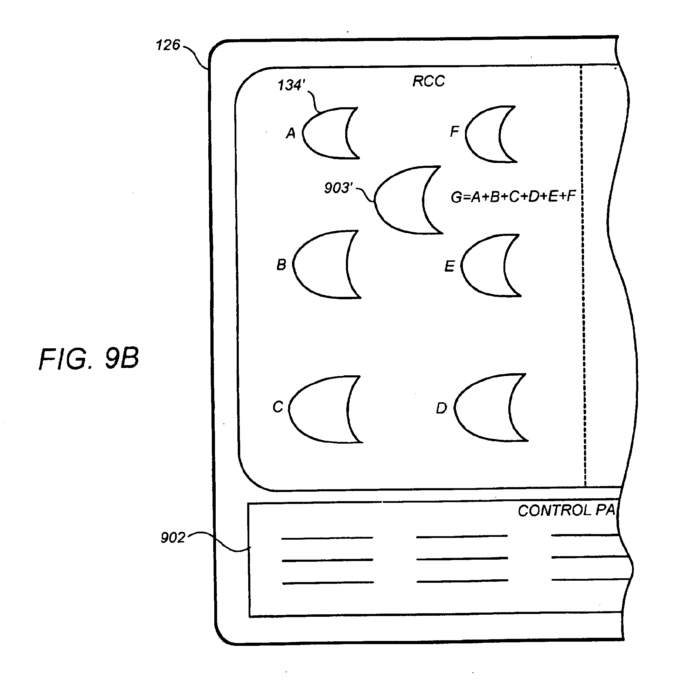

FIG. 9B illustrates an adjunct ultrasound display according to a preferred embodiment that is similar to FIG. 9A, except that the arcuate-trajectory scanning apparatus of FIG. 8D was used. As indicated in FIG. 9B, increased information near the medial and axillary regions of the breast is provided in the images 134' and 903'.

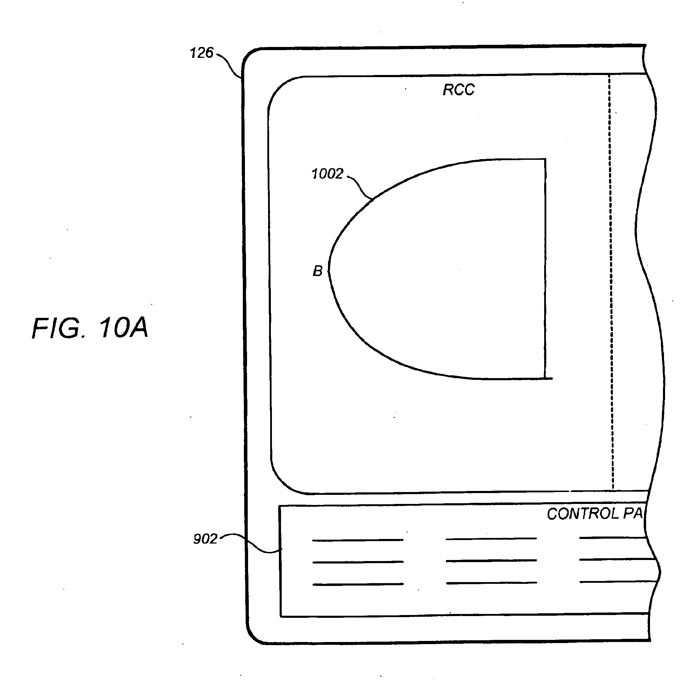

FIG. 10A illustrates a view of a single thick-slice volume "B" of the breast 314 in the form of a thick-slice image 1002. Preferably, the thick-slice image 1002 is identical in size to the x-ray mammogram of the breast to facilitate comparisons therebetween. In operation, the full-scale thick-slice image 1002 is caused to appear in place of the side-by-side thumbnail display of FIG. 9A by pressing an "enlarge" button on the control panel 902 and pressing the thumbnail image "B" on the touchscreen display.

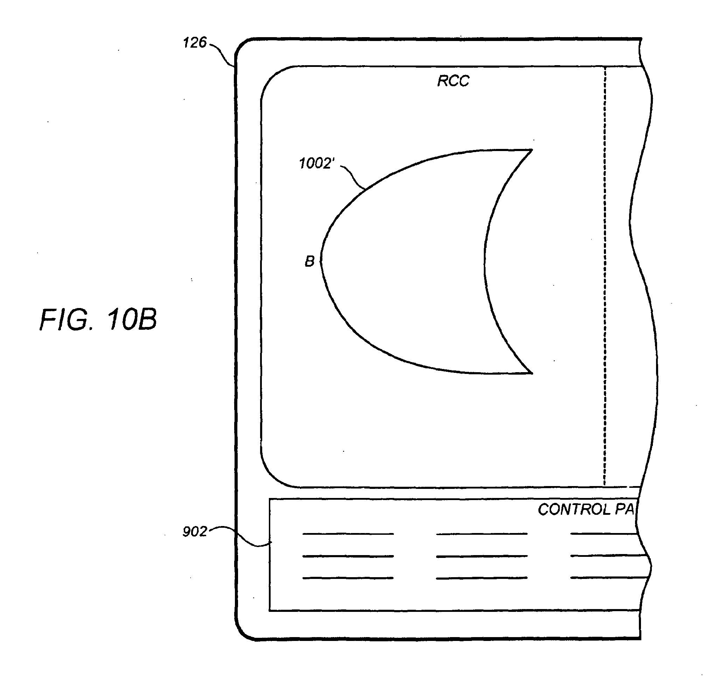

FIG. 10B illustrates an adjunct ultrasound display according to a preferred embodiment that is similar to FIG. 10A, except that the arcuate-trajectory scanning apparatus of FIG. 8D was used. As indicated in FIG. 10B, increased information near the medial and axillary regions of the breast is provided.

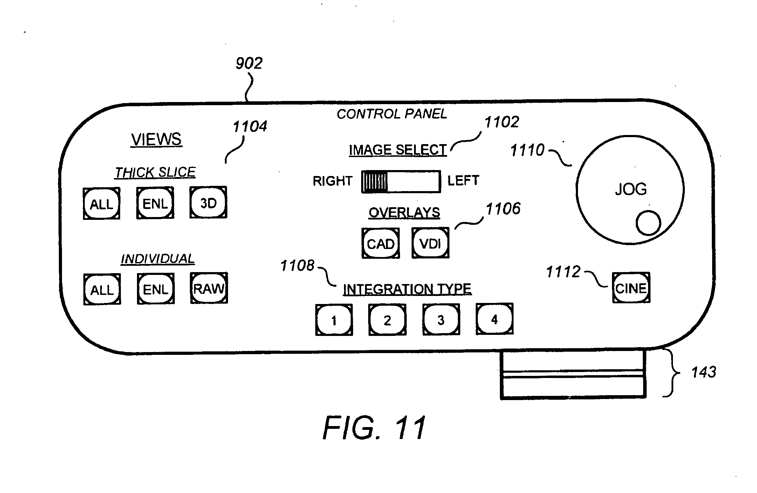

FIG. 11 illustrates a close-up view of the control panel 902 of an adjunct ultrasound display according to a preferred embodiment. A two-position image select switch 1102 allows the radiologist to separately control the displays of the right and left breasts, which appear side-by-side on the adjunct display 126 as shown in FIG. 1 supra. A view control area 1104 allows the radiologist to select among a variety of views. If a a particular view also requires an identification of a thumbnail image, the radiologist touches that thumbnail image on the touchscreen. By pressing the THICK SLICE-ALL button, the radiologist causes the side-by-side thumbnails of thick-slice images to appear as in FIG. 9, which is also the default view. The THICK SLICE-ENL button (enlarge) causes a full-scale thick-slice image to be displayed as in FIG. 10A. The THICK SLICE-3D button causes a three-dimensional perspective image of a particular thick-slice portion of the breast to be displayed.

As described supra, the thick-slice images represent integrations of individual component ultrasound slices. It is often the case that the radiologist may be interested in viewing each individual component ultrasound slice separately. The INDIVIDUAL-ALL button causes all component ultrasound slices of a given thick-slice image to be displayed side-by-side. The INDIVIDUAL-ENL (enlarge) button causes a selected component ultrasound slice to be enlarged to a full-scale image that is the same size (or almost the same size) as the x-ray mammogram image. It is to be appreciated that the size of the full-scale image, which is projected to full scale using known dimensions from the ultrasound scanning process, might vary slightly from the actual mammogram size. This is because the mammogram will be slightly larger than the breast due to some spreading of the x-ray beam on its way to the x-ray film or detector. For use in conjunction with this feature, a JOG control 1110 allows the radiologist to jog through all of the component ultrasound slices of the thick-slice image, and a CINE control 1112 allows the radiologist to alternatively view a cine-loop presentation of the component ultrasound slices.

As described supra, depending on the orientation from which the original ultrasound scans were taken, the individual component ultrasound slices that form a given thick-slice image may either be "raw" ultrasound data (if the original scans are taken in the same plane as the x-ray mammogram view plane), or may themselves be computational constructs from a three-dimensional volumetric representation of the breast (if the original scans are taken in a different plane than the x-ray mammogram view plane). In either case, the radiologist may wish to view the raw ultrasound slices obtained from the original ultrasound scan of the patient, which is done by pressing the INDIVIDUAL-RAW button. The JOG control 1110 is used to "travel" among the raw ultrasound slices, or alternatively the CINE feature may be used.

Control panel 902 further comprises overlay controls 1106 that permit the radiologist to overlay CAD results and/or VDI data onto the thick-slice image(s) or individual-slice image(s) being displayed, responsive to toggle buttons CAD and VDI, respectively. Control panel 902 further comprises an integration control 1108 that allows the radiologist to select from among several different integration methods for generating the thick-slice images from their component ultrasound slices. For example, button "1" may select a straight arithmetic averaging method, button "2" may select a geometric averaging method, button "3" may select a weighted arithmetic averaging method using a first set of weights, button "4" may select a weighted arithmetic averaging method using a second set of weights, and so on. Techniques for generating thick-slice images from component ultrasound slices according to the preferred embodiments include arithmetic averaging, geometric averaging, reciprocal averaging, exponential averaging, and other averaging methods, in each case including both weighted and unweighted averaging techniques. One particularly suitable technique is a weighted arithmetic averaging method. The bar code reader from FIG. 1, supra, is also shown in FIG. 11.

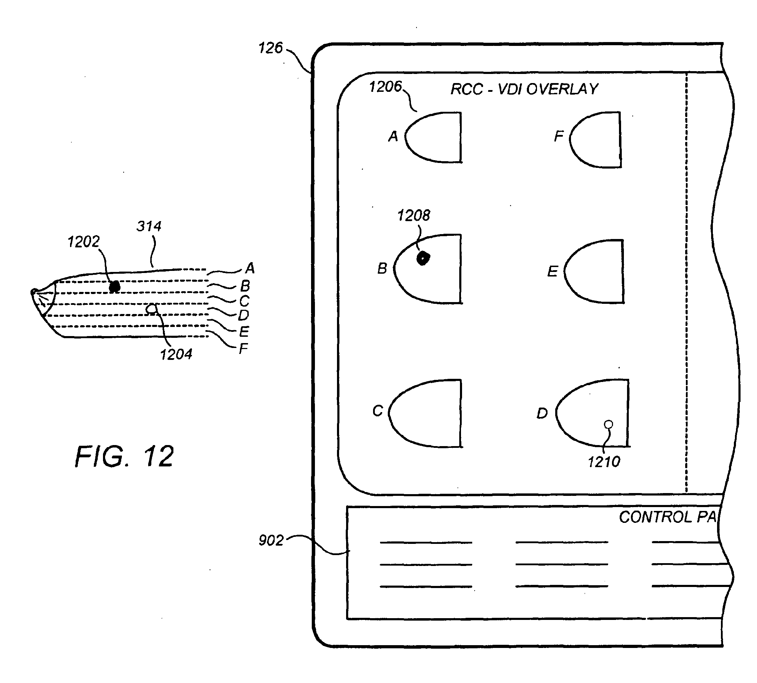

FIG. 12 illustrates a conceptual view of an adjunct ultrasound display with a VDI overlay, along with a side view of a breast 314 corresponding thereto. In this example, the breast 314 contains a hard tumor 1202 in thick-slice region B and a liquid-filled cyst 1204 in thick-slice region D for purposes of illustrating an advantageous use of a VDI overlay according to a preferred embodiment. The hard tumor 1202 will usually resonate less than the surrounding tissue when the VDI audio frequency is injected, and which will show up as reduced average Doppler velocity magnitude regions in the VDI overlay for the "B" slice, which is highlighted at location 1208 in FIG. 12. In contrast, the liquid-filled cyst 1204 will resonate roughly the same amount as the surrounding tissue and will tend to blend in the VDI overlay, as indicated by the weak dotted lines at location 1210 in FIG. 12. The ability to overlay VDI data onto the thick-slice images represents an important advantage afforded by an adjunctive ultrasound system in accordance with the preferred embodiments. Although shown in black-and-white in FIG. 12, the VDI overlays at locations 1208 and 1210 are preferably displayed according to a color map that maps different amounts of induced vibration into different colors, e.g., low amounts displayed in red, high amounts displayed in violet, etc. Preferably, the color map is generated individually for each set of VDI data and is normalized between the minimum and maximum VDI values for that data set.

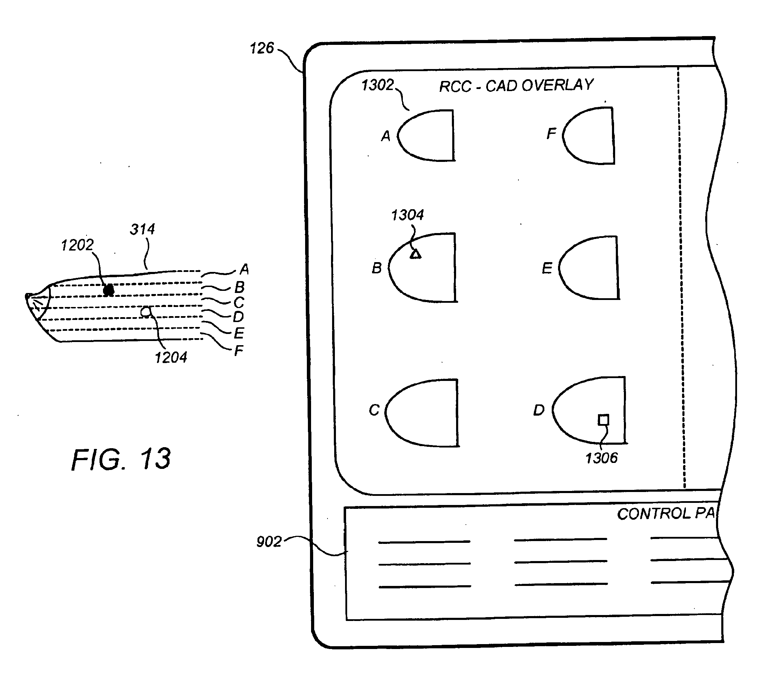

FIG. 13 illustrates a conceptual view of an adjunct ultrasound display with a CAD overlay corresponding to the breast presented in FIG. 12. By way of example, the problematic tumor 1202 has been singled out by a triangle 1304 to indicate a high degree of suspiciousness, while the cyst 1204 has been identified by a square 1306 to indicate a minimal degree of suspiciousness. Differently-sized icons may also be use to indicate different relative degrees of suspiciousness. Any of a variety of other CAD annotation schemes can be used, such as those described in U.S. Pat. 6,266,435 (Wang), which is incorporated by reference herein.

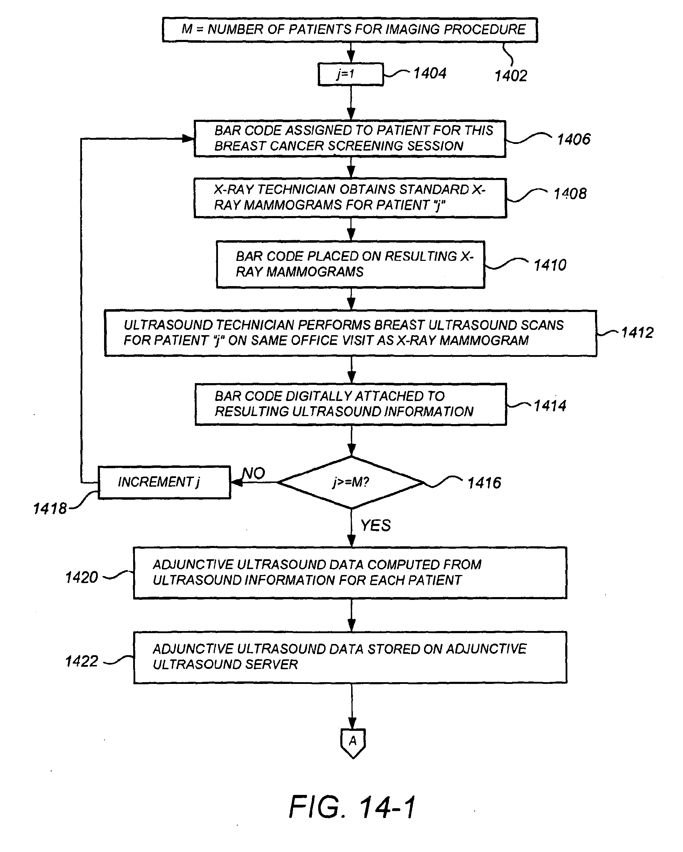

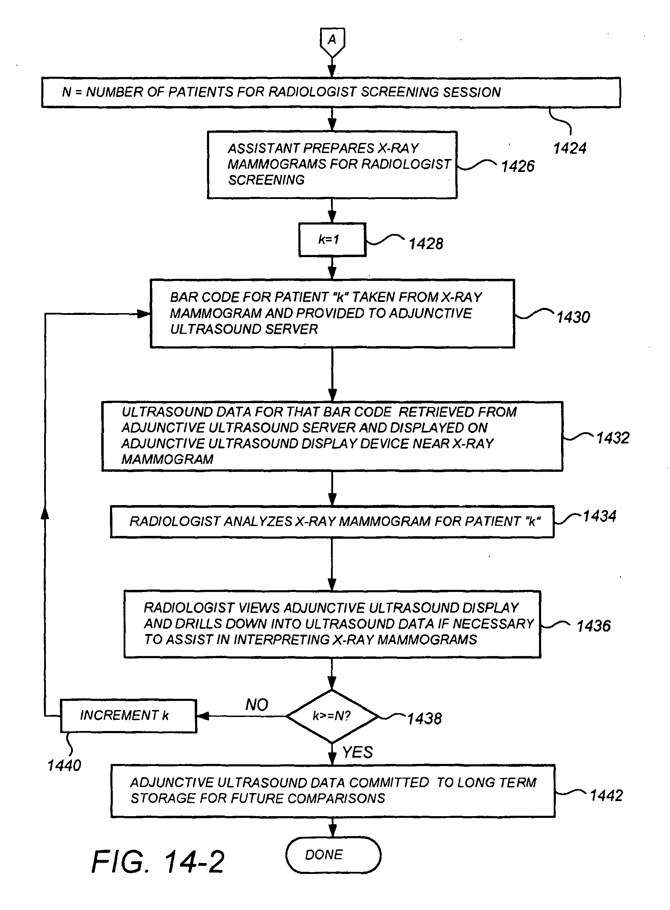

FIG. 14 illustrates steps for mass breast cancer screening using adjunctive ultrasound mammography according to a preferred embodiment. For the purposes of the flowchart of FIG. 14, "M" represents the number of patients to undergo ultrasound scanning (step 1402) with a counter variable "j" (step 1404), while "N" represents the number of x-ray mammograms to be screened in a single session by radiologist (step 1424) with a counter variable "k", it being understood that these two sets of events usually take place at substantially different intervals in time, e.g., 1-30 days apart. At step 1406, a bar code is assigned to the patient for the screening session. This is often done in large clinics by handing the patient a set of bar-coded stickers that are used to identify the patient in a plurality of tests on that visit such as blood tests, urine tests, etc. in addition to x-ray mammogram.

At step 1408, an x-ray technician obtains standard x-ray mammograms for the patient, and at step 1410 the x-ray technician places a bar code sticker on each x-ray mammogram film. As known in the art, if there is a palpable lesion in the breast, a small metallic marker or "BB" is taped to the breast at the tumor location to draw attention to this region. During the same visit, an ultrasound technician performs breast ultrasound scans on the patient, using a bar code scanner to scan in a bar code from one of the patient's stickers. This bar code is digitally attached to the resulting ultrasound information at step 1414, e.g. by insertion into a metadata field of each ultrasound information file. The same metallic marker or "BB" used for the x-ray mammogram may be used during the ultrasound scans to draw attention to palpable lesions. After completing the scanning process for all "M" patients (steps 1416-1418), at step 1420 adjunctive ultrasound data (i.e. the set of data to be available for presentation to the radiologist) is computed from the raw ultrasound data for each patient. At step 1422, the adjunctive ultrasound data is stored onto an adjunctive ultrasound server, i.e., the server(s) with which the radiologist will interact during the en masse screening process.

At step 1426, an assistant prepares "N" x-ray mammograms for en masse radiologist screening. This step varies in complexity from simply mounting the mammograms onto one or more light boxes to loading and sequencing an x-ray mammography CAD station. When the radiologist turns their attention to the x-ray mammogram of a given patient "k," at step 1430 they scan the bar code contained on the x-ray mammogram using a bar code reader (not shown in FIG. 1). Alternatively, if a more sophisticated x-ray mammography screening apparatus is used such as a CAD workstation with an automated conveyor system, the assistant preparing the x-ray mammograms scans the bar code from each x-ray mammogram as it is being loaded into the conveyor system. This causes the desired adjunctive ultrasound data to displayed in synchronization with the x-ray mammograms. At step 1432, ultrasound data for that bar code is retrieved from the adjunctive ultrasound server and displayed on the adjunct display positioned near the x-ray mammogram. At step 1434, the radiologist screens the x-ray mammogram, and at step 1436 the radiologist views the adjunct display and drills down into the adjunctive ultrasound data as necessary to assist in interpreting the x-ray mammograms. After the screening process for all "N" x-ray mammograms (steps 1438-1440) is completed, the adjunctive ultrasound data is committed to long-term storage for future comparisons (step 1442). In one preferred embodiment, the radiologist may view one or more years of historical adjunctive ultrasound data for a given patient to see if any changes have taken place.

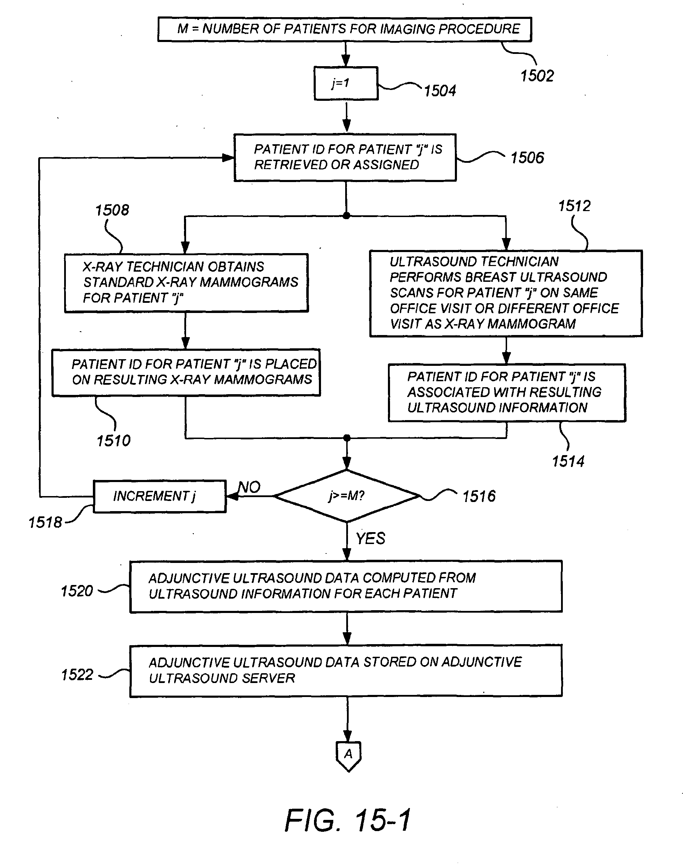

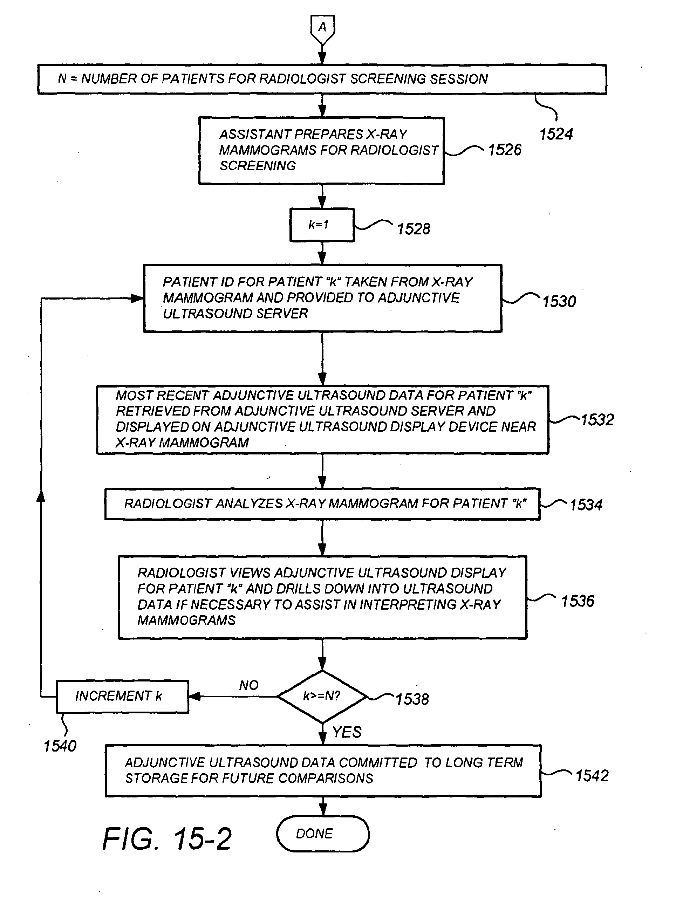

FIG. 15 illustrates steps for mass breast cancer screening using adjunctive ultrasound mammography according to a preferred embodiment. In the preferred embodiment of FIG. 15, it is not assumed that the patient undergoes x-ray mammography and ultrasonic mammography on the same visit, although same-day scenarios are accommodated. Steps 1502-1504, 1520-1526, and 1534-1542 proceed in a manner similar to steps 1402-1404, 1420-1426, and 1434-1442 of FIG. 14. However, at step 1506, a permanent patient ID (e.g., Social Security number, medical insurance number, hospital-assigned medical record number, etc.) is retrieved or assigned when the patient arrives at the clinic. At step 1508, an x-ray technician obtains standard x-ray mammograms for the patient, with the patient ID been placed directly on the x-ray mammogram film at step 1510. At step 1512, during that visit or another visit, an ultrasound technician performs breast ultrasound scans for the patient, and at step 1514 associates the patient ID with the resulting ultrasound information. At this time, a time stamp is automatically associated with that ultrasound information and patient ID. Later, during en masse x-ray mammogram screening, only the patient ID needs to be provided to the adjunctive ultrasound server (step 1530), and the most recent set of adjunctive ultrasound data is retrieved by the adjunctive ultrasound server for that patient ID (step 1532).

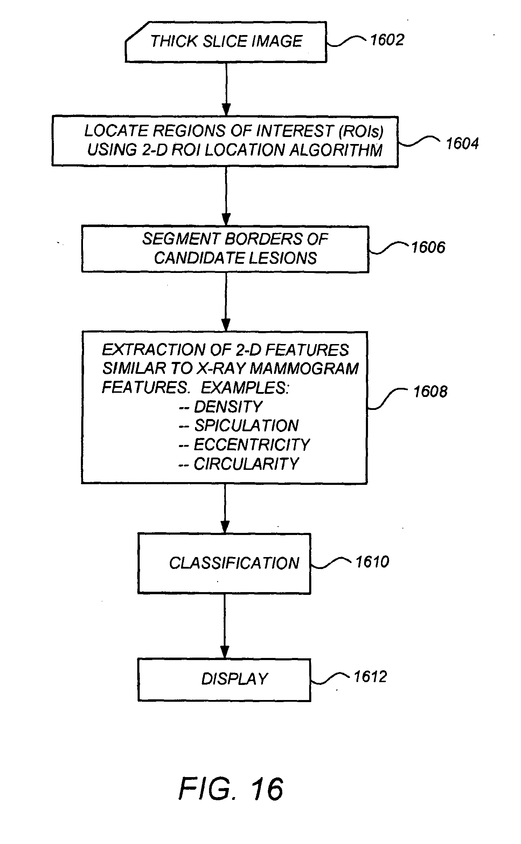

FIG. 16 illustrates steps for computer-aided diagnosis (CAD) of adjunctive ultrasound data according to a preferred embodiment. It has been found that the thick-slice ultrasound images described herein often possess substantially improved signal-to-noise ratios as compared to their component individual ultrasound slices, and are therefore more conducive to the use of two-dimensional CAD algorithms that have historically been used primarily for digitized x-ray mammograms. According to a preferred embodiment, on a two-dimensional thick-slice image provided at step 1602, regions of interest (ROIs) are located at step 1604 using a two-dimensional ROI location algorithm. Any of a variety of known two-dimensional ROI location algorithms can be used such as those described in: Singh, S. and Al-Mansoori. R., "Identification of Regions of Interest in Digital Mammograms," J. Intelligent Systems 10:2 (2000); U.S. Pat. 5,133,020; U.S. Pat. 5,491,627; U.S. Pat. 5,673,332; U.S. Pat. 5,790,690; U.S. Pat. 5,815,591; U.S. Pat. 6,035,056; U.S. Pat. 6,075,879; U.S. Pat. 6,198,838; U.S. Pat. 6,263,092; U.S. Pat. 6,278,793; U.S. Pat. 6,282,305; and U.S. Pat. 6,301,378, each of which is incorporated by reference herein. At step 1606, borders of candidate lesions are segmented at each ROI. Any of a variety of known two-dimensional segmentation algorithms can be used such as those described in: U.S. Pat. 5,671,294; U.S. Pat. 6,091,841; and Giger et. al., "Computer-Aided Diagnosis in Mammography," Handbook of Medical Imaging, Volume 2: Medical Image Processing and Analysis, Sonka and Fitzpatrick, eds., SPIE Press (2000) at Chapter 15 (pp. 915-1004), each of which is incorporated by reference herein.

At step 1608, two-dimensional features commonly associated with x-ray mammogram CAD algorithms are extracted. Such features include, for example, spiculation metrics, density/contrast metrics, eccentricity metrics, circularity metrics, border roughness metrics, location metrics (e.g., distance to chest wall, proximity to axilla, proximity to subareolar areas, etc.), edge shadow metrics, border metrics, texture metrics, size metrics, shape metrics, internal echo metrics, and tenting metrics. However, the scope of the preferred embodiments extends to a variety of other known two-dimensional features as well, such as those described in U.S. Pat. 5,815,591; and Giger et. al., "Computer-Aided Diagnosis in Mammography," supra at pp. 933-958. At step 1610, one or more classifier algorithms operate on the set of two-dimensional features extracted in step 1608. Any of a variety of classification methods known in the art may be used in accordance with the preferred embodiments, including linear classifier algorithms, quadratic classifier algorithms, K-nearest-neighbor classifier algorithms, decision tree classifier algorithms, and neural network classifier algorithms, as well as those described in the references cited above. At step 1612, based on one or more scores computed during the classification step, CAD outputs are displayed to draw the attention of the radiologist to certain locations in the thick-slice image and/or to provide other useful information.

It has been found that problematic breast cancer tumors exhibit certain acoustic behaviors that can be used to differentiate them from non-problematic lesions in some cases, such acoustic behaviors only being detectable using sonomammography, such acoustic behaviors being particularly apparent in the thick-slice images described herein. According to a preferred embodiment, features associated with these acoustic behaviors are extracted from the thick-slice images and classified in combination with the conventional features described supra to further improve both specificity and sensitivity in breast cancer screening.

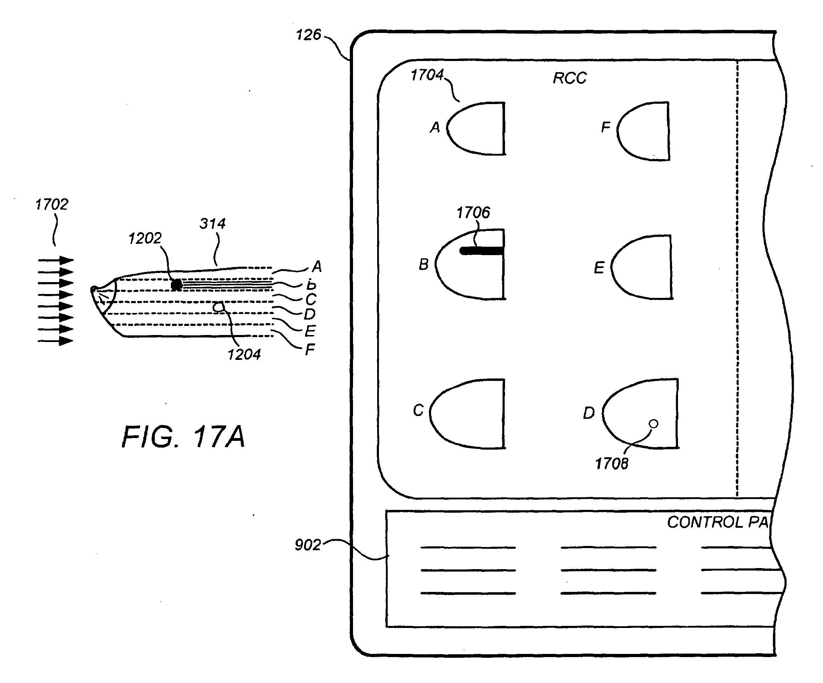

FIG. 17A illustrates a conceptual view of an adjunct ultrasound display comprising a plurality of thumbnail thick-slice images 1704, along with a side view of a corresponding breast 314. As in FIG. 12 supra, the breast 314 again contains a hard tumor 1202 in thick-slice region B and a liquid-filled cyst 1204 in thick-slice region D. In the example of FIG. 17A, it is presumed that the interrogating acoustic pulses 1702 are parallel to the CC plane and come from the front of the breast. The problematic hard tumor 1202 casts an acoustic shadow in the direction of the interrogating acoustic pulses 1702. However, because it is filled with a substantially acoustically transparent liquid, the liquid-filled cyst 1204 does not cast such a shadow. Indeed, the liquid-filled cyst often causes amplified readings at an edge where the acoustic interrogation signals exit the cyst, an effect known as posterior enhancement. Using the thick-slice image displays according to the preferred embodiments, the radiologist can readily see a shadow 1706 cast across thickslice image B, while no such shadow is cast across thick-slice image D.

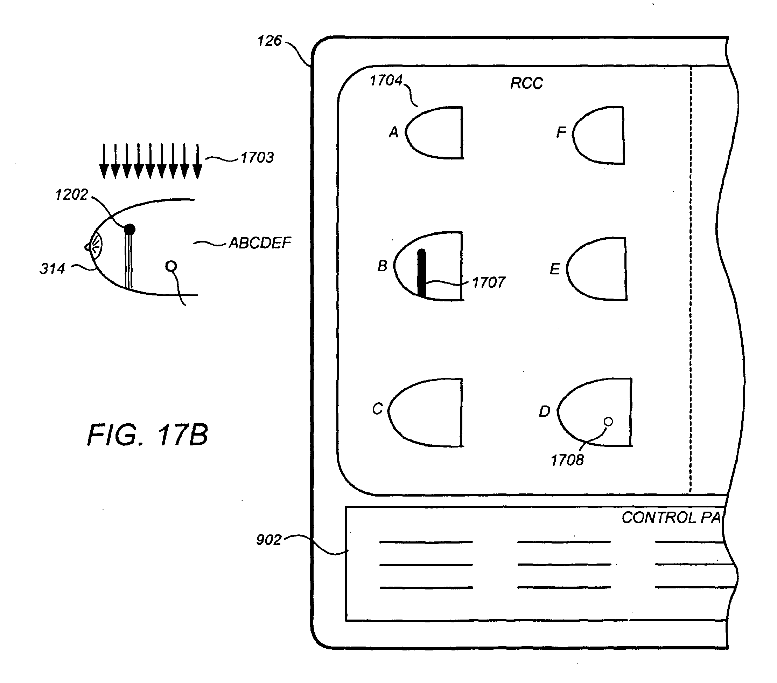

FIG. 17B illustrates a similar conceptual view of an adjunct ultrasound display comprising a plurality of thumbnail thick-slice images 1704, except that a top view of the corresponding breast 314 is shown. Interrogating pulses 1703 are introduced from the side of the compressed breast, which is often desirable to avoid nipple shadow effects. As indicated in FIG. 17B, a lateral shadow 1707 is cast across thick-slice image B perpendicular to the shadow of FIG. 17A. Again, however, no such shadow is cast across thick-slice image D and indeed there is a posterior enhancement effect. In view of the present disclosure, a person skilled in the art would readily be able to construct a CAD algorithm capable of extracting a lateral shadow feature exemplified in FIGS. 17A-17B, computing a lateral shadow metric corresponding thereto, and using such metric in a classifier algorithm to assist in classifying the suspect lesion.

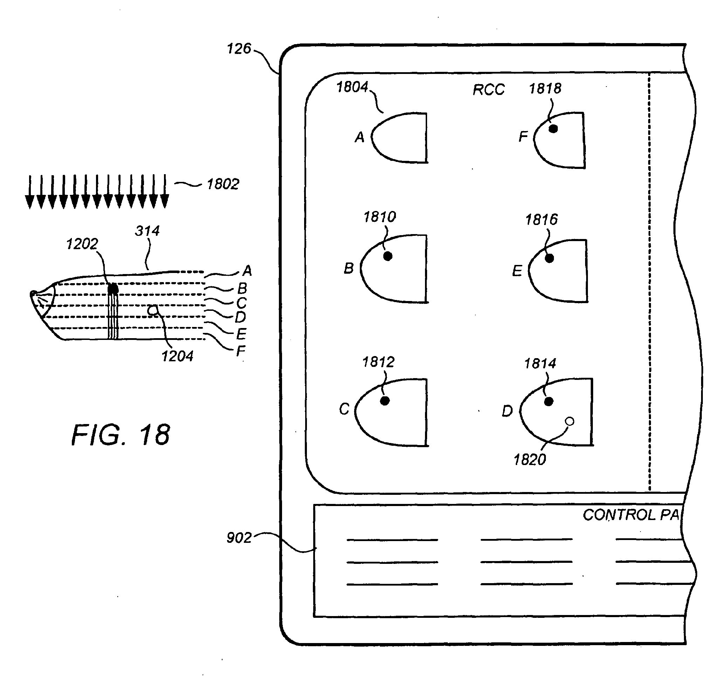

FIG. 18 illustrates a conceptual view of an adjunct ultrasound display and breast 314 similar to that of FIG. 17, except that interrogating acoustic pulses 1802 are introduced in planes perpendicular to the CC plain during the initial ultrasound scanning process. In this case, an acoustic shadow is cast across all lower slices C-F by the hard tumor 1202, while the cyst 1204 again produces no acoustic shadow (and indeed there is a posterior enhancement effect). Again by viewing the array of thick-slice images, with particular attention to the locations 1810-1818 shown in FIG. 18, the radiologist can readily see that some object lying in the B thick-slice region at location 1810 is acoustically opaque enough to cast a shadow down through the other slices. Moreover, in view of the present disclosure, a person skilled in the art would readily be able to construct a CAD algorithm capable of extracting this vertical shadow feature and use it to assist in classifying the suspect lesion.

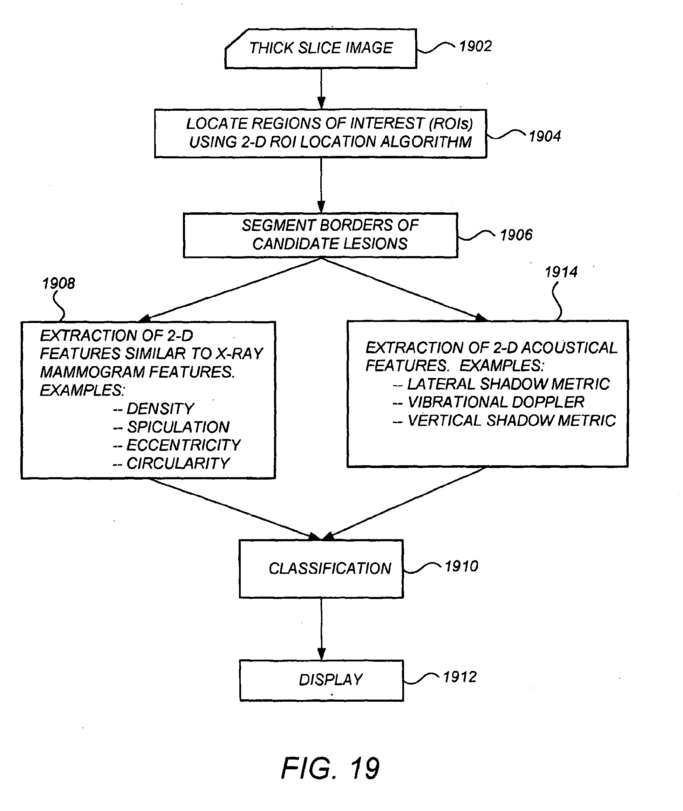

FIG. 19 illustrates steps for computer-aided diagnosis (CAD) of adjunctive ultrasound data according to a preferred embodiment. Thick-slice image acquisition (step 1902), ROI location (step 1904), and segmentation (step 1906), and extraction of conventional two-dimensional features (step 1908) proceed in a manner similar to steps 1602-1608 of FIG. 16, respectively. However, at step 1914, an additional step is carried out of extracting two-dimensional acoustical features including, for example, a lateral shadow metric, a vertical shadow metric, a VDI metric, and a posterior enhancement metric.

At step 1910, the results of both the steps 1908 and 1914 are processed by one or more classifier algorithms, and at step 1912, based on one or more scores computed during the classification step, CAD outputs are displayed. Notably, feature extraction results for a given thick-slice image may depend on the characteristics of other thick-slice images, as in the case of the vertical shadow metric described supra in FIG. 18.

Although two-dimensional CAD algorithms performed on the two-dimensional thick-slice images yield useful results, further useful results can be obtained by using the fact that thick-slice regions of the breast are indeed three dimensional in nature. Accordingly, useful three-dimensional thick-slice information can be obtained from a three-dimensional volumetric representation of the breast and used in conjunction with the two-dimensional information to even further increase specificity and sensitivity of the breast cancer screening process.

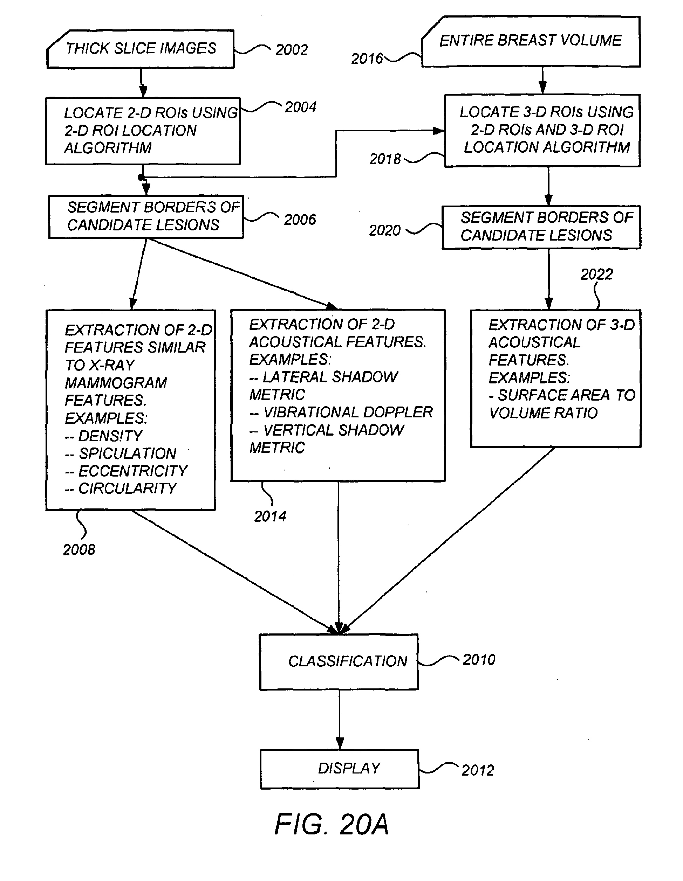

FIG. 20A illustrates steps for computer-aided diagnosis (CAD) of adjunctive ultrasound data according to a preferred embodiment. Thick-slice image acquisition (step 2002), ROI location (step 2004), segmentation (step 2006), extraction of conventional two-dimensional features (step 2008), and extraction of two-dimensional acoustical features (step 2014) proceed in a manner similar to steps 1902-1908 and 1914 of FIG. 19, respectively. Additionally, however, three-dimensional features are located and extracted from the three-dimensional volumetric representation of the breast volume in a manner that takes advantage of knowledge of the two-dimensional ROIs located in the thick-slice images at step 2004. At step 2106, the three-dimensional volumetric representation of the breast already exists by virtue of its computation from the initial raw ultrasound scans as part of the individual ultrasound slice generation process. At step 2018, ROIs are located in the thick-slice volume according to a method that takes advantage of the known two-dimensional ROI locations computed for the thick-slice images. For a given thick-slice image, there is a corresponding thick-slice volume contained in the three-dimensional volumetric representation of the breast. According to a preferred embodiment, the two-dimensional ROIs (x, z) found in that slick-slice image may be used as starting points in locating three-dimensional ROI locations (x, y, z) within that thick-slice volume. This can save computing time by reducing the ROI search to a one-dimensional search in the "y" direction for each (x, z) starting point.

At step 2020, the lesion borders are segmented at each ROI using a 3-dimensional segmentation algorithm. Any of a variety of three-dimensional segmentation algorithms can be used, including local thresholding algorithms, 3-D volume growing algorithms, or other algorithms as described, for example, in U.S. 6,317,617, which is incorporated by reference herein. At step 2022 three-dimensional acoustical features indicative of lesion suspiciousness are extracted for each candidate lesion. In one preferred embodiment, the three-dimensional features comprise one or more of the following features: a surface roughness metric for the candidate lesions, such as a surface area-to-volume ratio (higher ratios being associated with increased suspiciousness); a lesion compression metric that compares lesion depth in the direction of compression versus lesion height/width/area in a plane perpendicular to the direction of compression (if the lesion easily "squishes" in the direction of compression it is less suspicious); and volumetric echo uniformity metrics (a more uniform echo throughout the volume indicates less suspiciousness). Other three-dimensional features that can be used include three-dimensional spiculation metrics, three-dimensional density metrics, sphericity metrics, VDI metrics, shadow metrics, and other three-dimensional features described in the above references.

At step 2010, the results of all of the steps 2008, 2014, and 2022 are processed by one or more classifier algorithms. Extensions of known classification algorithms to data sets comprising combinations of two- and three-dimensional features could be carried out by a person skilled in the art without undue experimentation in view of the present disclosure. At step 2012, based on one or more scores computed during the classification step, CAD outputs are displayed.

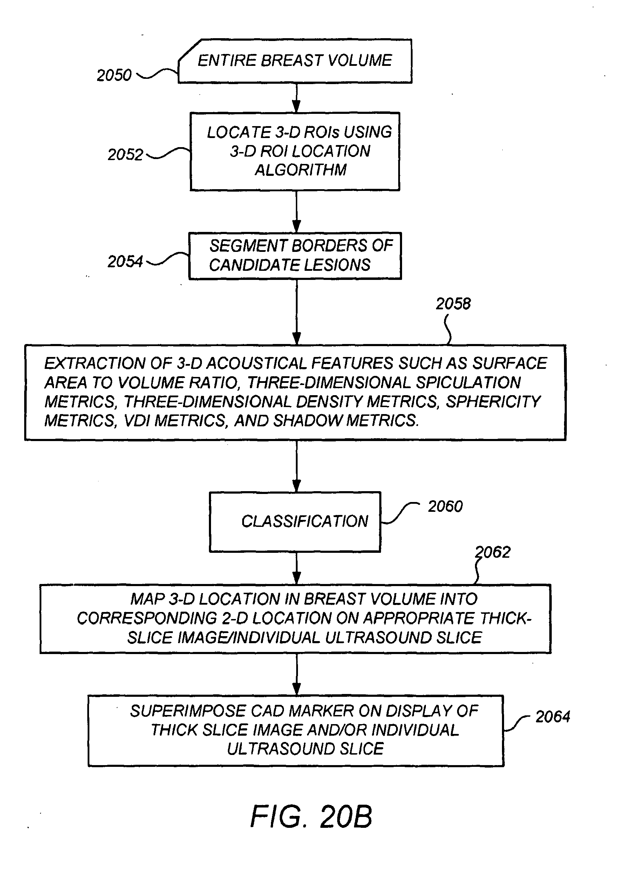

FIG. 20B illustrates steps for computer-aided diagnosis (CAD) of adjunctive ultrasound data according to a preferred embodiment, in which substantially all CAD processing is formed on the entire three-dimensional breast volume without regard to the thick-slice volumes. At step 2050 the entire breast volume exists by virtue of the three-dimensional volumetric representation of the breast computed from the initial raw ultrasound scans. At step 2052 ROI locations in the breast volume are determined according to a three-dimensional ROI detection algorithm. In one preferred embodiment, the three-dimensional ROI detection algorithm is similar to that used in step 2018 of FIG. 20A, except that it is applied to the whole breast volume at once rather than to separate thick-slice volumes. In another preferred embodiment, an ROI detection algorithm based on a three-dimensional Difference-of-Gaussians ("D.O.G.") operator is used, comprising an extension into three dimensions of the two-dimensional D.O.G. operator described, for example, at page 264 of Russ, The Image Processing Handbook, 3rd Edition, CRC Press/IEEE Press (1998).

At step 2054 the borders of candidate lesions are segmented at each ROI location using a three-dimensional segmentation algorithm. In one preferred embodiment, the three-dimensional segmentation algorithm is similar to that used in step 2020 of FIG. 20A. In another preferred embodiment, a three-dimensional region-growing algorithm is used similar to that described, for example, in the Handbook of Medical Imaging, Volume 2, supra at pp. 98-101. At step 2058, one or more three-dimensional features are extracted. The three-dimensional features may include three-dimensional spiculation metrics, three-dimensional density metrics, sphericity metrics, VDI metrics, shadow metrics, edge shadow metrics, border metrics, texture metrics, contrast metrics, size metrics, shape metrics, internal echo metrics, surface area-to-volume metrics, and other three-dimensional metrics.

At step 2060, any of a variety of known three-dimensional classifier algorithms is applied to the three-dimensional features to determine one or more metrics of suspiciousness therefrom. At step 2062, the locations of lesions found to require user attention are mapped from the breast volume into their corresponding thick-slice image. At step 2064, a marker for each such is placed on the relevant two-dimensional thick-slice image and/or the relevant individual ultrasound slice(s) on the user display.

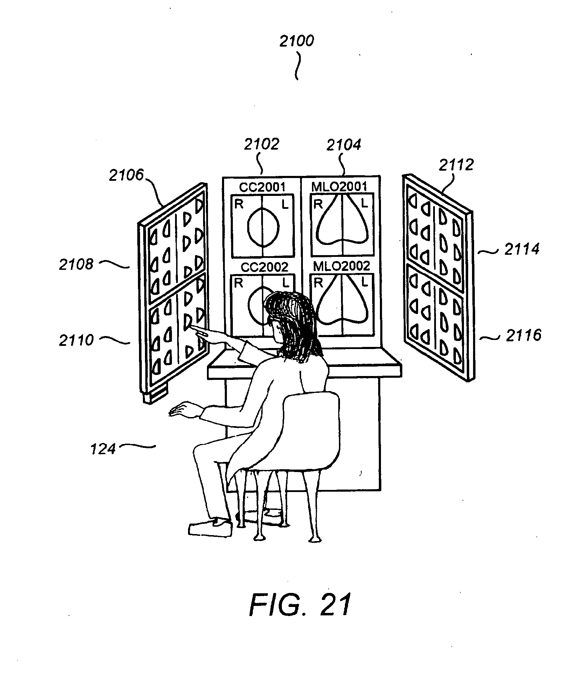

FIG. 21 illustrates an x-ray mammogram viewing station with adjunctive ultrasound displays according to a preferred embodiment in which the radiologist 124 is permitted to view the current x-ray mammogram against last year's results (or results from another previous time). An x-ray mammogram viewing station shows past and current CC x-ray mammogram views 2102, and adjacent thereto on the adjunct display 2106 is shown the corresponding past CC thick slices 2108 and current CC thick slices 2110. Likewise, the x-ray mammogram viewing station shows past and current MLO x-ray mammogram views 2104, and adjacent thereto on the adjunct display 2112 is shown the corresponding past MLO thick slices 2114 and current MLO thick slices 2116. Preferably, any given CC or MLO thick slice from any time period is positioned not more than 30 inches from its corresponding x-ray mammogram view from that time period or any other time period. Although data for only two years (or time periods) is shown in the example of FIG. 21, it is to be appreciated that the simultaneous display of any number of years (or time periods) of thick-slice images near their associated x-ray mammograms is within the scope of the preferred embodiments.

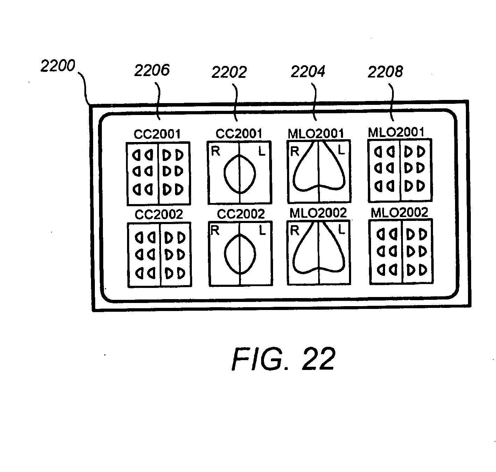

FIG. 22 illustrates an x-ray mammogram viewing monitor 2200 with adjunctive ultrasound displays according to another preferred embodiment, wherein all data including the x-ray mammograms are digital in form, and wherein the adjunctive ultrasound data is displayed near the x-ray mammogram data on a common monitor. Past and present CC thick slices 2206 are displayed near past and present CC x-ray mammogram views 2202, and past and present MLO thick slices 2208 are displayed near past and present MLO x-ray mammogram views 2204. The monitor 2200 may be, for example, a large-screen high-definition display such as an HDTV display, a high-definition projection monitor, or the like that is large enough to display the image data with sufficient resolution for breast cancer screening purposes.

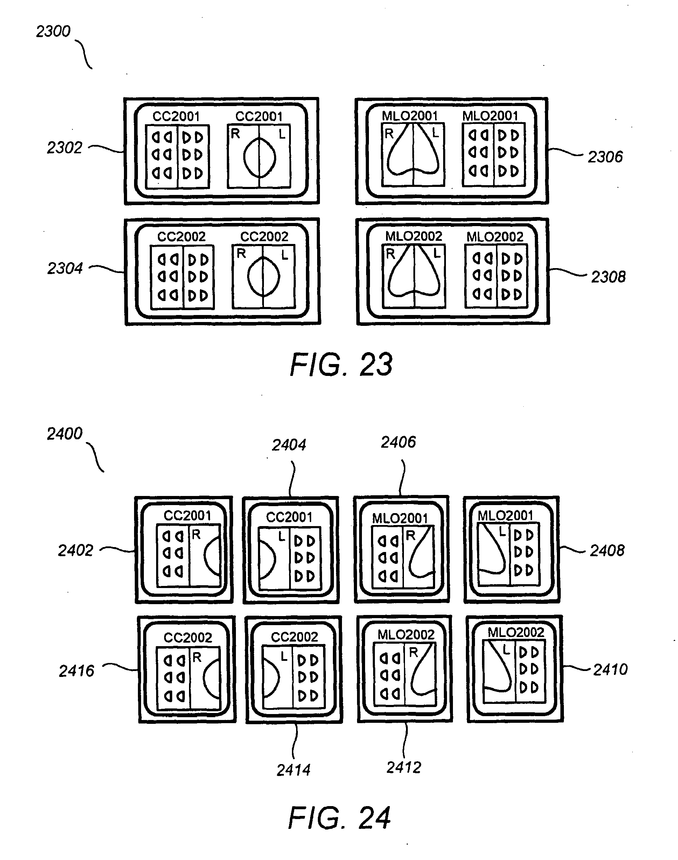

FIGS. 23-24 illustrate x-ray mammogram viewing monitors with adjunctive ultrasound displays according to other preferred embodiments, wherein different combinations of x-ray mammogram and adjunctive ultrasound data are displayed on different monitors placed close to each other. It is to be appreciated that the examples of FIGS. 22-24 are by no means limiting, and that many other combinations of monitor content and positioning are within the scope of the preferred embodiments.

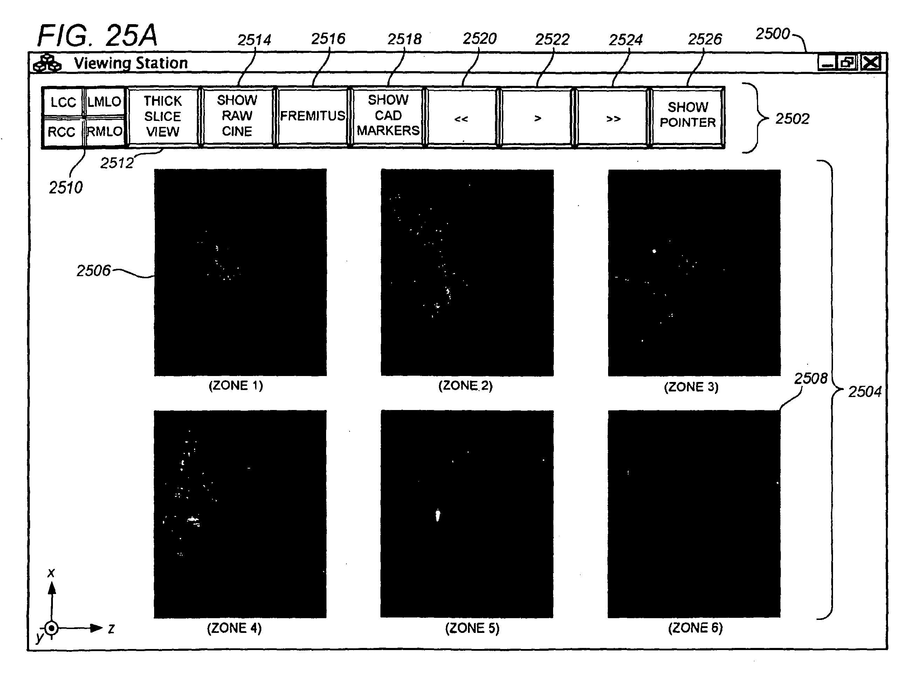

FIG. 25A illustrates an adjunct ultrasound display 2500 according to a preferred embodiment, which may be used alternatively or in conjunction with the adjunct ultrasound displays described supra. The adjunct ultrasound display 2500 may be implemented, for example, on a Microsoft Windows® 2000 operating system platform using Microsoft Visual C++. Adjunct ultrasound display 2500 is designed to interactively and intuitively display image information relating to a breast ultrasound scan. All computationally intensive computing algorithms (CAD, fremitus, etc.) are presumed to have already taken place in non-real time prior to invocation of the viewing program, although to scope of the preferred embodiments is not so limited. The precomputed images are stored, for example, in BMP format on a FAT32 or NFS file system and are associated by filename, with a first portion of the filename containing patient and scan data information and a second portion of the filename identifying the type of ultrasound image stored.

Adjunct ultrasound display 2500 generally has the look and feel of a conventional Windows® application. The user supplies inputs using a conventional personal computer keyboard and mouse (not shown). Context-sensitive soft buttons 2502 are provided that are selectable in a point-and-click fashion, and include a breast view selection button 2510, a thick slice view button 2512, a raw cine view button 2514, a fremitus button 2516, a CAD button 2518, a cine reverse button 2520, a cine play/pause button 2522, a cine forward button 2524, and a pointer show/hide button 2526. For clarity of description, a set of reference axes is displayed in the lower left hand corner of FIGS.25A-25F, which may not appear on the actual output screen. For the purposes of describing the particular images of FIGS. 25A-25F, the "x" direction represents the lateral (left-right) direction across the chest, the "y" direction represents the vertical (head-toe) direction, and the "z" direction represents the anterior-posterior direction (pointing outward from the chest wall).

In a first view, adjunct ultrasound display 2500 shows thick-slice thumbnails 2504 as illustrated in FIG. 25A. A first thick-slice thumbnail image 2506 represents the uppermost thick-slice region of the breast (see region "A" of FIG. 9A) that is denoted zone 1, the thick-slice thumbnails proceeding in order through a final thick-slice thumbnail image 2508 representing the lowermost thick-slice region (see region "F" of FIG. 9A). Depending on the number of thick-slice regions in the breast, the thick-slice thumbnail images 2504 can actually be quite large, approaching several inches in size. Indeed, in one preferred embodiment in which there is a large enough display monitor, the thumbnail images can actually be full-scale, although this preferred embodiment would be more expensive than if smaller thumbnails thick-slice images are displayed.

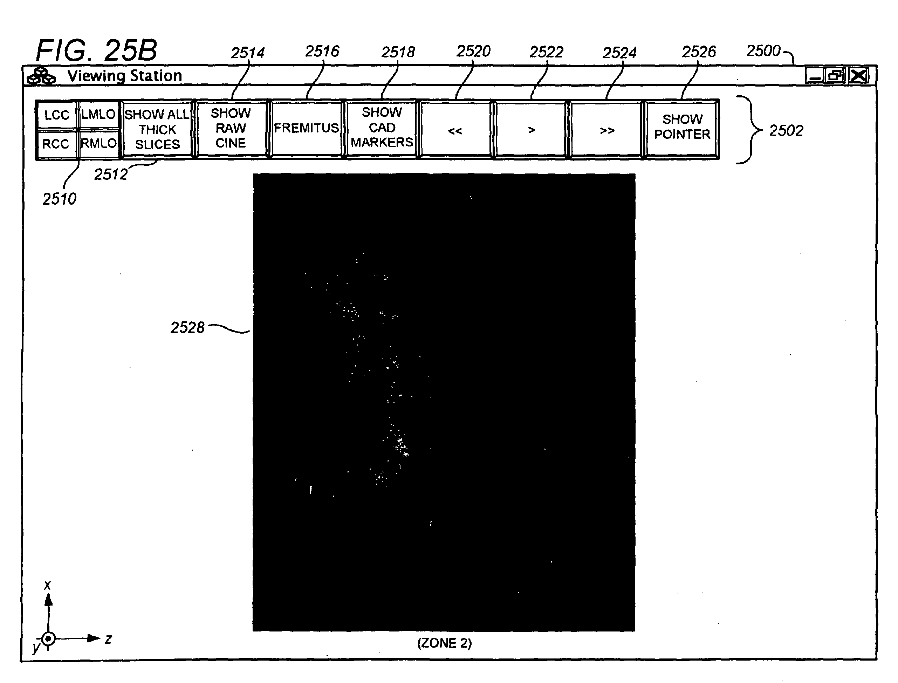

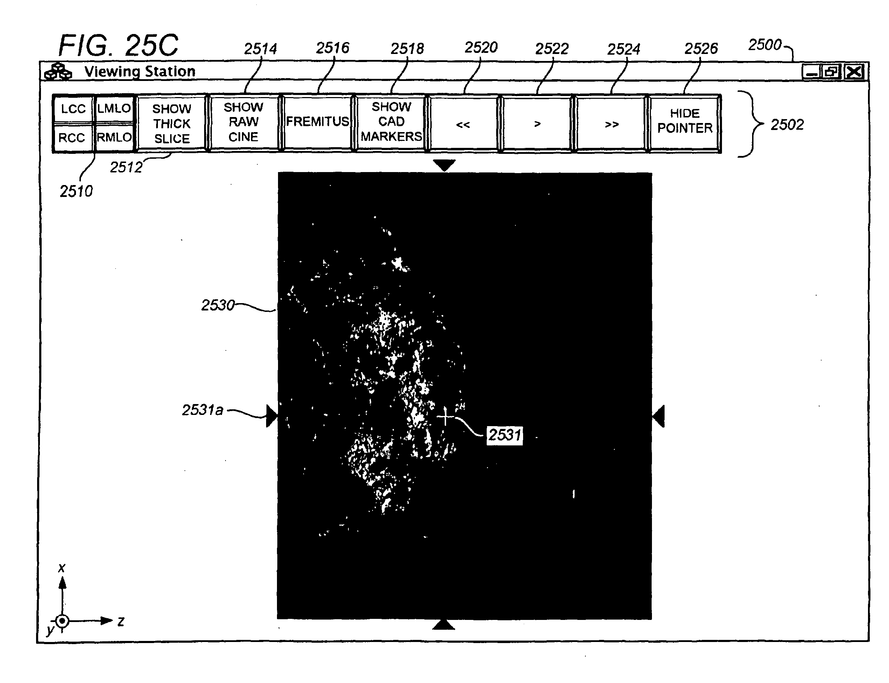

The soft buttons 2502 are context sensitive, displaying options appropriate to the present state of the adjunct ultrasound display 2500. Generally speaking, the user has the option of pressing one or more of the soft buttons 2502, and/or one or more of the onscreen images being displayed and/or a particular location thereon, to proceed to the desired display of interest. The breast view selection button 2510, which is actually four smaller buttons, enters the user into one of the LCC, RCC, LMLO, or RMLO view. It is to be appreciated that while FIGS. 25A-25F show data for the LCC or RCC view, equivalent display options for the LMLO/RMLO view are also provided and are within the scope of the preferred embodiments. When the view of FIG. 25A is shown, the thick slice view button 2512 reads "THICK SLICE VIEW" and, if the user clicks on one of the thick-slice images, a single full-scale thick-slice image 2528 of FIG. 25B appears. When the view of FIG. 25B is showing, the thick slice view button 2512 then reads SHOW ALL THICK SLICES and, if pressed, returns the display to the thick-slice thumbnail display of FIG. 25A. Also when the view of FIG. 25A is shown, if the cine play/pause button 2522 is pressed, a cine presentation of individual ultrasound slices parallel to the CC plane (x-z plane) is presented, one such individual ultrasound slice 2530 being shown in FIG. 25C, the cine presentation beginning at the uppermost slice of the breast volume and proceeding to the lowermost slice.

When the view of FIG. 25B is showing and the cine play/pause button 2522 is pressed, a cine presentation of individual ultrasound slices corresponding to the thick-slice image being displayed (zone 2 for the particular example of FIG. 25B) is also presented as in FIG. 25C, except that the cine presentation begins at the uppermost slice of the zone 2 thick-slice volume and proceeds to the lowermost slice of that thick-slice volume. With reference to FIG. 25C, the user uses the cine control buttons 2520-2524 to control the playback. While in forward play mode, the play/pause button 2522 indicates a PAUSE icon (not shown), and while in pause mode as indicated in FIG. 25C, the play/pause button 2522 indicates a forward play (">") symbol.

The view of FIG. 25C is a paused cine view of individual ultrasound slices that are computed from a 3-D volumetric representation of the breast from raw ultrasound slices. The raw ultrasound slices are themselves taken in planes parallel to the y-z plane as the ultrasound probe is swept across the breast in a lateral (right-to-left) direction, which corresponds to the "x" direction in the image of FIG. 25C.

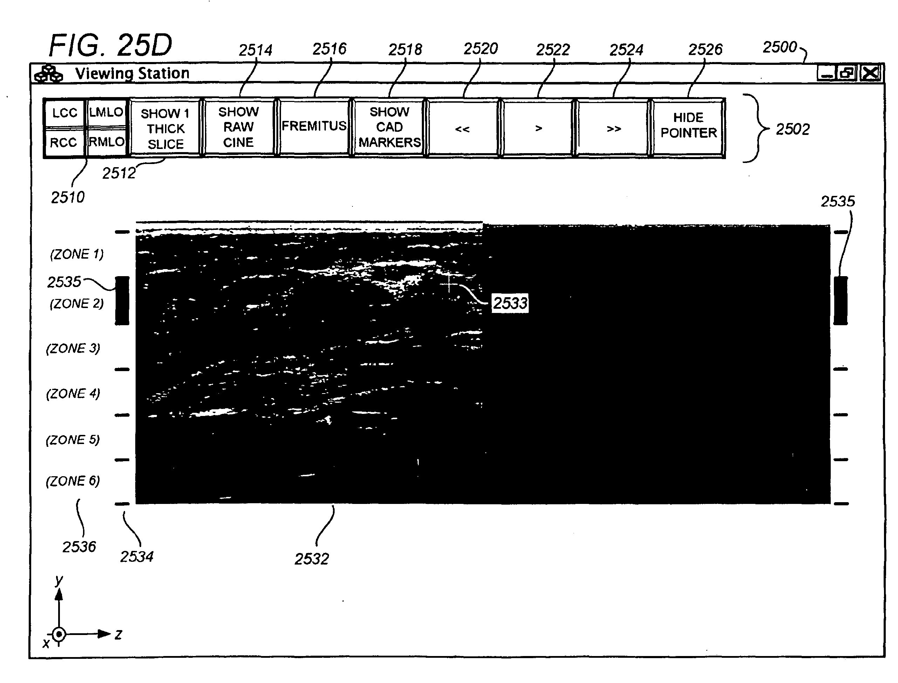

FIG. 25D illustrates a raw B-mode frame 2532. This view can be invoked in several ways. The raw B-mode frame 2532 represents one frame of a cine presentation of raw B-mode frames that can be controlled by the cine control buttons 2520-2524 when the view of FIG. 25D is being displayed. If the view of FIG. 25A or FIG. 25B is showing and the user selects the raw cine view button 2514, the view of FIG. 25D will be entered and will proceed to display a cine presentation of all raw B-mode frames as gathered during the raw ultrasound scanning process. If the view of FIG. 25C is showing and the user clicks anywhere on the image, the view of FIG. 25D appears for a plane corresponding to the "x" location of the cursor when clicked. As with the other images shown in FIGS. 25A-25F, the raw B-mode image 2532 is a "stitched" image comprising two halves corresponding to different passes of the ultrasound probe over the breast. In alternative preferred embodiments in which a wider probe is used, the need for multiple probe passes and image stitching would not be necessary. Zone markers 2534 are provided on the display of FIG. 25D that demarcate the different thick-slice volumes (zones) 1-6 of the breast, as well as a zone indicator 2535 that indicates the zone that was being displayed if the prior view was of FIG. 25B or FIG. 25C.

According to a preferred embodiment, a convenient location-mapping feature is provided to assist the user in switching back and forth between the views of FIGS. 25C and 25D. Starting, for example, in the view of FIG. 25D, which shows a given raw B-mode frame 2532 taken at a particular lateral location "x1", the user can click the cursor on a given point (y1, z1) on that image. Responsive to that click, the view of FIG. 25C is presented showing the individual ultrasound slice that corresponds to the vertical "y1" position. Furthermore, a pointer 2531 appears on the image of FIG. 25C at (x1, z1). This is especially useful in correlating breast locations where the z-axis scales of FIGS. 25C-25D are not the same. Likewise, starting, for example, on the view of FIG. 25C, which shows a given individual ultrasound slice 2530 computed for a particular vertical location "yl", the user can click the cursor on a given point (x1, z1) in that image. Responsive to that click, the view of FIG. 25D is presented showing the raw B-mode ultrasound frame that corresponds to the lateral position "xl" of that point, and a pointer 2533 appears on the image of FIG. 25D at (y1, z1). The pointer can be removed from the current view by selecting the HIDE POINTER button 2526.

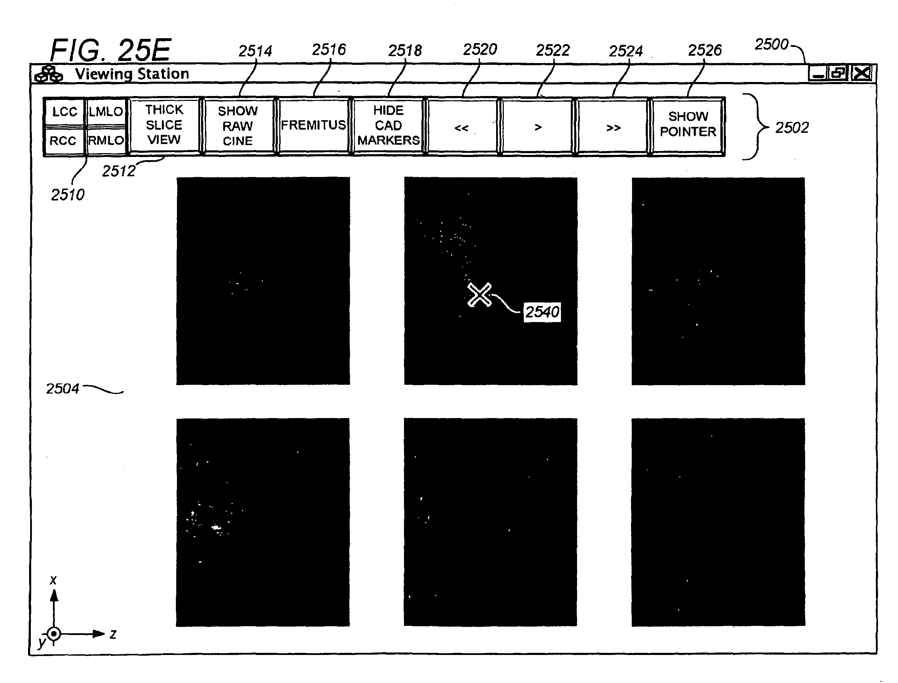

The display of FIG. 25D also corresponds to a single frame of a cine loop presentation of the raw B-mode data that was acquired. Accordingly, the display of FIG. 25D is responsive to activation of any of the cine controls 2520-2524 and will proceed to display the raw B-mode ultrasound frames of the initial scans taken parallel to the y-z plane as the probe was translated in the x direction. If the user presses the thick slice view button 2512 when the view of FIG. 25D is displayed, the display will revert to the thick-slice display of FIG. 25B for the zone indicated by the zone markers 2535. FIG. 25E illustrates the adjunct ultrasound display 2500 showing the thumbnail thick slice views 2504 with a superimposed CAD marker 2540. This view is enabled by pressing the CAD button 2518 when the display of FIG. 25A is active. After being pressed to make the CAD markers appear, the CAD button 2518 reads HIDE CAD MARKERS, and the CAD markers are removed if the CAD button 2518 is pressed again.

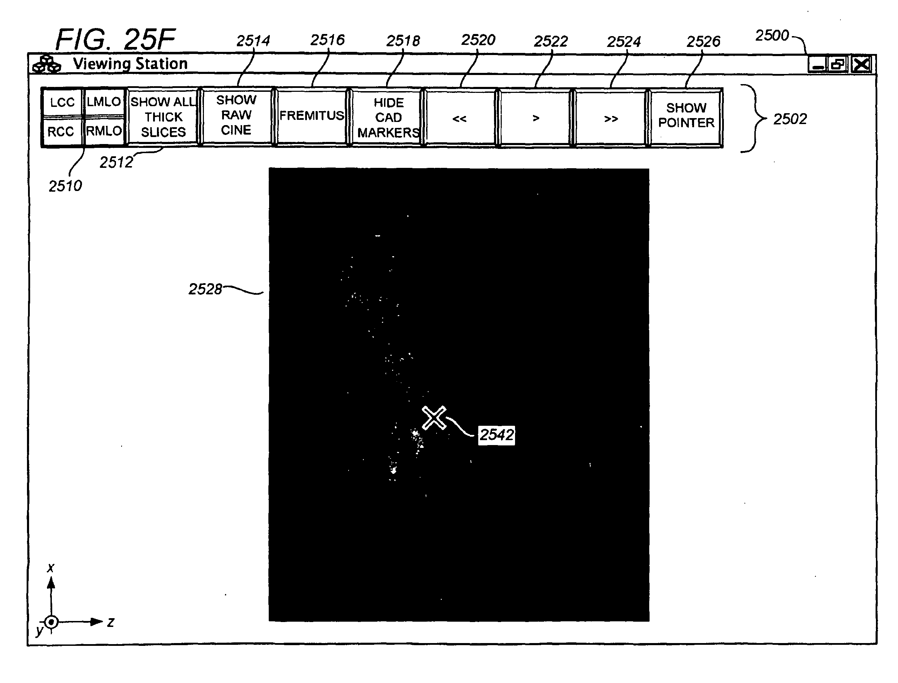

FIG. 25F illustrates the adjunct ultrasound display 2500 showing the single thick slice view 2528 with a superimposed CAD marker 2542, which is enabled by pressing the CAD button 2518 when the display of FIG. 25B is active. In one preferred embodiment, a star or cross-hair symbol is used to denote a suspicious mass, while triangles are drawn around suspected microcalcifications.

Although described in the preferred embodiments supra in terms of a computer display for viewing the adjunctive ultrasound data, it is to be appreciated that any of a wide variety of output modalities may be used without departing from the scope of the preferred embodiments, including paper printouts, film printouts, and holographic displays. For these output modalities, a manual indexing system can be used to order the images, with the thumbnail thick-slice image views serving as a "table of contents" for the book of images. All of the thick-slice images should be provided in full scale, preferably with the CAD results superimposed thereon, with ordered subsets (e.g., every kth member) of the raw B-mode images and the component ultrasound slices also being provided. In view of the large amount of digital data associated with each adjunctive ultrasound scan session for each patient, which can extend into the gigabyte range if uncompressed, it may be desirable to archive the data in such a hardcopy format. Alternatively or in conjunction therewith, the digital data can be compressed and digitally archived. One particularly desirable compression algorithm is the JPG (Joint Photographic Experts Group) algorithm, which has been found to work very efficiently with ultrasound images. Using a JPG algorithm, a data compression ratio of 20:1 (as compared to an original uncompressed BMP format) or better can be achieved with negligible loss of information.

As known in the art, many conventional ultrasound machines measure acoustic reflectivity of internal tissues at a higher bitwise precision (e.g., 14 bits per pixel) than is displayed on the output display monitor (e.g., 8 bits per pixel). In the examples herein, 14 bits are assumed for the higher precision gray scale value and 8 bits are assumed for the lower precision gray scale value, it being understood that these are nonlimiting examples. After the higher-precision measurements are acquired, conventional ultrasound machines perform a lossy gray scale compression algorithm that maps the higher precision 14-bit pixel values into the lower-precision 8-bit pixel values. Generally speaking, conventional ultrasound systems comprise high-speed internal hardware that performs the gray scale compression in real time prior to display, printout, storage, or other processing of the ultrasound image data by "downstream" systems. One reason the gray scale compression algorithm is performed at "upstream" points in the data flow process is to achieve better cost efficiency, it being more cost intensive to carry along an entire 14 bits of precision in the data stream that cannot be appreciated on an 8 bit display.

The simplest gray scale compression algorithm, of course, is a linear mapping that simply chops off the 6 least significant bits of a 14-bit pixel value to achieve an 8-bit pixel value. Instead, however, most conventional ultrasound systems perform a nonlinear mapping designed to enhance image contrast on the output display while also reducing image precision from 14 bits to 8 bits. Unfortunately, in the output images resulting from these conventional nonlinear mappings, it is often difficult to perceive microcalcifications, which are very small (e.g., 0.1 mm - 0.3 mm diameter), very hard, and very acoustically reflective objects known to be a local by-product of some breast tumors, and which would be desirable to detect in ultrasound images.

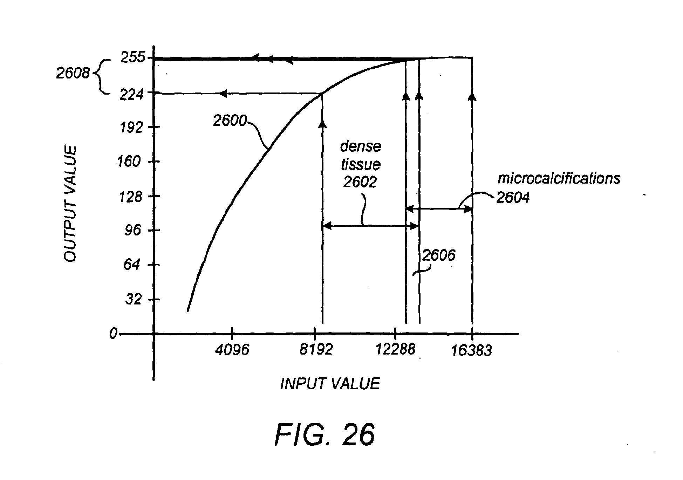

FIG. 26 illustrates a conceptual plot of a conventional remapping curve 2600 according to a conventional gray scale compression algorithm. Each 14-bit pixel value is remapped through the remapping curve 2600 into an 8-bit pixel value by high-speed upstream hardware components of the ultrasound system prior to display, printout, storage, or other downstream data processing. The remapping curve 2600 is commonly a quasilogarithmic curve designed to maximize the contrast effect between denser areas and less dense areas of the breast. As indicated in FIG. 26, dense breast tissue usually has high acoustic reflectivities falling in the range 2602 of FIG. 26, while typical microcalcifications usually have even higher acoustic reflectivities falling in the range 2604 of FIG. 26, there being some degree of overlap 2606 between the two ranges. Unfortunately, as indicated in FIG. 26, the microcalcifications and the dense breast tissues tend to map into a very high saturation range 2608 of output levels on the display monitor. Accordingly, in view of their small size, these microcalcifications are often not clearly visible on the output display, especially when surrounded by dense breast tissue.

According to a preferred embodiment, a computer-assisted microcalcification-highlighting algorithm is performed whose output is optionally displayed by the user in an overlay fashion on the thick-slice images and/or individual ultrasound slices. In this preferred embodiment, it is presumed that the scanning ultrasound system has already performed the 14-to-8 bit compression during the scanning process using the conventional remapping curve 2600 of FIG. 26. First, the 8-bit image is thresholded on a pixel-by-pixel basis using a predetermined threshold value Tmc, the threshold value Tmc being selected to separate the microcalcifications from the dense breast tissue and other breast tissue, in a statistically reliable manner. In one preferred embodiment, the threshold value Tmc is selected to lie in the middle of the overlap region 2606 shown in FIG. 26, which can be empirically determined from a population of sample images. For those pixels lying above the threshold Tmc, simple region-growing algorithms are performed in which neighboring above-threshold pixels are clustered together. Any cluster having an average diameter greater than a predetermined size threshold related to microcalcification size is discarded. In one preferred embodiment, this predetermined size threshold is about 1 mm. The remaining clusters constitute the locations that will be highlighted on the microcalcification-enhanced overlay display. When this feature is invoked by the user, these clusters are displayed in a highly noticeable color, e.g. a bright pink or blue, on an otherwise black-and-white display of the thick-slice image or individual ultrasound slice.

According to another preferred embodiment, referring back to the overall adjunctive ultrasound system of FIG. 1, the scanning ultrasound system 118 is configured to simply pass along the entire 14-bit pixel values to the adjunctive ultrasound server 106 without compressing the data to 8 bits. Substantially all processing is performed on 14-bit data instead of 8 bit data. In this case, the threshold Tmc can be set to a very high value near the maximum 14-bit value for more statistically reliable separation of microcalcifications from dense breast tissue. The adjunctive ultrasound display, of course, will usually have only 8 bits of dynamic range. The user may elect, either directly through keyboard inputs or indirectly through brightness/contrast controls, the type of 14-to-8 bit compression to be used prior to display. Again, it is to be appreciated that the particular use.

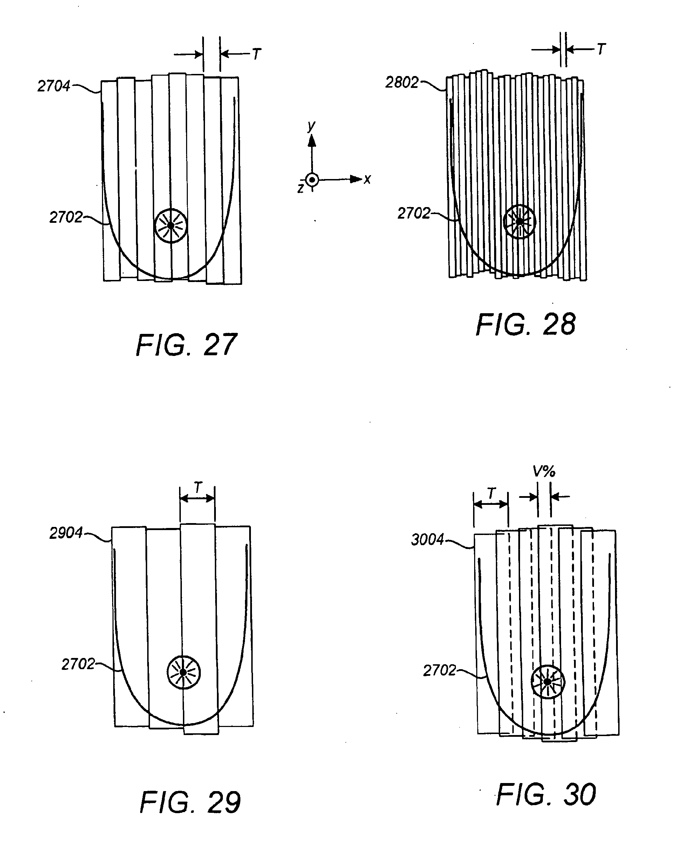

FIGS. 27-31 illustrate conceptual diagrams of thick-slice regions as superimposed onto a breast 2702 for the purpose of further describing the nature of the thick-slice regions and images according to the preferred embodiments. In the examples of FIGS. 27-31, the thick-slice regions are parallel to the standard MLO x-ray mammogram view plane, i.e., the y-z plane in FIG. 27, it being understood that similar descriptions apply to CC thick-slice regions, i.e., the x-z plane in FIG. 27, or to other standard or custom x-ray mammogram view planes. In the embodiment of FIG. 27, thick-slice regions 2704 are non-overlapping and have a uniform thickness T. Preferably, the thickness T lies between 0.5 cm to 1.2 cm for capturing suspicious masses within that size range. For a typical case in which there are perhaps 400 individual ultrasound slices taken for the entire breast volume, each of the eight (8) thick-slice images are generated by mathematically combining (e.g., arithmetically averaging) about 50 individual ultrasound slices.

An intrinsic benefit of thick-slice generation according to the preferred embodiments is an inherent spatial filtering effect that removes noise while preserving structures dimensioned on the order of the slice thickness T or larger. As indicated in FIG. 28, the thickness T of the thick-slice regions can be reduced to increase sensitivity to smaller lesions. As indicated in FIG. 29, the thickness T of the thick-slice regions can be increased to further reduce noise effects and to capture only the larger lesions. Thus, the slice thickness T also serves as a spatial filtering parameter in addition to dictating the number of thick slices requiring display. While the above stated slice thickness range of 5 mm - 12 mm is generally preferred, it has been found that useful results can be obtained with slice thicknesses ranging from 2 mm- 20 mm.

Whereas the thick-slice regions of FIGS. 27-29 are non-overlapping in space, FIG. 30 illustrates overlapping thick-slice regions 3004. In this embodiment, each thickslice region is quite thick (e.g., T = 20 mm), but there is a spatial overlap among adjacent slices (e.g., V = 40% overlap on each side) so that a sufficient number of images are available for viewing by the radiologist. Accordingly, the overlapping thick-slice embodiment of FIG. 30 offers the advantages of very thick thick-slice regions, while avoiding the limited visual data problems associated with very thick thick-slice regions when they are non-overlapping, as in FIG. 29.

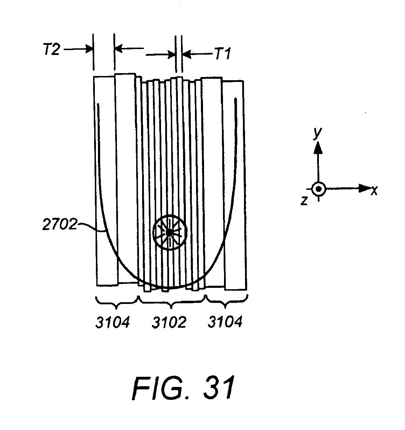

FIG. 31 illustrates a conceptual diagram of thick-slice regions as superimposed onto a breast 2702, wherein a first set of thick-slice regions 3102 near the center plane of the breast have a first thickness T1, and wherein second set of thick-slice regions 3104 near the outer planes of the breast have a second thickness T2 > T1. In this preferred embodiment, the thinner inner thick-slice regions 3102 provide for increased spatial sensitivity in the inner planes of the breast, where cancerous lesions are more likely to occur. The thicker outer thick-slice regions 3104 provide the ability to reduce the number images that need to be viewed by the radiologist on the adjunctive ultrasound output display, the reduced sensitivity being acceptable because cancerous lesions are less common near the outer planes of the breast. Also within the scope of the preferred embodiments are thick-slice regions of more than two different thicknesses, and thick-slice regions that both (i) spatially overlap each other, and (ii) are of two or more different thicknesses.

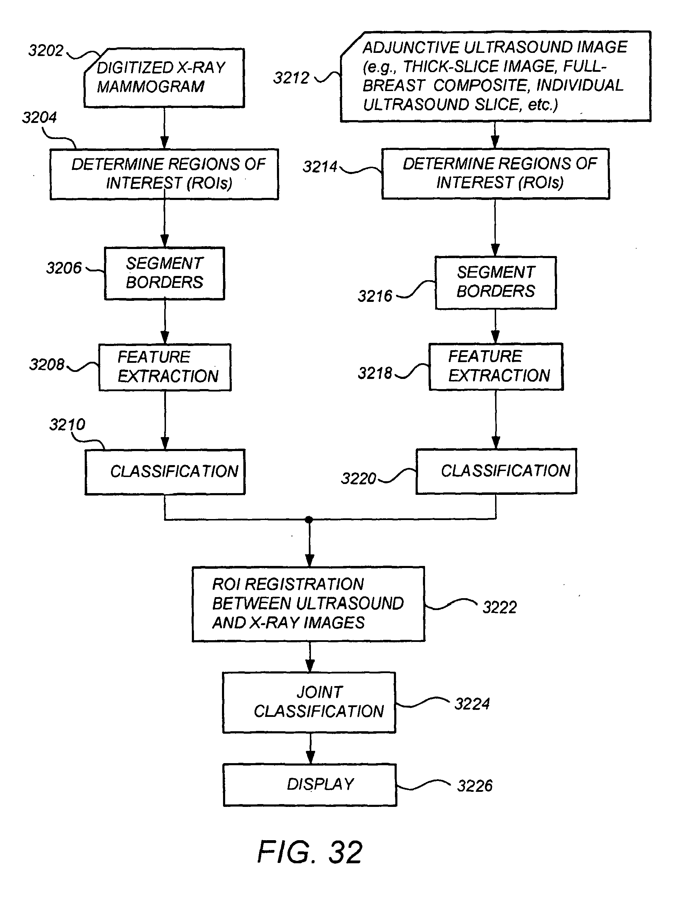

FIG. 32 illustrates steps for joint computer-aided diagnosis of digitized x-ray mammogram data and adjunctive ultrasound data according to a preferred embodiment. Using known methods such as those described in U.S. Pat. 5,133,020, supra, x-ray CAD algorithms are performed including the steps of acquiring a digitized x-ray mammogram (step 3202), determining regions of interest (ROIs) (step 3204), segmenting (step 3206), extracting features indicative of lesion suspiciousness (step 3208), and classification (step 3210). A scalar or vector index of suspiciousness ("score") results from the x-ray CAD algorithm for each ROI. In a separate process, CAD algorithms are performed on adjunctive ultrasound image data (e.g., thick-slice image, full-breast composite, individual ultrasound slice, etc.) including the steps of acquiring the adjunctive ultrasound image data (step 3212), determining ROIs (step 3214), segmenting (step 3216), extracting features indicative of lesion suspiciousness (step 3218), and classification (step 3220). The steps 3212-3220 are carried out, for example, according to the adjunctive ultrasound CAD algorithms described supra with respect to FIGS. 19 and 20. The adjunctive ultrasound CAD algorithm also yields a scalar or vector "score" corresponding to lesion suspiciousness for each ROI.

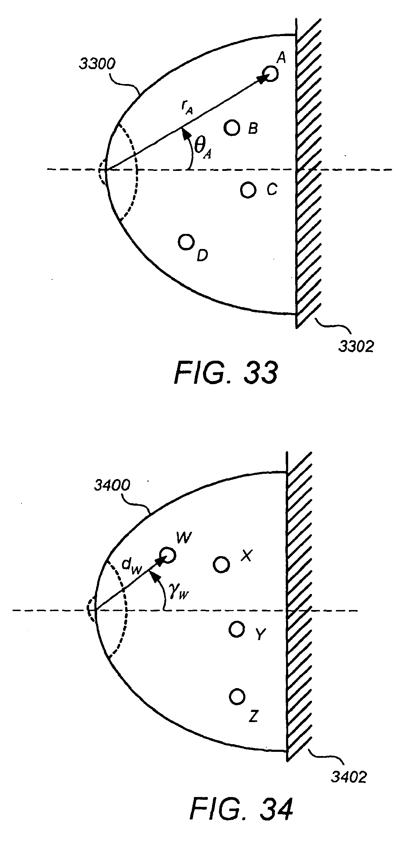

At step 3222, regions of interest (ROIs) in the x-ray mammogram and adjunctive ultrasound images are registered with each other. Preferably, the x-ray and ultrasound ROIs are registered without the need for traditional registration of the entire x-ray and ultrasound images, which can be complex and time-consuming. Rather, a simple but statistically reliable lesion-centric registration process using nipple distance information, or using a combination of nipple distance information and nipple angle information, is used to match corresponding regions of interest in the x-ray mammogram view and the adjunctive ultrasound views, as is described further infra with respect to FIGS. 33-36.

At step 3224, each ROI is jointly classified using the scores from both the x-ray CAD process and the adjunctive ultrasound CAD process. By way of example, if the x-ray CAD algorithm and the adjunctive ultrasound CAD algorithm each yield a score that is a single, scalar metric of lesion suspiciousness (e.g., Sx and Su, respectively), these metrics can be added at step 3224 to yield an overall joint score Sjoint = Sx + Su for that ROI. If a given ROI in the x-ray mammogram does not map into a corresponding ROI in the adjunctive ultrasound view, the value Su is set to zero by default, and the joint score Sjoint for that ROI is simply equal to Sx. Likewise, if a given ROI in the adjunctive ultrasound view does not map into a corresponding ROI in the x-ray mammogram, the value Sx is set to zero by default, and the joint score Sjoint for that ROI is simply equal to Su. In another preferred embodiment, the joint score can be a weighted combination of the individual scores, e.g., Sjoint = aSx + bSu. At step 3226, the x-ray mammogram and/or the adjunctive ultrasound images are displayed in a manner that communicates lesion suspiciousness according to the joint score Sjoint. It is to be appreciated that the examples given here are by way of illustration only, and that many different methods for combining the scalar or vector scores from the x-ray and ultrasound CAD algorithms is within the scope of the preferred embodiments.

FIG. 33 illustrates a conceptual diagram of a digitized x-ray mammogram 3300 and regions of interest (ROIs) A, B, C, and D therein. Each ROI is a fixed distance rA, rB, rC, and rD, respectively, from the nipple as can be determined using known methods. Also, each ROI is at an angle θA, θB, θC, and θD with respect to an axis perpendicular to the chest wall and passing through the nipple, as can be determined using known methods.

FIG. 34 illustrates a conceptual diagram of an adjunctive ultrasound view 3400 and regions of interest (ROIs) W, X, Y, and Z contained therein. Each ROI is a fixed distance dW, dX, dY, and dZ, respectively, from the nipple as can be determined using known methods. Also, each ROI is at an angle γW, γX, γY, and γZ with respect to an axis perpendicular to the chest wall and passing through the nipple, as can be determined using known methods. More generally, the angles γW, γX, γY, and γZ can be measured from any fixed axis through the nipple, provided it is the same fixed axis that the angles θA, θB, θC, and θD from the x-ray mammogram of FIG. 33 are measured from.

The goal of x-ray CAD/ultrasound CAD ROI registration according to the preferred embodiments is to find out (i) which of the ROIs in the adjunctive ultrasound view 3400, if any, represent the same physical locus in the breast as the ROIs of the x-ray mammogram 3300, and (ii) which of the ROIs in the x-ray mammogram 3300, if any, represent the same physical locus in the breast as the ROIs of the adjunctive ultrasound view 3400. Traditionally, this would involve a complex image registration process where the entire images mathematically mapped onto each other, often requiring additional information that is extrinsic to the medical images themselves, such as position sensing readouts. However, it has been found that a much simpler method for registering ROIs between x-ray and adjunctive ultrasound views based on nipple distance, and optionally nipple angle, yields sufficiently reliable results.

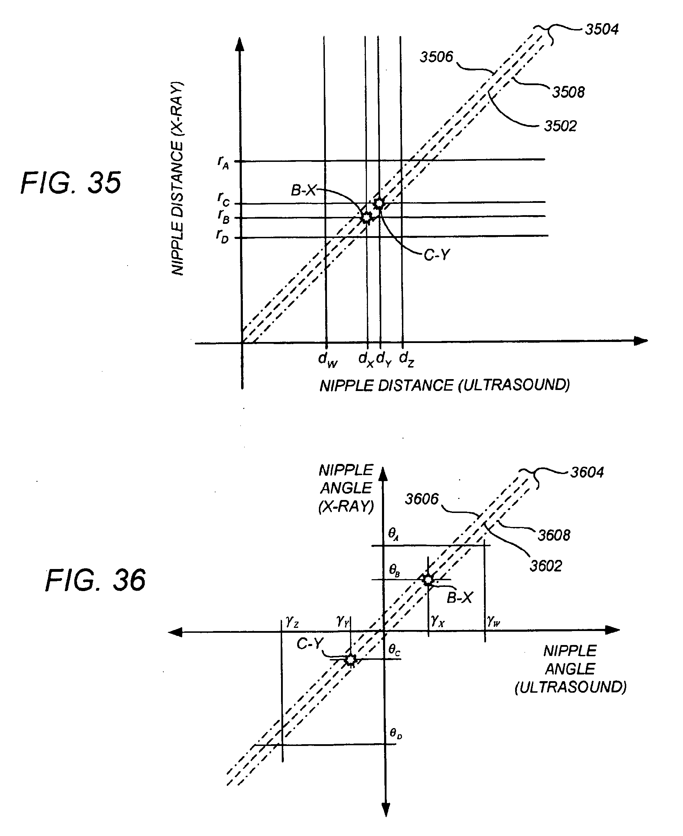

FIG. 35 illustrates a plot of nipple distance in the x-ray mammogram view versus nipple distance in the adjunctive ultrasound view for registering ROIs therebetween. For each ROI A, B, C, and D in the x-ray mammogram view of FIG. 33, the nipple distance metric rA, rB, rC, and rD, respectively, is computed using known methods. Horizontal lines are drawn at ordinates corresponding to rA, rB, rC, and rD. For each ROI W, X, Y, and Z in the adjunctive ultrasound view of FIG. 34, the nipple distance metric dW, dX, dY, and dZ, respectively, is computed using known methods. Vertical lines are drawn at the abscissas corresponding to dW, dX, dY, and dZ. According to a preferred embodiment, if any horizontal line intersects with any vertical line within a predetermined tolerance of a 45-degree line 3502, then the x-ray ROI for that horizontal line is identified as being "registered" with the ultrasound ROI for that vertical line.

In the example of FIGS. 33-35, it is seen that the ROI "B" from the x-ray mammogram registers to the ROI "X" from the adjunctive ultrasound view, and that the ROI "C" from the x-ray mammogram registers to the ROI "Y" from the adjunctive ultrasound view. The particular locations of the upper and lower boundaries 3506 and 3508 around the 45-degree line 3502 that define a correlation region 3504 can be determined from a training process that would be readily apparent to a person skilled in the art in view of the present disclosure. Importantly, for the preferred embodiment in which nipple distance is the sole correlating factor, it is not required that the x-ray and adjunctive ultrasound images be taken from the same view (e.g., CC or MLO). Rather, it is only required that the scales of the respective medical images are known, so that actual nipple distances can be measured. It is to be appreciated that, while described in terms of a graphical process in FIG. 35, a computer program implementing this algorithm will, of course, achieve its results in purely numerical fashion without requiring the actual plotting of data on a two-dimensional plane. More particularly, the computer program would register any two ROIs whose difference falls within a predetermined absolute or relative tolerance.

FIG. 36 illustrates a plot of nipple angle in an x-ray mammogram view versus nipple angle in an adjunctive ultrasound view for registering regions of interest therebetween with additional precision. For the purposes of FIG. 36, it is presumed that the x-ray and adjunctive ultrasound images are taken from the same standard view (e.g., CC or MLO) or otherwise from the same angle relative to the patient. For each pair of ROIs that are determined to be registered by nipple distance metric supra, the nipple angles in both the x-ray mammogram and ultrasound view are determined using known methods. In the present example in which ROI B correlates to ROI X and ROI C correlates to ROI Y according to the nipple distance metric, the nipple angles θB and θC are determined and horizontal lines drawn at corresponding ordinates in FIG. 36, and the nipple angles γX and γY are determined and vertical lines drawn at corresponding abscissas in FIG. 36. According to a preferred embodiment, the ROI pair B-X only continues to be identified as "registered" if the horizontal line for θB intersects with the vertical line for γX within a predetermined tolerance of a 45-degree line 3602, and likewise for ROI pair C-Y. The particular locations of the upper and lower boundaries 3606 and 3608 around the 45-degree line 3602 that define a correlation region 3604 can be determined from a training process that would be readily apparent to a person skilled in the art in view of the present disclosure. As with the nipple distance criterion, it is to be appreciated that a computer program would carry out the above steps in a purely numerical fashion without regard to visual graphical plots.

In another preferred embodiment, the above lesion-centric registration process also uses lesion size as a factor in matching corresponding regions of interest in the x-ray mammogram view and the adjunctive ultrasound views. In still another preferred embodiment, the above lesion-centric registration process also uses lesion distance from the chest wall as a factor in matching corresponding regions of interest in the x-ray mammogram view and the adjunctive ultrasound views. For a given lesion, the chest wall distance corresponds, for example, to a horizontal distance between that lesion and the chest wall 3302/3402 of FIGS. 33/34, respectively. Thus, for example, an ROI pair that would be identified as "registered" between the x-ray mammogram view and the adjunctive ultrasound views based on nipple distance and nipple angle would continue to be identified as "registered" only if they are of substantially similar size and have a substantially identical distance from the chest wall.

Thus, according to the preferred embodiments of FIGS. 32-36, the advantages of using a combination of both x-ray CAD data and ultrasound CAD data are provided, without requiring extrinsic information to correlate the two imaging modalities with each other. From a practical viewpoint, this provides a key advantage in integrating ultrasound mammography into the current mass breast cancer screening environment, because the current x-ray mammogram infrastructure can remain substantially undisturbed. If desired by the HMO or health care institution, the adjunctive ultrasound data can be obtained in an entirely separate clinical process, and yet the adjunctive ultrasound CAD results can still be advantageously and automatically combined with x-ray mammogram CAD results for increased specificity and sensitivity.

Also within the scope of the preferred embodiments is a computer program product for instructing one or more processors to carry out one or more of the methods of the preferred embodiments, such computer program product being amenable to ready implementation by a person skilled in the art in view of the present disclosure. In one preferred embodiment, the computer program product is executed primarily by the ultrasound server 106 of FIG. 1, with the other system devices of FIG. 1 performing simple input/output, display, and storage functions. In other preferred embodiments, the computer program product is distributed across the different systems of FIG. 1, with different algorithmic portions being carried out by different systems or subsystems. Ultrasound server 106 comprises a computer system that includes a cabinet, a display monitor, a keyboard, and a mouse, the mouse having one or more buttons for interacting with a graphical user interface. The cabinet typically houses a CD-ROM, zip, and/or floppy disc drive, system memory and a hard drive which can be utilized to store and retrieve software programs incorporating computer code that implements the preferred embodiments, data for use with the invention, and the like. An external hard drive is also shown in FIG. 1. Although CD-ROM, zip, and floppy discs represent common computer readable storage mediums, other computer readable storage media including tape, flash memory, system memory, and hard drives can be used. Additionally, a data signal embodied in a carrier wave, such as in a network including the Internet or an intranet, can form the computer readable storage medium.

Whereas many alterations and modifications of the present invention will no doubt become apparent to a person of ordinary skill in the art after having read the foregoing description, it is to be understood that the particular embodiments shown and described by way of illustration are in no way intended to be considered limiting. By way of example, although described supra in terms of adjunctive ultrasound screening, in view of the present disclosure one skilled in the art would readily be able to apply the thick-slice display apparatus of the preferred embodiments in the context of computerized tomography (CT) and/or magnetic resonance imaging (MRI) environments. In each case, individual image slices generated from CT scans or MRI scans of the breast are compounded so as to form thick-slice images of slab-like portions of the breast along planes parallel to a standardized x-ray mammogram view plane, and the thick-slice images are displayed to the radiologist in close proximity to an x-ray mammogram of the breast to assist in interpreting that x-ray mammogram. Preferably, a single composite view of the whole breast is shown together with the thick-slice image views, these views having their gray-scale polarities toggled and/or remapped such that they are reminiscent of x-ray mammogram views taken from the standardized direction. By way of further example, real-time implementations of the preferred embodiments may be readily extended to operate with an ultrasound-guided, computer-controlled biopsy apparatus. A surgeon can instantiate an automatic biopsy extraction procedure by graphically selecting the precise three-dimensional location (x, y, z) of a target lesion on the real-time adjunct ultrasound display as the breast is held steady by the compression device. A biopsy needle is automatically manipulated by a motorized mechanism that translates the biopsy needle in two dimensions to the desired (x, y) location, and then automatically inserts the needle in the "z" direction to enter the tumor, all the while being controlled by a feedback control system to keep the needle on-target. In this example, the "z" direction can be parallel to the compression plates, or alternatively can be perpendicular the compression plates where adequate holes or gaps in one of the compression plates are provided to allow the needle to pass therethrough. In other preferred embodiments, apparatuses similar to those described in one or more of the following references can be used, which are incorporated by reference herein: U.S. Pat. 5,078,142; U.S. Pat. 5,660,185; U.S. Pat. 5,833,627; and U.S. Pat. 6,102,866. Therefore, reference to the details of the preferred embodiments are not intended to limit their scope, which is limited only by the scope of the claims set forth below.

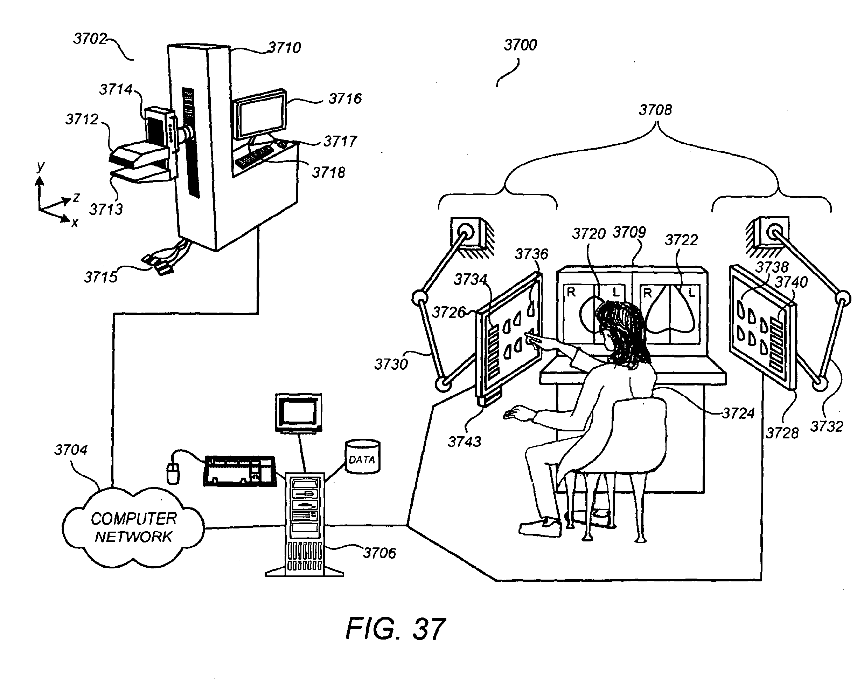

FIG. 37 illustrates a conceptual diagram of a system 3700 and associated methods for breast cancer screening using adjunctive ultrasound mammography according to a preferred embodiment. Adjunctive ultrasound mammography refers to the acquisition and display of breast ultrasound information during the breast cancer screening process in a manner that supplements x-ray mammogram information. System 3700 comprises an ultrasound scanning station 3702, a computer network 3704, an adjunctive ultrasound server 3706, and an adjunctive ultrasound screening station 3708.

Ultrasound scanning station 3702 comprises an apparatus designed to flatten and immobilize a breast while volumetric ultrasound scans are acquired. The breast is flattened along a plane substantially parallel to a standard x-ray mammogram view plane such as the CC and MLO view planes, although the ultrasound scanning station 3702 is capable of flattening the breast along a variety of other planes as well. Ultrasound scanning station 3702 comprises a housing 3710 movably supporting a gantry 3714, the gantry 3714 in turn supporting an upper compression/scanning assembly 3712 and a lower compression plate 3713 in a vertically movable manner.

For clarity of description herein, the y direction represents the head-to-toe direction with respect to the patient, the x-axis represents the left-to-right direction, and the z direction extends outward from the chest wall. The x-y, y-z, and x-z planes thus correspond to the coronal, sagittal, and axial planes, respectively. The patient may stand or sit in front of the ultrasound scanning station 3702, facing the +z direction in FIG. 37, with one breast placed between the upper compression/scanning assembly 3712 and lower compression plate 3713. Responsive to control by an operator using foot pedals 3715, a keyboard 3718, a mouse 3717, buttons on the gantry 3714, and/or other input methods, the breast is compressed and an ultrasound probe head (not shown) contained inside upper compression/scanning assembly 3712 is linearly translated over the top of the breast while two-dimensional ultrasound slices are acquired. Ultrasound scanning station 3702 further comprises an ultrasound processor (not shown) coupled to the ultrasound probe head that receives the acoustic echo signals and forms the two-dimensional ultrasound slices therefrom. The ultrasound slices may be viewed in real-time on a monitor 3716 as they are generated, the monitor 3716 also serving as an interface display for controlling of the overall operation of the ultrasound scanning station 3702.

Preferably, the upper compression/scanning assembly 3712 is similar to that described in U.S. Ser. No. 60/415,385. The breast skin surface contacts one side of a taut sheet of acoustically transparent material such as Mylar^{®} while the other side of the taut sheet is in actual or imminent contact with the probe head. Acoustic coupling between the taut sheet and the probe head is facilitated by a stream, drip, or bath of water or other lowviscosity, acoustically conductive fluid. The gantry 3714 is rotatable in a plane parallel to the coronal plane of the patient, *i.e.*, around the z-axis in FIG. 37, such that scans of the breast flattened along the MLO plane or other view planes can be achieved. The gantry 3714 can also be tilted forward or backward relative to the patient, *i*.*e*., around the x-axis in FIG. 37, between a range of roughly plus 30 degrees to minus 30 degrees.

According to one preferred embodiment, a breast scan for a given view is acquired by a single sweep of the probe contained within upper compression/scanning assembly 3712. In this case, it is required that the scan penetration depth extend as far as possible toward the lower compression plate. For larger breasts, this can be 6 cm or greater, in which case a lower probe frequency is required and a correspondingly lesser resolution is obtained than for high-frequency scans. According to another preferred embodiment, dual sweeps can be taken for a given view, with the gantry being rotated 180 degrees around the y-axis in FIG. 37 between sweeps. In this case, the scans only need to penetrate through half the breast thickness and so a higher scan frequency can be used. The resulting ultrasound "half-slices" from the first and second sweeps can then be stitched together to form complete slices. In still another preferred embodiment, the lower compression plate 3713 is replaced with a second compression/scanning assembly including a second probe head. In this case the separate upper and lower probe sweeps can be achieved without requiring the intermediate 180 degree rotation of the gantry, and issues relating to registration between the half-slices are avoided.

During or after the ultrasound scanning process, the raw ultrasound data is provided across the computer network 3704 to the adjunctive ultrasound server 3706, where the raw ultrasound data is processed into adjunctive ultrasound data that will be made available to the screening radiologist, the adjunctive ultrasound data including ultrasound slices, thick-slice images, CAD outputs, and other useful information. It is to be appreciated that the processing of the raw ultrasound data into the adjunctive ultrasound data may be performed by any of a variety of different computing devices coupled to the computer network 3704 and then transferred to the adjunctive ultrasound server 3706.

In current mass breast cancer screening environments based on x-ray mammography, a screening radiologist 3724 examines x-ray mammograms for many patients en masse in a single session using an x-ray viewing station 3709. The x-ray viewing station 3709 may range from a simple light box, as in FIG. 37, to more complex x-ray CAD workstations that automatically move the x-ray mammograms past the radiologist 3724 on a conveyor belt as a nearby CAD display highlights suspicious areas of the mammogram. Almost universally, left and right CC x-ray views 3720 are positioned on one side of the x-ray viewing station 3709, and left and right MLO x-ray views 3722 are positioned on the other side. The radiologist 3724 quickly examines the x-ray mammograms. For some x-ray mammograms the radiologist needs only a few seconds, while for other x-ray mammograms the radiologist needs up to five minutes, with an average being about two minutes per mammogram.

According to a preferred embodiment, this existing arrangement remains substantially undisturbed, but is augmented with equipment and data that facilitates fast and thorough x-ray mammogram screening by giving the radiologist a quick ultrasonic "second look" at the internal breast structure. Adjunctive ultrasound screening station 3708 comprises first and second adjunct displays 3726 and 3728 conveniently positioned near the x-ray viewing station 3709 such that the radiologist 3724 can (i) easily look back and forth between the first adjunct display 3726 and the CC x-ray views 3720, and (ii) easily look back and forth between the second adjunct display 3728 and the MLO x-ray views 3722.

Preferably, adjunct displays 3726 and 3728 display thick-slice images 3736 and 3738, respectively, corresponding to thick-slice regions of the breast volume substantially parallel to the CC and MLO view planes, respectively, acquired while the breast was flattened along the CC view plane and the MLO view plane, respectively. This allows the spatial content of the thick-slice images to roughly correspond to the spatial content of the corresponding x-ray mammograms, facilitating ready comparisons therebetween. However, the scope of the preferred embodiments is not necessarily so limited. According to an alternative preferred embodiment, the benefits of meaningful "second look" information inside the breast structure is still provided even where (i) the breast is compressed along a non-standard plane during the volumetric scans, or (ii) the breast is not compressed at all during the volumetric scans, or (iii) the thick-slice images correspond to planes not parallel to a standard x-ray mammogram view plane. In view of the thick-slice segmentation and inversion process described herein and other features and advantages according to the preferred embodiments, such non-standard compressions or non-standard thick-slice planes can still result useful thick-slice adjunctive ultrasound images for viewing, especially where a spatial guide similar to the iconic representations *infra* are displayed to properly "orient" the reader to the position and orientation of the non-standard thick-slice image.

According to a preferred embodiment, adjunct displays 3726 and 3728 are designed to facilitate quick, intuitive, and interactive navigation among different views of the thick-slice images and other adjunctive ultrasound data. Adjunct displays 3726 and 3728 are preferably touch-screen displays but other input devices just as a PC keyboard and mouse (not shown) can be used. FIG. 37 also shows control buttons 3734 and 3740 on adjunct displays 3726 and 3728, respectively, that have layouts and functionalities described further *infra.* A bar code reader 3743 reads a bar code of the x-ray mammogram, wherein the associated adjunctive ultrasound data for that breast is automatically retrieved from the ultrasound server 3706. In the event that the x-ray mammograms are loaded onto a motorized viewer, the bar codes from the x-ray mammograms are read as the images are loaded into the apparatus by a technician. Alternatively, the user can scan the bar code directly from the x-ray mammogram when it appears in front of them using a hand-held bar code scanner, wherein the corresponding adjunctive ultrasound data is retrieved from the adjunctive ultrasound server 3706. For clarity of presentation, the user interface description herein is presented relative to the CC adjunct display 3726, it being understood that analogous descriptions apply to the MLO adjunct display 3728 or to non-standard-plane adjunct displays in general. Additionally, although most of the exemplary displays herein show the CC data for a single breast (left or right), it is to be appreciated that concurrent viewing of the CC data for the other breast on the same display, or combinations of CC/MLO/non-standard views for both left and right breasts, are clearly within the scope of the preferred embodiments.

The adjunct display 3726 of FIG. 37 contains an array of smaller, or thumbnail, thick-slices images 3736, with the terms small and thumbnail simply indicating that the images are small enough to fit on the same monitor while communicating structures of more than one thick-slice region of the breast, and are smaller in size than the enlarged versions. Due to practical display size limitations, it is expected that the small or thumbnail images will be less than full-scale images and the enlarged versions will be greater than full-scale, although the scope of the preferred embodiments is not so limited. In one alternative preferred embodiment both the thumbnail and enlarged versions are less than full-scale, while in another preferred embodiment the thumbnail images are at full-scale and the enlarged versions are greater than full-scale, while in still another alternative preferred embodiment the thumbnail and enlarged versions are both greater than full-scale.

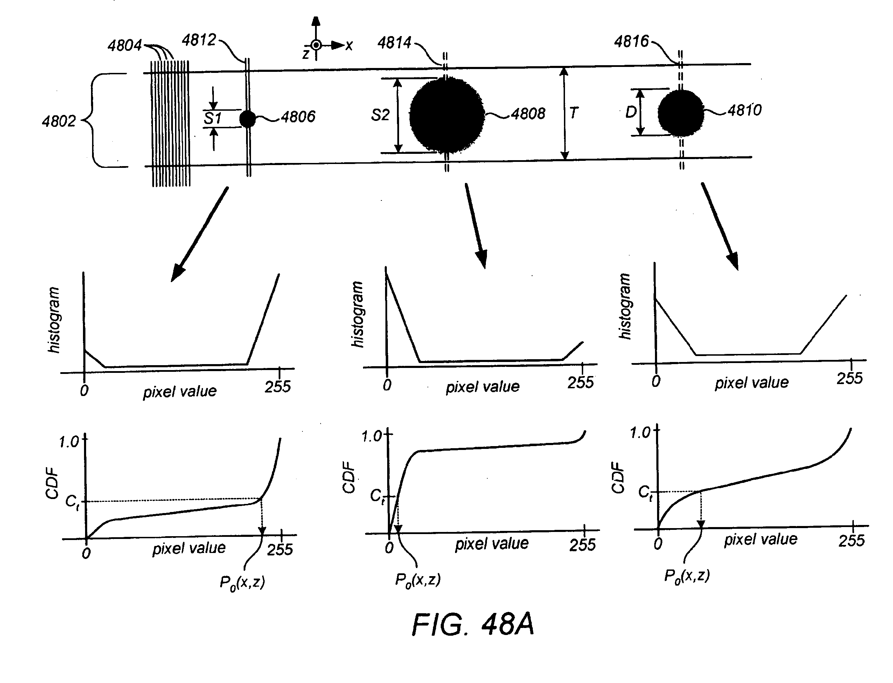

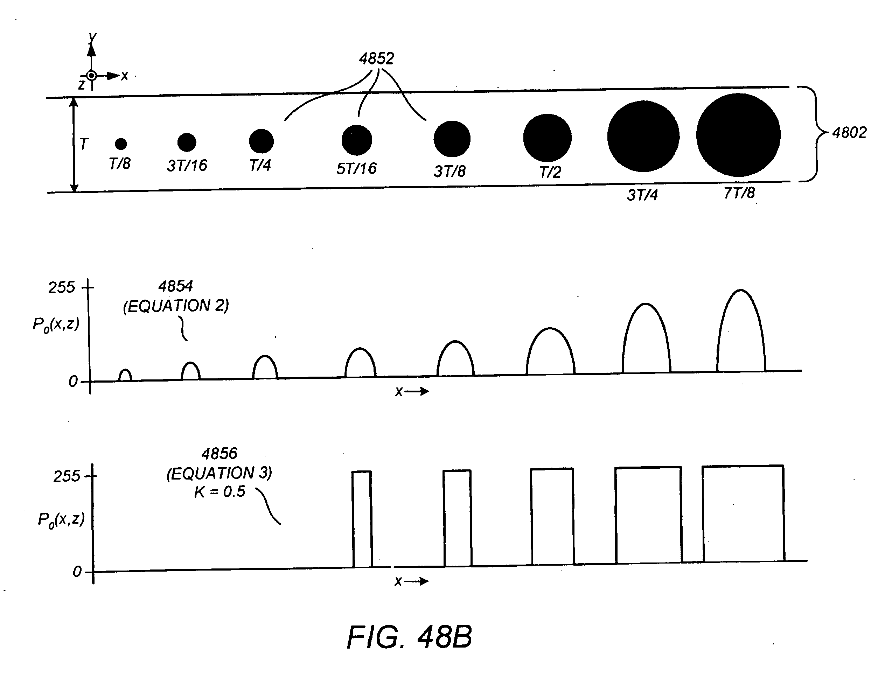

As described in U.S. Ser. No. 10/160,836, the thickness of the slab-like or thick-slice volume corresponding to each thick-slice image may lie, for example, in the range of 2 mm to 20 mm, although the scope of the preferred embodiments is not so limited, and thicknesses in the range of 7 mm to 12 mm are likely to be suitable for most breast cancer screening purposes. Techniques for integrating the component ultrasound slices into thick-slice images according to the preferred embodiments include arithmetic averaging, geometric averaging, reciprocal averaging, exponential averaging, and other averaging methods, in each case including both weighted and unweighted averaging techniques. Other suitable integration methods may be based on statistical properties of the population of component ultrasound slices at common locations, such as maximum value, minimum value, mean, variance, or other statistical algorithms. One particularly suitable algorithm for generating thick-slice images from a volumetric representation of a breast is described *infra* with respect to FIGS. 48-49.

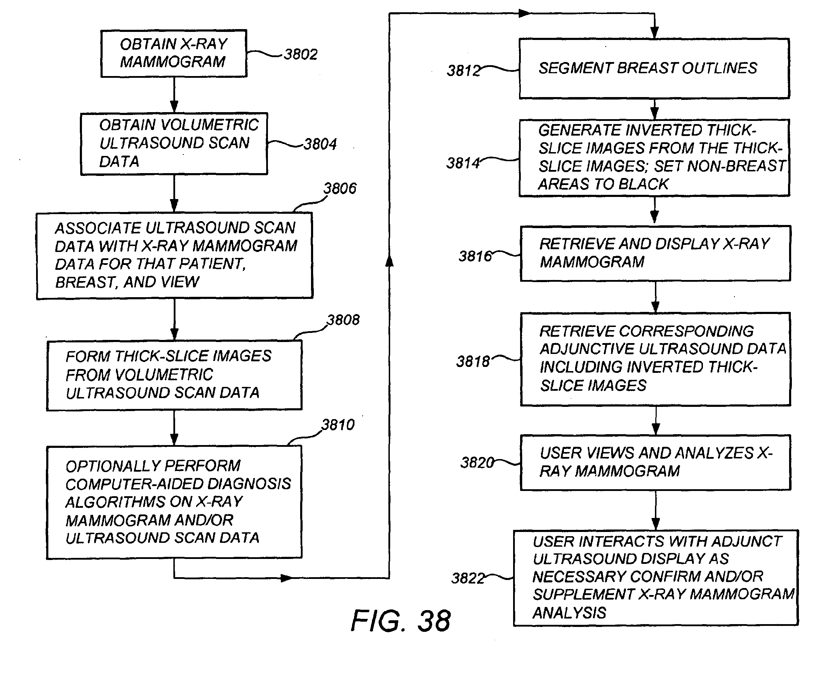

FIG. 38 illustrates steps for breast cancer screening using adjunctive ultrasound mammography according to a preferred embodiment. At step 3802 an x-ray mammogram is obtained and at step 3804 volumetric ultrasound scan data is obtained. At step 3806 the ultrasound scan data is associated with the x-ray mammogram data for the patient, breast, view, date, etc., as generally described in parent application Ser. No. 10/160,836, *supra.* At step 3808 thick-slice images are formed from the volumetric ultrasound scan data according to the method described *infra* with respect to FIGS. 48-49. For CC thick-slice images, *i.e.*, thick-slice images representing thick-slice volumes substantially parallel to the CC plane, each thick-slice image is a scalar function of coordinates (x,z). At step 3810 an optional step is performed wherein computer-aided diagnosis (CAD) algorithms are applied to the ultrasound scan data and/or x-ray mammogram data. In accordance with a preferred embodiment, the resulting CAD markers may be superimposed upon one or more of the thick-slice images described throughout this application, on any of planar ultrasound views, on any of the x-ray mammogram views, or any combination of them to achieve a CAD-enabled display.

At step 3812 the breast tissue is segmented from outlying areas using any of a variety of known segmentation algorithms. Preferably, a three-dimensional segmentation algorithm is performed directly on the three-dimensional volumetric scan data, although in other preferred embodiments a two-dimensional segmentation algorithm is separately applied to each thick-slice image. The segmentation step 3812 results in a mask for each thick-slice image identifying the breast tissue boundary.

At step 3814 inverted thick-slice images are computed from the ordinary or non-inverted thick-slice images. As used herein, a non-inverted or ordinary thick-slice image generally conforms to a standard medical ultrasound display convention in which readings of lesser acoustic reflections are displayed as "darker" (blacker, dimmer, darker gray, lower-intensity, etc.) and in which readings of higher acoustic reflections are displayed as "brighter" (whiter, lighter, higher-intensity, etc.). An inversion algorithm converts each non-inverted thick-slice image pixel P₀(x,z) into a complementary or inverted value P_{INV}(x,z) that is then "brighter" in regions of lesser acoustic reflection and "darker" in regions of higher acoustic reflection.

It has been found that displaying inverted thick-slice images can substantially enhance the viewing and screening process, and can facilitate the dismissal of benign lesions better than a display of non-inverted thick-slice images. This is at least because, for inverted thick-slice images, differential viewing of breast lesions versus surrounding tissue structures is provided on a similar basis as that for x-ray mammograms. For example, most radiologists have developed years of expertise in differentiating "bright" lesions from surrounding ligaments on x-ray mammograms, the surrounding ligaments also being "bright" but having different visual cues. The use of inverted thick-slice images allows their years of expertise to be extended over to the thick-slice ultrasound data, in contradistinction to the conventional ultrasound display method that would require the difficult task of differentiating "dark" lesions based on different visual cues than "dark" surrounding ligaments.

Although any of a variety of inversion algorithms could be used in accordance with the preferred embodiments at step 3814, it has been found beneficial to use an inversion algorithm that also performs some degree of contrast-enhancement when mapping the darker values of P₀(x,z) into the brighter values of P_{INV}(x,z). For an exemplary situation in which the display monitor pixels are brightest at value 255 and lowest at value 0, one particularly suitable algorithm is given in Eq. (1) below, with γ ("gamma") being set to 0.5: ${P}_{INV} \left(x⁢z\right) = 255 ⁢ {\left(\frac{255 - {P}_{0} \left(x⁢z\right)}{255}\right)}^{{/}_{γ} 1}$

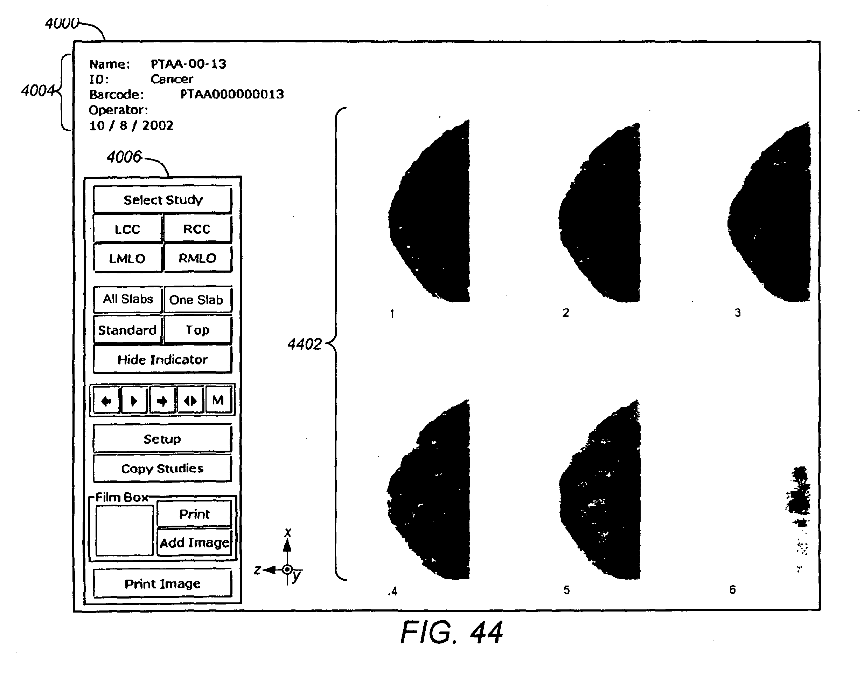

During image inversion, non-breast areas of the thick-slice images, which are initially dark, are converted to bright as displayed in the example of FIG. 44, *infra.* According to a preferred embodiment, the non-breast areas of the thick-slice images, as identified by the masks previously computed at step 3812, are reset to dark. This step is performed so that the displayed thick-slice images are more reminiscent of an x-ray mammogram, which is dark in the unexposed regions lying outside the breast. It is to be appreciated, however, that this step can be skipped without departing from the scope of the preferred embodiments, as some users may end up preferring the white background.

Generally speaking, the ultrasound processing steps 3808-3814 are usually not performed in real-time, but rather are performed during an interval between the scanning process and viewing process, which can be a period of several hours or more. However, the scope of the preferred embodiments is not so limited, and the steps 3802-3814 may also be performed in real-time if practicable in a given clinical setting.

At step 3816 the x-ray mammogram information is retrieved and displayed to the user. At step 3818 the corresponding adjunctive ultrasound data is retrieved, including the inverted thick-slice images. At step 3820 the user views and analyzes the x-ray mammogram image. In a manner analogous to FDA-approved practices for "second-look" x-ray mammogram CAD results, the user should first examine the x-ray mammogram without reference to the thick-slice images, first arriving at an independent conclusion based on the x-ray mammograms alone. Only after the independent x-ray mammogram analysis should the user view the thick-slice images (step 3822), wherein the user interacts with the adjunct ultrasound display as necessary to confirm and/or supplement the x-ray mammogram analysis. Optionally, to ensure the proper order of viewing, the thick-slice images are withheld from view until the user verifies that an independent x-ray mammogram analysis is completed by pressing, for example, a confirmation toggle button or entering an appropriate user command.

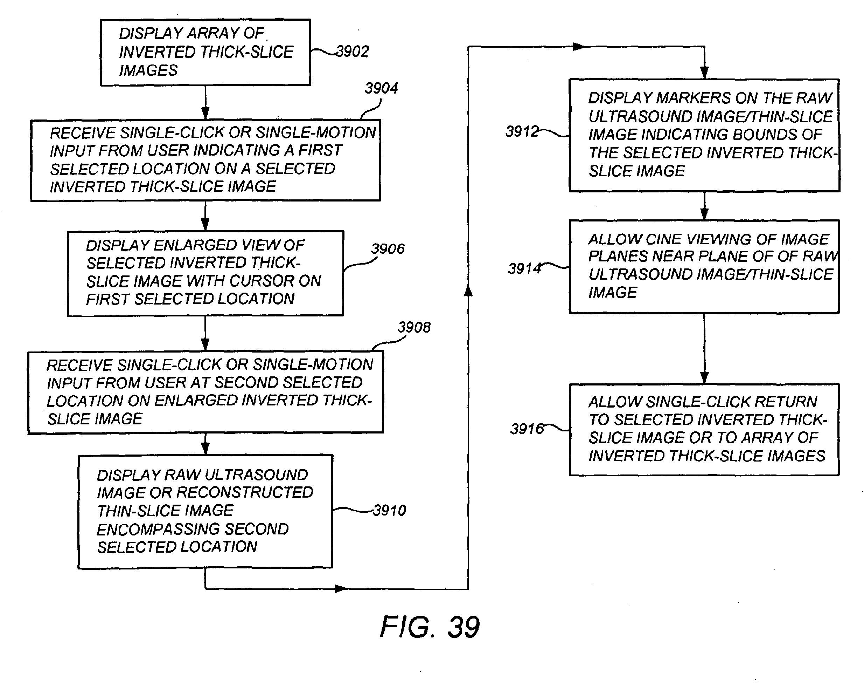

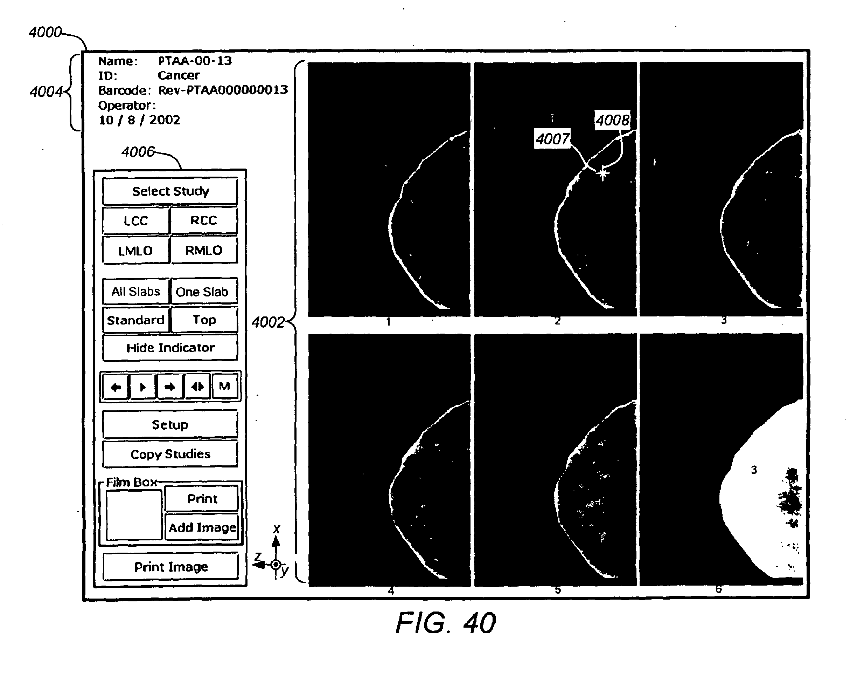

FIG. 39 illustrates steps for interactively displaying adjunctive ultrasound mammography information to a user according to a preferred embodiment. FIGS. 40-42 illustrate an adjunct display screen 4000 at particular stages during the steps of FIG. 39. At step 3902 an array of inverted thick-slice images is displayed. FIG. 40 illustrates an example of a thick-slice image array 4002 on the adjust display screen 4000. Adjunct display screen 4000 further comprises a patient ID section 4004 comprising patient identification and other relevant information. Adjunct display screen 4000 further comprises a control button array 4006 comprising buttons that can be actuated by a touchscreen press or a mouse/trackball inputs. Also shown in FIG. 40 is a selection marker 4007 appearing at a location 4008 superimposed on a thick-slice image 4010. If a mouse/trackball input is used, the default mouse pointer changes to the selection marker 4007 when guided over any one of the thick-slice images 4002.

At step 3904 a single-click or single-motion input from the user is received indicating a first selected location on a selected inverted thick-slice image. With reference to FIG. 40 this is achieved by making a mouse click with the selection marker 4007 at the current position (4008) or by pressing the touchscreen at position 4008. In the case of a touchscreen, it is recommended that a pointing device finer than a human finger be used, such as the stylus of a personal digital assistant (PDA), to touch the screen at the location 4008. Responsive to the single-click or single-motion input, at step 3906 an enlarged view 502 of the selected inverted thick-slice image 4010 is displayed. A selection marker 4103 is displayed at a location 4104 that corresponds to the same relative position on the breast as the location 4008 on the thick-slice image 4010.

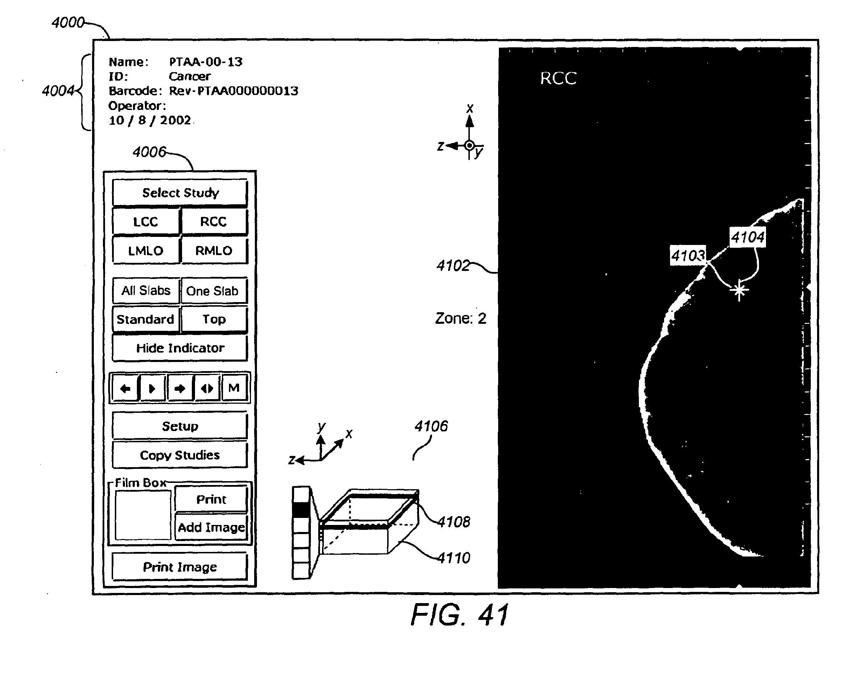

Also shown in FIG. 41 is a three-dimensional icon 4106 that roughly illustrates the thickness, location, and orientation of the thick-slice volume corresponding to the thick-slice image 4102. In particular, the iconic thick-slice volume 4108 is shown relative to an iconic rectangular solid 4110 that roughly corresponds to the compressed breast. Although not illustrated as such in FIG. 41, it is preferable to display an iconic frame that more closely resembles a perspective view of the breast rather than the iconic rectangular solid 4110. The iconic frame can comprise a three-dimensional rendering of a semi-transparent breast, and the iconic thick-slice volume 4108 can be suspended at the appropriate location and orientation therein. Because the three-dimensional icon 4106 does not provide any specific diagnostic information to be relied upon, it is not necessary for the iconic frame to be a perfect replica of the actual scanned breast. However, to increase the intuitiveness of the display, especially to new users, it is preferable that the semi-transparent rendering be of at least sufficient quality to be recognizable as a breast volume.

At step 3908 a second single-click or single-motion input is received from the user indicating a second selected location on the enlarged inverted thick-slice image. By default, the second selected location is at the same point relative to the enlarged inverted thick-slice breast as the first selected location was to the thumbnail or smaller thick-slice image, unless the user affirmatively moves the selection marker 4103 prior to clicking. In the example of FIGS. 40-42, the second selected location is at location 4104 of FIG. 41.

At step 3910 a raw ultrasound image or reconstructed thin-slice image encompassing the second selected location is displayed.

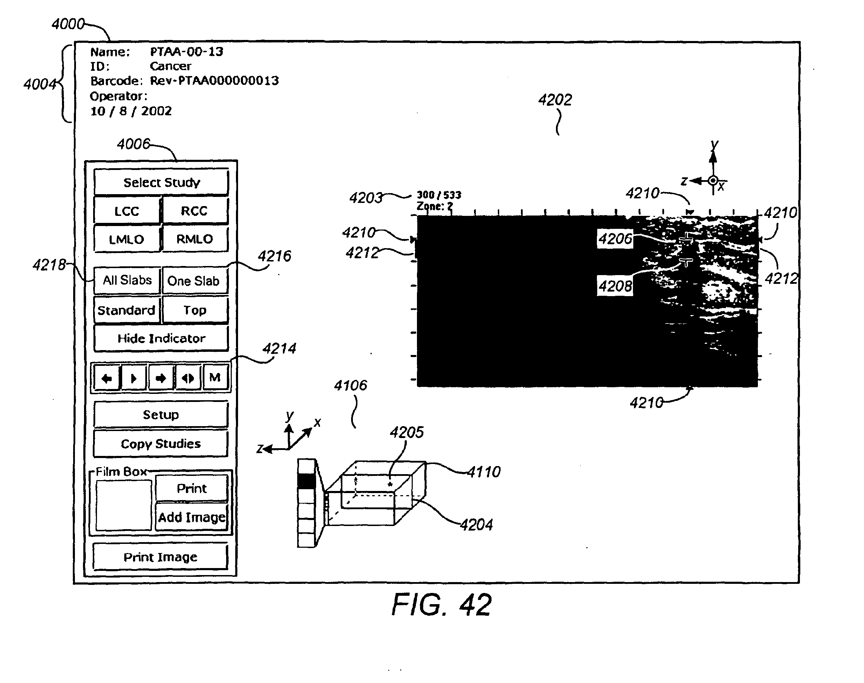

FIG. 42 illustrates a raw ultrasound slice 4202 displayed responsive to the second single-click or single-motion input. The raw ultrasound slice 4202 is preferably shown in a standard, non-inverted format because this direct form of ultrasound viewing is already familiar to most users. However, it is within the scope of the preferred embodiments for the raw ultrasound slice 4202 to be inverted prior to display. The raw ultrasound slice 4202 represents one example of a planar ultrasound image representing sonographic properties of the breast along a plane cutting through the breast. In particular, the raw ultrasound slice 4202 represents a first planar ultrasound image representing values along a first plane perpendicular to the orientation of the thick-slice region corresponding to the thick-slice image being displayed, wherein the first plane is also parallel to an ultrasound scan plane of the ultrasound probe that is swept across the breast during the volumetric scanning process. Most examples of planar ultrasound images described herein are raw ultrasound slices or orthogonal thin-slice images, the orthogonal thin-slice images being planar ultrasound images representing values along a second plane perpendicular to both (i) the raw ultrasound image, and (ii) the orientation of the thick-slice region corresponding to the thick-slice image being displayed. The orthogonal thin-slice images need to be reconstructed from the three-dimensional volumetric representation, as opposed to the raw ultrasound slices which can be taken directly from the raw data used to construct the three-dimensional volumetric representation. It is to be appreciated that while most examples of planar ultrasound images described herein are raw ultrasound slices or orthogonal thin-slice images, the scope of the preferred embodiments is not limited as such and extends to general cases of first and second planar ultrasound images having differing orientations.

Several features are provided on the adjunct display of FIG. 42 to assist the user in visualizing the location and orientation of the raw ultrasound slice 4202 relative to the breast volume. First, an alphanumeric representation 4203 of the actual raw slice number (in this example, slice number 300) out of the total number of raw slices (in this example, 533 slices) taken during the scanning process is shown. The three-dimensional icon 4106 including the iconic rectangular solid 4110 that roughly represents the breast volume outline is maintained in this view, with the position and orientation of the raw ultrasound slice 4202 being shown therein by an iconic plane 4204. In accordance with step 3912 of FIG. 39, upper and lower range markers 4206 and 4208, respectively, indicate the upper and lower "y" for the thick-slice image 4102 previously being viewed at a horizontal "z" position corresponding to the selected location 4104 from FIG. 41. Depth range markers 4212 indicate the vertical "y" extent for the thick-slice image 4102 as well. Hash markers 4210 also assist in locating the range markers 4206 and 4208. Finally, the location of range markers 4206 and 4208 relative to the raw ultrasound plane is also indicated by an iconic marker 4205 on the three-dimensional icon 4106.

In the example of Fig. 42, it is a raw ultrasound slice that is displayed because the ultrasound scans were taken directly in the y-z plane by the ultrasound scanning device 102 of FIG. 1. In a more general case, the raw ultrasound scans may not have been taken in planes perpendicular to the thick-slice region of the thick-slice image of FIG. 41. In those circumstances, a planar or thin-slice ultrasound image is reconstructed from the volumetric ultrasound data, the planar ultrasound image representing values along a plane perpendicular to the orientation of the relevant thick-slice region and passing through the second selected location of FIG. 41 The planar or thin-slice ultrasound image is preferably computed to represent as thin a slice as possible while remaining accurate, or to represent a slice having a thickness corresponding to the elevation beamwidth of the linear probe used to scan the breast if such type of probe was used.

At step 3914 cine viewing of raw ultrasound or thin-slice image frames is accommodated, beginning at the frame of raw ultrasound slice 4202. In the case of a mouse input, the cine can be controlled by a mouse wheel or mouse-mounted trackball. In the case of a touchscreen display, cine control can be achieved by a separate trackball provided near the display. A foot pedal can also be used for cine control to free up the hands of the user. Onscreen cine control buttons 4214 can also be used.

At step 3916 single-click or single-motion return to the selected enlarged inverted thick-slice image or to the array of inverted thick-slice images is accommodated. For example, a left-click of a mouse (or single-tap of a stylus on a touchscreen display) at a third selected location on the raw ultrasound slice 4202 can bring up the enlarged thickslice image corresponding to "y" depth of the third selected location, with a marker thereon indicating the (x,z) location of the third selected point. Alternatively, an onscreen "one slab" onscreen button 4216 can be pressed to bring up the previous enlarged thick-slice image 4102. A right click of a mouse (or double tap of a stylus on the touchscreen display) at the third selected point can bring up the array 4002 of thick-slice images, with markers indicating the (x,z) position of the third selected location superimposed on the appropriate one of the thick-slice images for the "y" depth of the third selected location. Alternatively, an "all slab" onscreen button can be pressed to bring up the array 4002 of thick-slice images.

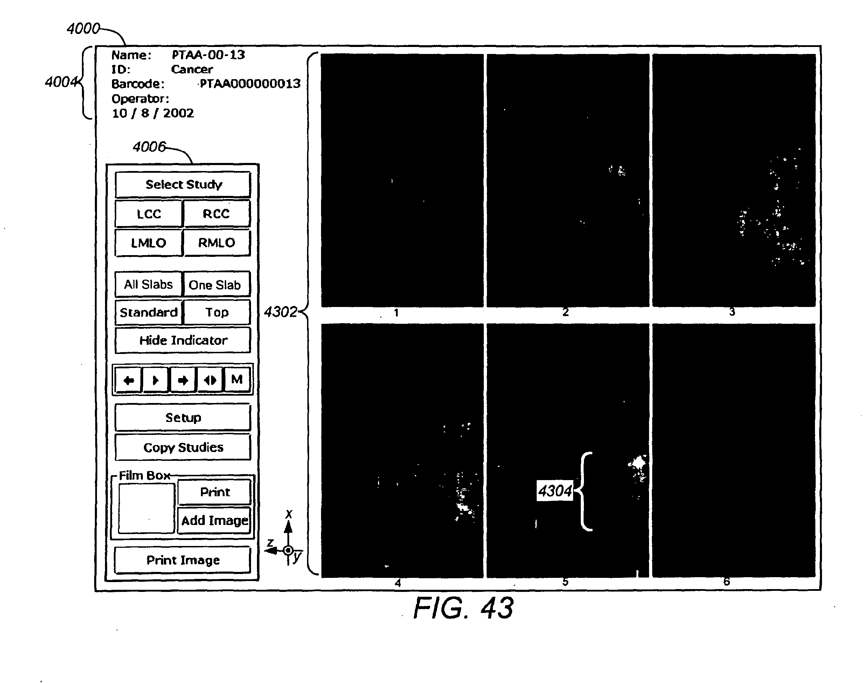

FIG. 43 illustrates the adjunct ultrasound display screen 4000 according to an alternative preferred embodiment in which an array 4302 of non-inverted thick-slice images are displayed. The non-inverted thick-slice image array 4302 corresponds to the same breast and ultrasound scanning session as for the inverted thick-slice image array of FIG. 40. With reference to a particular region 4304 circled in FIG. 43, it has been found that the non-inverted thick-slice images 4302 have a tendency to contain substantial areas of darkness in the breast tissue, wherein it is more difficult to detect subtle texture differences in breast tissue because it requires the differentiation of "dark" lesions from "less dark" or "differently dark" surrounding ligaments. This can lead to a larger number of false positives and also to user fatigue and frustration. In distinction, the inverted thickslice images of FIG. 40 are easier to analyze, at least because of (i) the many years of training that most radiologists have in differentiating "bright" lesions from "bright" surrounding ligaments in x-ray mammograms, and (ii) the contrast enhancement performed at brighter output levels during the inversion step 214 *supra.*

FIG. 44 illustrates the adjunct ultrasound display 4000 according to another preferred embodiment presenting an array 4402 of thick-slice images that were inverted but for which the background segmentation and darkening steps were omitted. The thickslice image array 4402 corresponds to the same breast and ultrasound scanning session as for the thick-slice image arrays of FIG. 40 and FIG. 43. As indicated in FIG. 44, the outlying non-breast areas are.bright instead of dark. In addition to being less familiar to most radiologists trained on x-ray mammograms, it is believed that this preferred embodiment might also lead to greater eyestrain. However, this preferred embodiment might nevertheless represent a desirable display format for some nonzero population of users.

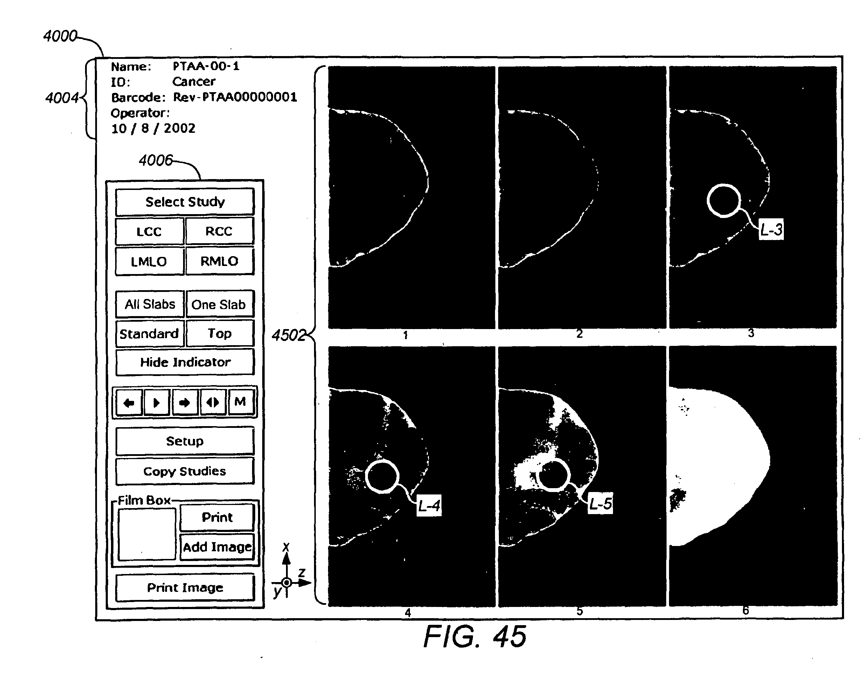

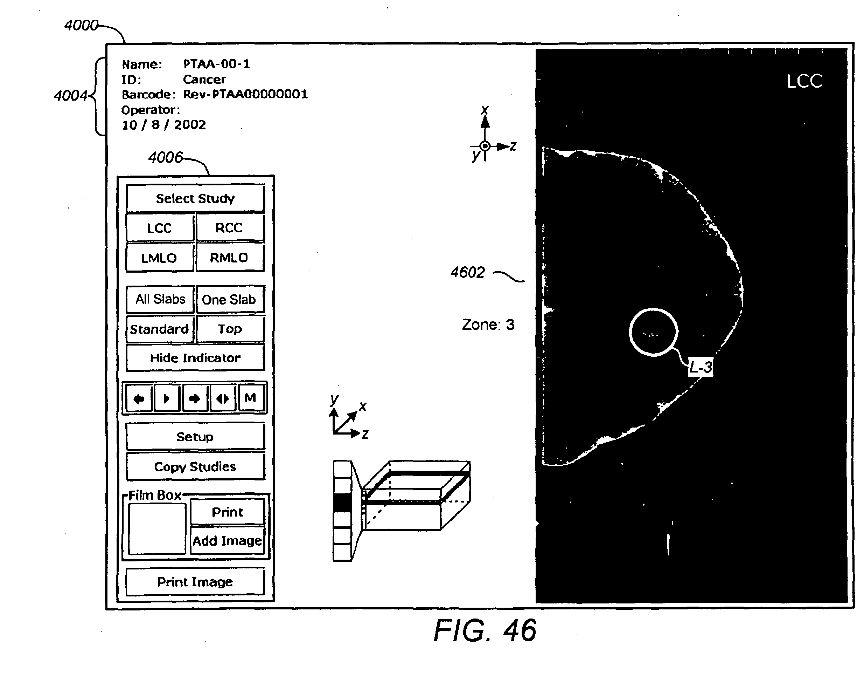

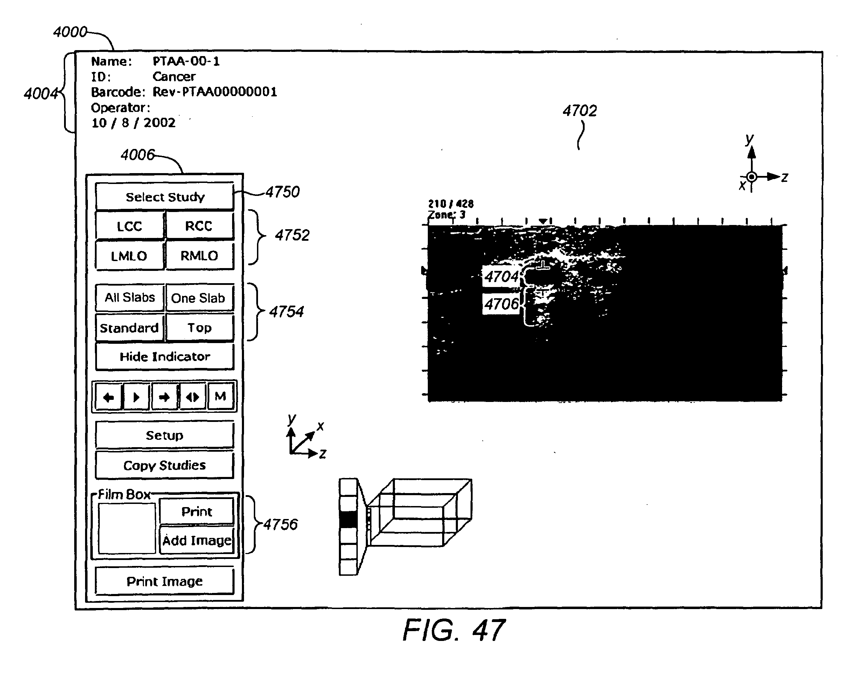

FIGS. 45-47 illustrate a thick-slice image array 4502, an enlarged thick-slice image 4602, and a raw ultrasound slice 4702, respectively, in a manner analogous to the images 4002, 4102, and 4202 of FIGS. 40-42, but for a different patient. FIGS. 45-47 illustrate one of the many features and advantages of the preferred embodiments wherein in which lesion shadows among successive thick-slice images, or the lack thereof, can rapidly assist in a diagnosis. Circled in FIG. 45 are locations L-3, L-4, and L-5 to indicate a particular (x,z) location L at thick-slice zones 3, 4, and 5, respectively. Notably, there is a white spot in the third zone at L-3, as illustrated on both FIG. 45 and FIG. 46, but there are dark spots in corresponding areas L-4 and L-5 of the fourth and fifth zones lying underneath the third zone. This indicates that there were low acoustic reflection readings for the third zone at L-3, while there were high acoustic reflection readings for two zones directly therebelow at L-4 and L-5. Accordingly, it is highly likely that this is a benign liquid-filled cyst that can be readily ignored. This can be quickly verified by viewing the raw ultrasound slice 4702 of FIG. 47, which shows a dark area 4704 of low reflections over an area 4706 of accentuated reflections.

Thus, according to a preferred embodiment, a method for quickly diagnosing the presence of a benign, liquid-filled cyst is provided, comprising the step of viewing an array of inverted thick-slice ultrasound images including a first inverted thick-slice image and a plurality of neighboring inverted thick-slice images, the neighboring inverted thickslice images corresponding to thick-slice regions directly below the thick-slice region of the first inverted thick-slice image relative to an ultrasound detector. The method further comprises the steps of observing a bright spot in the first thick-slice image and searching for dark spots in the plurality of neighboring inverted thick-slice images at locations corresponding to the bright spot. If such dark spots are present, the bright spot of the first inverted thick-slice image is diagnosed to be a benign, liquid-filled cyst. The method further comprises the step of verifying such diagnosis by viewing a raw ultrasound slice or reconstructed thin-slice image having a plane that passes through the breast location indicated by the bright.

With reference to FIG. 47, selected onscreen control buttons 4006 are now described. An onscreen "select study" onscreen button 4750 activates a file selection dialog box in which the user can select adjunctive ultrasound data for a particular patient and scan session. Onscreen "view selection" buttons 4752 select the desired view of LCC, RCC, LMLO, and RMLO. Onscreen display selection buttons 4754 allow the user to select the "all slab" view of FIG. 40, the "one slab" view of FIG. 41, the "standard" view of FIG. 42, or a "top" view representing an integration of all thick-slice regions for that view. Onscreen "film box" buttons 4756 allow for easy collection and/or printing of interesting images for subsequent review.

FIG. 48A illustrates a conceptual view of a thick-slice or slab-like region 4802 substantially parallel to the CC plane, along with hypothetical mass lesions 4806, 4808, and 4810, presented for describing thick-slice image computation according to a preferred embodiment. As indicated by the provided reference axes, the thick-slice region 4802 is being viewed from the +z direction, *i.e.*, from the front of the woman. The thick-slice region 4802 has a thickness T which can be about 1 cm for this example. The described thick-slice image computation algorithms operate on standard, non-inverted ultrasound voxel values for which zero is "dark" and 255 is "bright," although it is to be appreciated that it could be readily adapted by a person skilled in the art in view of the instant disclosure to operate on inverted ultrasound voxel values or otherwise-remapped voxel values.

Shown in FIG. 48A are side views of pixel columns 4804, including pixel columns 4812, 4814, and 4816 passing through the hypothetical mass lesions 4806, 4808, and 4810, respectively. A given pixel column extending downward in the y direction passing through the thick-slice region at pixel location (x,z) could comprise, for example, 40-60 voxels, each having an 8-bit value V_{xz}(yₙ). For each pixel column passing through the thick-slice region 4802, a scalar output value Po(x,z) is computed that constitutes the thick-slice image at that pixel location. In a simplest preferred embodiment, the thick-slice computation algorithm simply computes an average pixel value, as described by Eq. (2) below, where N is the number of voxels in the pixel column at (x,z): ${P}_{0} \left(x⁢z\right) = \frac{{\sum }_{n = 1}^{N} {V}_{xz} \left({y}_{n}\right)}{N}$

However, it has been found that thick-slice images more useful in breast cancer screening can be achieved by using a thick-slice computation algorithm that detects particular statistical variations of V_{xz}(yₙ) in the neighborhood of masses in a manner that emphasizes masses larger than a predetermined target size and de-emphasizes smaller masses in the in the resulting thick-slice image.

Shown in FIG. 48A for each of the pixel columns 4806, 4808, and 4810 passing through the respective hypothetical mass lesions is a histogram of the number of pixel elements at each respective gray value. Under the non-inverted convention of FIG. 48, the mass lesions of interest for breast cancer screening purposes tend to show up as "dark" regions against "whiter" backgrounds. It is to be observed that pixel columns passing through smaller masses, such as pixel column 4812 passing through mass 4806 having size S1, have substantially more high pixel values than low pixel values, as revealed by the associated histogram curve. As such, the area under the histogram from zero to a pixel value, termed herein the CDF or cumulative distribution function of V_{xz}(yₙ), remains low for most pixel values and only increases as higher pixel values are reached. Conversely, pixel columns passing through larger masses, such as pixel column 4814 passing through mass 4808 of size S2, have substantially more low pixel values than high pixel values, as revealed by the associated histogram curve and CDF.

According to a preferred embodiment, a target size D is established representing a mass size expected to be interesting from a breast cancer screening point of view. For purposes of this example, and not by way of limitation, a suitable target size D can be about 0.5 cm, which yields a target size to slab thickness ratio (D/T) equal to 0.5. The output pixel value Po(x,z) is set equal to that pixel value for which the cumulative distribution function of V_{xz}(yₙ) is equal to a preselected value K times the target size to slab thickness ratio. This is graphically illustrated in FIG. 48 and expressed in Eq. (3) below: ${CDF}_{{V}_{xz}} \left[{P}_{0}\left(x⁢z\right)\right] = K \left(\frac{D}{T}\right)$

FIG. 48B illustrates a conceptual view of the thick-slice region 4802 and lesions 4852 contained therein for a single fixed value of Z=Z_{fixed}, along with plots 4854 and 4856 of thick-slice image values P0(x,z_{fixed}) along a single line, the thick-slice images being computed according to Eqs. (2) and (3), respectively. For ease of computation and presentation, the lesions and surrounding breast tissue are assigned the extreme brightness values of 0 and 255, respectively. Although this conceptualizes the example somewhat by using an "extremely" bimodal voxel value distribution, it is to be appreciated that regions near mass lesions often have histogram distributions with a roughly bimodal characteristics, and so the results of FIG. 48B are roughly representative of the actual results obtained. A preselected value of K = 0.5 was used in the application of Eq. (3). As indicated by a comparison of plot 4854 versus the plot 4856, the use of the CDF-based method of Eq. (3) yields a resulting thick-slice image that enhances the visibility of lesions as they approach and exceed the target size D, while de-emphasizing smaller lesions. Different values ofK may be used in Eq. (3), *e.g.*, in the range of 0.25 to 0.75, and the value may be optimized for a given configuration based on empirical results. Generally speaking, as K is increased there is decreased "sensitivity" with more of the smaller lesions being ignored. In an alternative preferred embodiment, the value of K may be dynamically assigned based on local volume characteristics, for example, as a function of the local statistical variance of the voxel values.

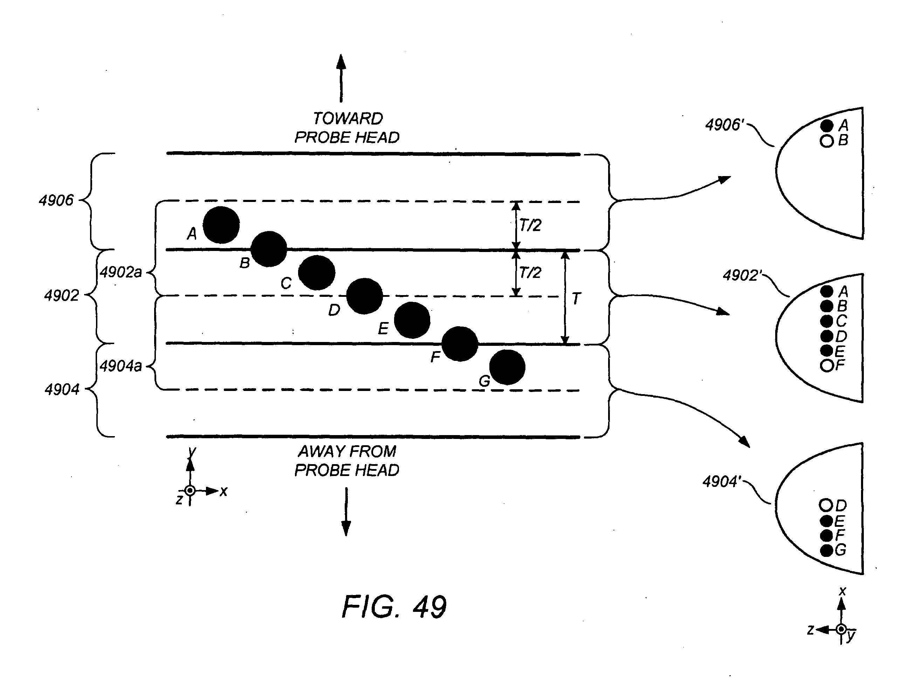

FIG. 49 illustrates a border compensation method for thick-slice generation according to the preferred embodiments that compensates for the possibility that lesions may straddle thick-slice borders. Shown in FIG. 49 is an exemplary thick-slice region 4902 vertically adjacent to an upper thick-slice region 4906 and a lower thick-slice region 4904. Also shown in FIG. 49 are exemplary mass lesions A-G, some of which are entirely contained in one of the thick-slice regions and some of which straddle the thick-slice region borders. As indicated in FIG. 49, the terms upper and lower are indicative of a distance from an ultrasound probe used to scan the breast volume. According to a preferred embodiment, when computing a thick-slice image, two alternative thick-slice values for each location (x,y) are computed, including a first value corresponding to the actual borders of the thick-slice region and a hypothetical value corresponding to the borders of a hypothetical thick-slice region having a similar thickness but extending into the next upper thick-slice region. In the example of FIG. 49, a hypothetical thick-slice regions 4902a and 4904a correspond to thick-slice regions 4902 and 4904, respectively. For each location (x,z) in the thick-slice image, the lesser (*i.e.*, "darker") of the first value and the hypothetical value is used.

Also shown in FIG. 49 are conceptual thick-slice images 4902', 4904', and 4906' corresponding to thick-slice regions 4902, 4904, and 4906, respectively. In this example, it is presumed that there are no lesions in the breast other than the mass lesions A-G. For purposes of presentation, a filled-in circle is shown when the "entire effect" of a mass lesion is present in the thick-slice image, whereas an empty circle is shown when only a "half effect" is present. Notably, whereas the lesion B would only be "half-present" in both of the thick-slice images 4906' and 4902' in an uncompensated scenario (not shown), it is "fully-present" in the thick-slice image 4902' as well as "half present" in thick-slice image 4906' in the compensated scenario shown in FIG. 49. Lesion A, which would be fully-present in thick-slice image 4906' and not present in thick-slice image 4902' in an uncompensated scenario, is fully-present in both of the thick-slice images 4906' and 4902' in the compensated scenario. It has been found that this border-straddling compensation strategy, for which other examples are presented in FIG. 49, represents a desirable result even though it tends to "redundantly" show the same lesion twice. It has been found that it is more desirable to provide "redundant" showings of the same mass lesion in two adjacent thick-slice images than to fail to display the "full effect" of a mass lesion straddling the borders of those slices. Also, because the display of the ultrasound thick-slices is only used in an adjunctive diagnostic sense, and not in an absolute diagnostic sense, there are few if any practical implications for overall system specificity.

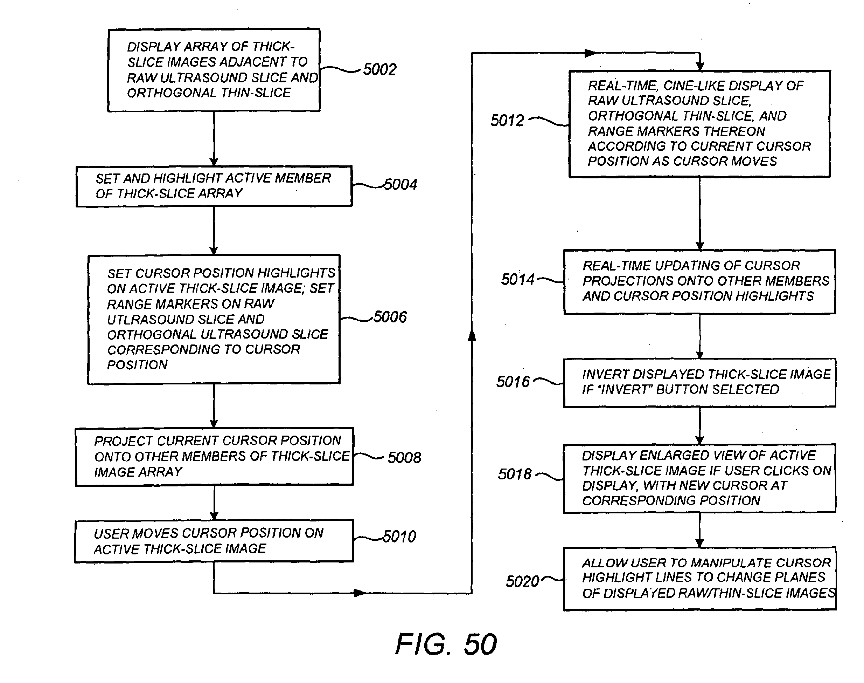

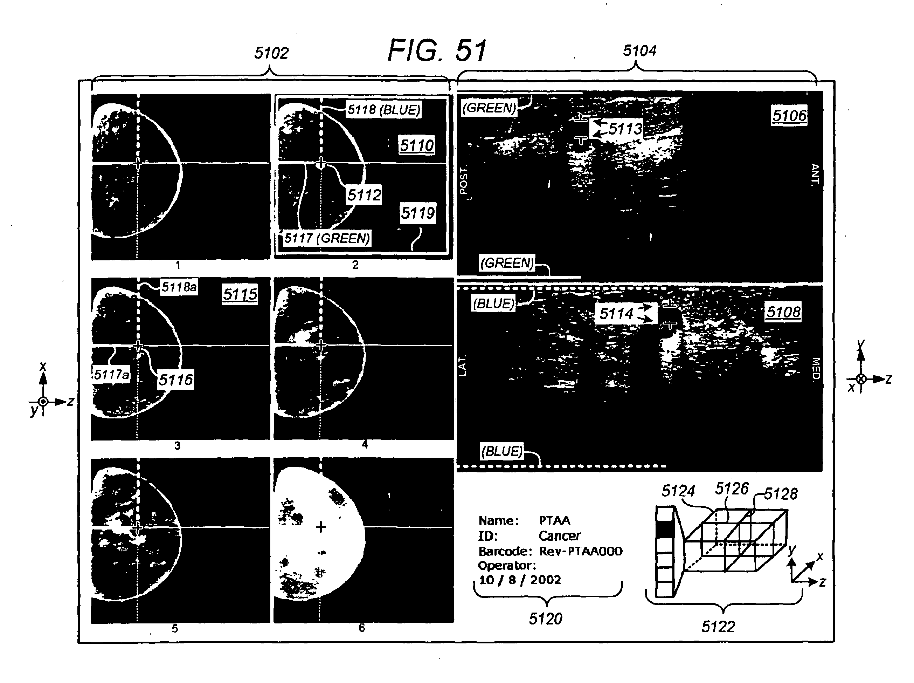

FIG. 50 illustrates steps for interactive display of adjunctive ultrasound mammography information according to a preferred embodiment. The steps of FIG. 50 are further illustrated in the adjunct displays of FIGS. 51-53. At step 5002, an array of thickslice images is displayed adjacent to a raw ultrasound slice and an orthogonal thin-slice image. With respect to FIG. 51, the array of thick-slice images 5102 is displayed next to planar ultrasound images 5104 including a raw ultrasound image 5106 and an orthogonal thin-slice image 5108. At step 5004, an active member of the thick-slice array is selected, such as by receiving a simple mouse point-and-click from the user, and is then highlighted. With respect to FIG. 51, thick-slice array 5102 comprises an active member 5110 having a highlighted marking 5119 shown thereon. First and second range markers 5113 and 5114, respectively, are provided on the raw ultrasound image 5106 and the orthogonal thin-slice image 5108, respectively, marking the upper and lower border of the thick-slice region of the active slice 5110 at a horizontal position corresponding to a cursor marker 5112 thereon.

Superimposed on active member 5110 are first and second plane indicators 5117 and 5118, respectively. The first plane indicator 5117 corresponds to a first plane through the breast volume as displayed by the raw ultrasound image 5106, and is visually related thereto by means of a color such as green that matches colored lines at the edges of the raw ultrasound image 5106. Preferably, the colored lines at the edges of raw ultrasound image 5106 extend from the posterior edge thereof across to the current position of the range marker 5113, which makes it easier to keep track of the first range marker 5113 as the cursor 5112 is moved by the user. The plane indicator 5117 is likewise heavily drawn from the posterior edge to the cursor position and then lightly drawn over to the anterior edge to assist tracking. According to an alternative preferred embodiment, the first range marker 5113 can remain fixed at the center of the frame, while the rest of the raw ultrasound slice dynamically shifts around it according to user cursor movements. Similar descriptions apply to second plane indicator 5118, orthogonal thin-slice image 5108, and second range marker 5114.

At step 5008 the position of cursor 5112 on active member 5110 is projected onto other members of the thick-slice image array. Shown in FIG. 51 with respect to a representative second member image 5115 is a projected cursor 5116. Also shown in the second member image 5115 are projected first and second plane indicators 5117a and 5118a.

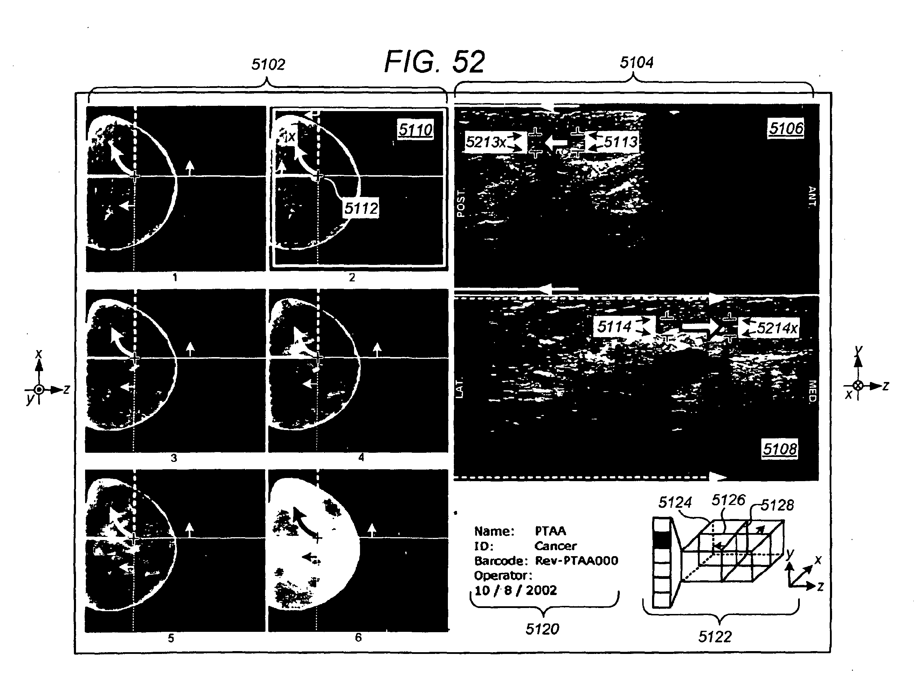

At step 5010 the user moves the cursor position on the active thick-slice image. At step 5012 in a real-time "cine-like" display is provided wherein the planar images 5104 are refreshed in real-time to keep up with the current position of plane indicators 5117 and 5118, which follow the cursor position. At step 5014, real-time updating of the cursor projections and the plane indicator projections is provided. According to a preferred embodiment, shifts in the position of cursor 5112 and corresponding shifts in the plane indicators 5117 and 5118 are mirrored in the other members of the thick-slice array, as illustrated in FIG. 52. FIG. 52 also shows how range markers 5113 and 5114 are shifted to new positions 5213x and 5214x, respectively, during the cursor shift, as well as the manner in which the colored edges of the planar images shift with cursor position.

Also shown in FIGS. 51 and 52 is a patient ID 5120 and a three-dimensional icon 5122. The three-dimensional icon 5122 comprises a frame structure 5124 similar to the frame structure 510 of FIG. 5, and two iconic plane indicators 5126 and 5128 thereon corresponding to plane indicators 5117 and 5118, respectively. As indicated in FIG. 52, the iconic plane indicators 5126 and 5128 also move dynamically with the cursor position. In one preferred embodiment, the look and feel of the cursor movement is analogous to a computer-aided drawing display (*e.g.*, Microsoft Visio) in which the cursor can, at the user's option, "stick" to horizontal and vertical "grid lines". This allows for one of the planar ultrasound images 5106 or 5108 to remain perfectly static if the cursor is moved along the horizontal or the vertical directions. As described *supra*, in an alternative preferred embodiment the range markers 5113 and 5114 can remain fixed at the center of the planar ultrasound frame, while the images move around them, it being found that many users can focus better on the tissue near the cursor position using this method.

In the display of FIGS. 51-52, soft buttons analogous to those of FIGS. 40-47 are omitted in favor of a mechanical keypad described *infra*, although the scope of the preferred embodiments is by no means so limited. At step 5016, the displayed thick-slice images are inverted (see FIG. 8, *supra*) when the user presses an "invert" button on the keypad. Preferably, when an inverted convention is used, the background is segmented out and returned to dark. At step 5018 an enlarged view of the active thick-slice image is displayed if the user clicks or taps on a selected cursor position or enters a "one slab" keypad entry.

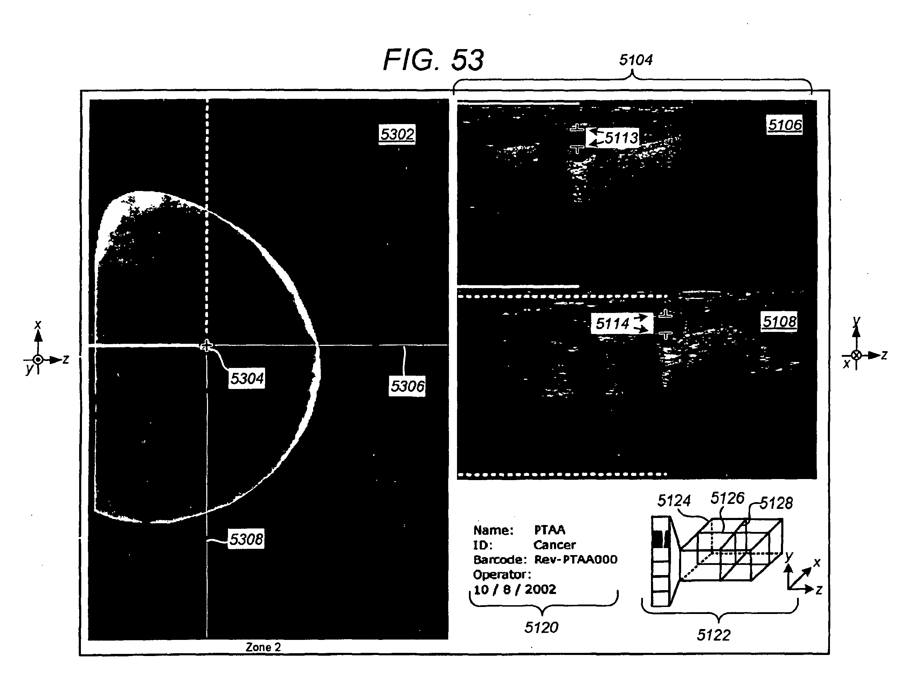

FIG. 53 illustrates an enlarged view 5302 of the active thick-slice image member 5110 of FIGS. 51-52 that appears at step 5018. The cursor position 5304 corresponds to the same breast location as the selected cursor position 5112 of FIG. 51. At step 5020, the user is permitted (on either the one-slab or all-slab displays) to manipulate the orientation and positions of plane indicators 5306/5308 or 5117/5118 in a manner similar to the way a computer draftsperson manipulates lines in a graphical illustration, while in real-time the planar ultrasound images 5104 remain updated to reflect corresponding planes through the breast volume.

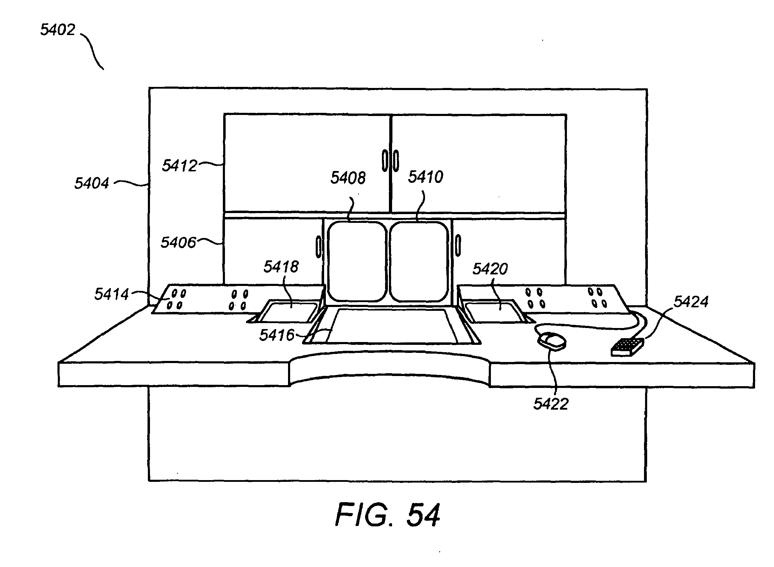

FIG. 54 illustrates an exterior view of an adjunctive ultrasound mammography display unit 5402 in accordance with a preferred embodiment. Adjunctive ultrasound mammography display unit 5402 comprises an x-ray film conveyor and display unit 5404 with modifications made to accommodate adjunctive ultrasound display. X-ray film conveyor and display unit 5404 comprises a lower film conveyor 5406 and an upper film conveyor 5412, the lower conveyor 5412 being turned off in this example and the lower conveyor 5406 displaying two x-ray mammogram images 5408 and 5410. Conventional control buttons 5414 provided with most commercially available x-ray film conveyor and display units are provided to control the x-ray mammogram film conveyors.

According to a preferred embodiment, adjunctive ultrasound mammography display unit 5402 comprises a full-sized LCD display 5416 integrated into a front table portion thereof as shown in FIG. 54. Preferably, LCD display 5416 is large enough to display two full-scale thick-slice ultrasound images simultaneously, including a left image corresponding to the x-ray mammogram image 5408 and a right image corresponding to x-ray mammogram image 5410. A 17-inch diagonal LCD display has been found suitable to achieve this objective. In contrast to the preferred embodiment of FIG. 1, *supra*, LCD display 5416 is conveniently located to allow back-and-forth viewing between the thickslice images and the x-ray images in an up-and-down manner, with minimal head motion. As discussed previously, even subtle ergonomic issues can be of crucial importance in the breast cancer screening process, because if the image viewing apparatus leads to tired or physically fatigue radiologists, such fatigue can unfortunately transform into a potentially fatal missed diagnosis or, alternatively, the inefficient process of an unnecessary patient callback.

In distinction contrast to cathode ray tube (CRT) monitors of equivalent viewable size, the LCD display 5416 can be advantageously placed in the middle of the table portion without interfering with the knees of the user. Adjunctive ultrasound mammography display unit 5402 further comprises CRT monitors 5418 and 5420 directly to the left and right of the LCD display 5416, respectively. Preferably, the CRTs have a 7-inch diagonal screen. It has been found more desirable to present the above planar ultrasound images (*e.g.*, raw ultrasound slices and orthogonal thin-slice views) on smaller CRT displays on the side of a larger LCD thick-slice display. The smaller CRT display allows for a substantially increased brightness range and also represents a more familiar optical characteristic to users familiar with traditional ultrasound displays. Among these characteristics is a slight low-pass filtering effect due to the finite width of the cathode-ray beam, in distinction to a pixellating effect that can be observed on LCD monitors. Because the CRT monitors 5418 and 5420 are set back and to the side of the LCD monitor 5416, they do not interfere with the knees of the user. Adjunctive ultrasound mammography display unit 5402 further comprises a mouse 5422 and a keypad 5424 for controlling the adjunctive ultrasound mammography displays.

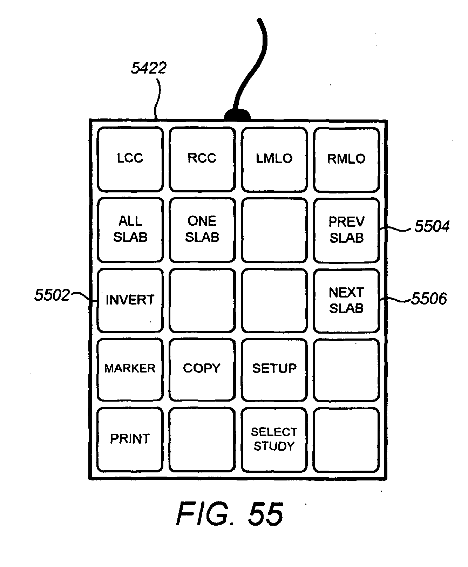

FIG. 55 illustrates the keypad 5422 of FIG. 54 in more detail. The keypad 5422 provides functionality similar to the soft buttons described *supra* with respect to FIG. 47, with some additional capabilities relevant to the preferred embodiments of FIGS. 51-53. An "invert" key 5502 causes the display of all thick-slice images to be inverted, as described *supra* with respect to FIG. 50. Also, a "prev slab" key 5504 and "next slab" key 5506 are provided as an additional method for allowing the user to change the active member of the thick-slice array being displayed.

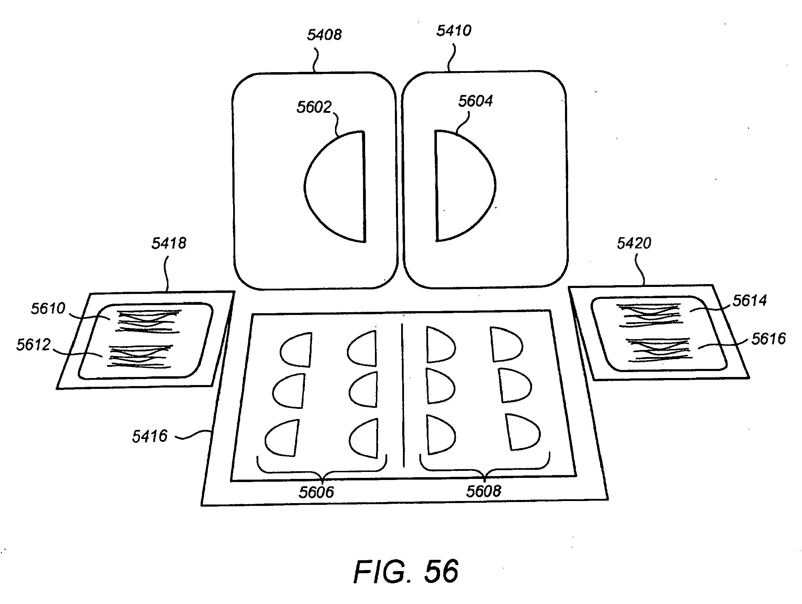

FIG. 56 illustrates an x-ray/adjunctive ultrasound mammography display according to a preferred embodiment as implemented on the apparatus of FIG. 54. An example is shown for the CC view, it being understood that an equivalent display is also provided for the MLO view. The MLO x-ray views may be shown, for example, on the upper conveyor 5412 of FIG. 54, while a toggle button allows the corresponding adjunctive ultrasound images for the MLO view to be displayed on monitors 5416, 5418, and 5420. Shown in FIG. 56 is an RCC x-ray mammogram film 5602 and an LCC x-ray mammogram film 5604. On the left hand side of LCD display 5416 is a thick-slice array 5606 for the CC view of the right breast, and on the right hand side of LCD display 5416 a thick-slice array 5608 for the CC view of the left breast. CRT monitor 5418 displays a raw ultrasound image 5610 and orthogonal thin-slice view 5612 corresponding to a current active cursor position on the RCC thick-slice display 5606. CRT monitor 5420 shows equivalent planar ultrasound images 5614 and 5616 for the LCC thick-slice display 5608.

According to an alternative preferred embodiment, only a single CRT display is provided to one side of the central LCD display, the central LCD display showing thickslice images for only a single breast, and the CRT display showing the corresponding planar ultrasound images. This alternative preferred embodiment can be advantageous for cost and space considerations, as well as in recognition of the fact that the user will generally only be closely analyzing a single breast of a time.

According to another alternative preferred embodiment, one side of FIG. 56 displays CC views (CC x-ray, CC thick-slice images, and CC planar ultrasound images) for a single breast, while the other side of FIG. 56 displays MLO views (MLO x-ray, MLO thick-slice images, and MLO planar ultrasound images ) for that breast. This preferred embodiment can be advantageous insofar as all standard information for the breast is now provided in a single set of images being concurrently displayed, rather than requiring the user to physically instantiate switches between CC and MLO views of the same breast during analysis.

A variety of other configurations, each having its particular advantages, is also within the scope of the preferred embodiments. By way of example, the thick-slice images may instead be provided on paper or on film, while the electronic display of thin-slice images are an optional addition to the unit. Where the thick-slice images are provided on film, a technician can simply load the thick-slice image films into the conveyor unit adjacent to or above the standard x-ray films.

According to another preferred embodiment relating generally to the method of FIG. 50, the user may instantiate a cine display for the raw ultrasound image 5106, the orthogonal thin-slice image 5108, or a combination of both along a predetermined cursor trajectory. By way of example, responsive the user pressing an additional button "lat-med cine" (not shown) on keypad 5422, a cine display of the raw ultrasound slice 5106 is displayed with the cursor 5112/5304 moving automatically in a vertical direction on thickslice image 5110/5302 at its current horizontal position. The orthogonal thin-slice image 5108 will remain static. The user can control the cine display using additional cine control buttons (not shown) on keypad 5422. Likewise, responsive the user pressing an additional button "post-ant cine" (not shown) on keypad 5422, a cine display of the orthogonal thin-slice image 5108 is displayed with the cursor 5112/5304 moving automatically in a horizontal direction on thick-slice image 5110/5302 at its current vertical position, the raw ultrasound image 5106 remaining static. It is preferable that this preferred embodiment be used in conjunction with the particular above-described preferred embodiment in which the range markers 5113 and 5114 are fixed at the center of the frame.

In a slightly more complicated preferred embodiment, controls are provided for which the user can select a custom cine trajectory, wherein both of the planar ultrasound images 5106 and 5108 will change as the cursor 5112/5304 automatically follows that trajectory. The user can select the custom cine trajectory, for example, by manipulating the plane indicators 5117/5118 in a Visio-like manner as described previously. The user can then activate the cine action by pressing the "lat-med" cine button to make the cursor 5112/5304 move along the plane indicator 5118, or by pressing the "lat-med" cine button to make the cursor 5112/5304 move along the plane indicator 5117.

According to another preferred embodiment, CAD markers identifying suspicious lesions are superimposed on the thick-slice images 5102 to direct the attention of the user to those particular locations. Preferably, the CAD markers are generated from the volumetric ultrasound scan data used to generate the displayed thick-slice images 5102, although the scope of the preferred embodiments is not so limited. According to a preferred embodiment, the CAD markers are displayed responsive to the user pressing a "show CAD marker" button (not shown) on keypad 5422, and are superimposed on the currently-showing thick-slice display, i.e. the thick-slice array 5102 or the enlarged thickslice image 5302. The CAD markers may be color-coded, size-coded, shape-coded, modecoded (blinking, flashing, etc.), etc., and/or accompanied by nearby alphanumeric tags or text messages to conveniently portray higher-suspiciousness lesions versus lower-suspiciousness lesions. Preferably, where the user presses the "show CAD marker" button as the thick-slice array 5102 is displayed, the active thick-slice image is automatically selected to be that member containing the most-suspicious lesion, the cursor 5112 is automatically relocated to the location of that lesion, and the planar ultrasound images 5106 and 5108 thereby automatically show the location of that lesion in the center of the planar image displays and mark that location with the range markers 5113-5114 and an optional additional indicator.

According to another preferred embodiment for use in conjunction with each of the above embodiments is a reverse-locating capability from a first location of interest on a planar ultrasound image to a corresponding location on the proper corresponding thickslice image. During review of the planar ultrasound images 5106 and 5108 as the user is moving the cursor 5112/5304 around the active thick-slice member image 5110, the user may see an interesting location appear somewhere on one of the planar ultrasound images 5106 or 5108. When this occurs, the user can right-click the mouse or press a "reverse locator" button (not shown) on keypad 5422. Responsive thereto, the cursor 5112/5304 transforms into a reverse locator pointer, such as by turning into a bright-yellow arrow. The user then moves this arrow over to the first location of interest in the planar ultrasound image 5106 or 5108 and then left-clicks or presses the "reverse locator" button again. Responsive to the selection of the first location of interest, (i) the yellow arrow disappears, (ii) the thick-slice image corresponding to the first location of interest becomes the active thick-slice member image, and (iii) the cursor 5112/5304 re-appears at a location corresponding to that first location of interest. The reverse-locating capability is usable regardless of whether the current display mode is of the thick-slice array 5102 or the enlarged thick-slice view 5302. In the event that the current display mode is the enlarged thick-slice view 5302, the currently-displayed thick-slice image is replaced, if necessary, by a different thick-slice image corresponding to the depth of the first location of interest.

An adjunctive ultrasound system according to the preferred embodiments does not supplant existing x-ray mammogram screening methods. Indeed, reference to the adjunctive ultrasound data is optional depending on the contents of the x-ray mammogram image, and for many patients it may not even be used at all. Rather, the adjunctive ultrasound system is there to assist the radiologist in performing their pre-existing professional duties with respect to "difficult" or "marginal" mammograms. As such, a medical establishment faces little risk of failure in acquiring an adjunctive ultrasound system according to the preferred embodiments. In a worst-case scenario, the adjunctive ultrasound system would be met with indifference by the entrenched "x-ray-only" believers, because it would not disturb their pre-existing routines. However, the adjunctive ultrasound system will be there standing by to assist in the "difficult" cases, and it is expected that even the "x-ray-only" believers will eventually find the system useful and will increasingly rely on it to increase their sensitivity and specificity performance.

Also within the scope of the preferred embodiments is a computer program product for instructing one or more processors to carry out one or more of the methods of the preferred embodiments, such computer program product being amenable to ready implementation by a person skilled in the art in view of the present disclosure. In one preferred embodiment, the computer program product is executed primarily by the ultrasound server 3706 of FIG. 37, with the other system devices of FIG. 37 performing simple input/output, display, and storage functions. In other preferred embodiments, the computer program product is distributed across the different systems of FIG. 37, with different algorithmic portions being carried out by different systems or subsystems. Ultrasound server 3706 comprises a computer system that includes a cabinet, a display monitor, a keyboard, and a mouse, the mouse having one or more buttons for interacting with a graphical user interface. The cabinet typically houses a CD-ROM, zip, and/or floppy disc drive, system memory and a hard drive which can be utilized to store and retrieve software programs incorporating computer code that implements the preferred embodiments, data for use with the invention, and the like. An external hard drive is also shown in FIG. 37. Although CD-ROM, zip, and floppy discs represent common computer readable storage mediums, other computer readable storage media including tape, flash memory, system memory, and hard drives can be used. Additionally, a data signal embodied in a carrier wave, such as in a network including the Internet or an intranet, can form the computer readable storage medium.

Whereas many alterations and modifications of the present invention will no doubt become apparent to a person of ordinary skill in the art after having read the foregoing description, it is to be understood that the particular embodiments shown and described by way of illustration are in no way intended to be considered limiting. By way of example, although described *supra* in terms of adjunctive ultrasound screening, in view of the present disclosure one skilled in the art would readily be able to apply the thick-slice display apparatus of the preferred embodiments in the context of computerized tomography (CT) and/or magnetic resonance imaging (MRI) environments. In each case, individual image slices generated from CT scans or MRI scans of the breast are compounded so as to form thick-slice images of slab-like portions of the breast along planes parallel to a standardized x-ray mammogram view plane, and the thick-slice images are displayed to the radiologist in close proximity to an x-ray mammogram of the breast to assist in interpreting that x-ray mammogram. Preferably, a single composite view of the whole breast is shown together with the thick-slice image views, these views having their gray-scale polarities toggled and/or remapped such that they are reminiscent of x-ray mammogram views taken from the standardized direction. By way of further example, the preferred embodiments described *supra* may also be used with different ultrasound modalities other than B-mode scans, including power or color Doppler modalities, and may also be used in conjunction with vibrational Doppler imaging (VDI) modalities. Therefore, reference to the details of the preferred embodiments are not intended to limit their scope, which is limited only by the scope of the claims set forth below.

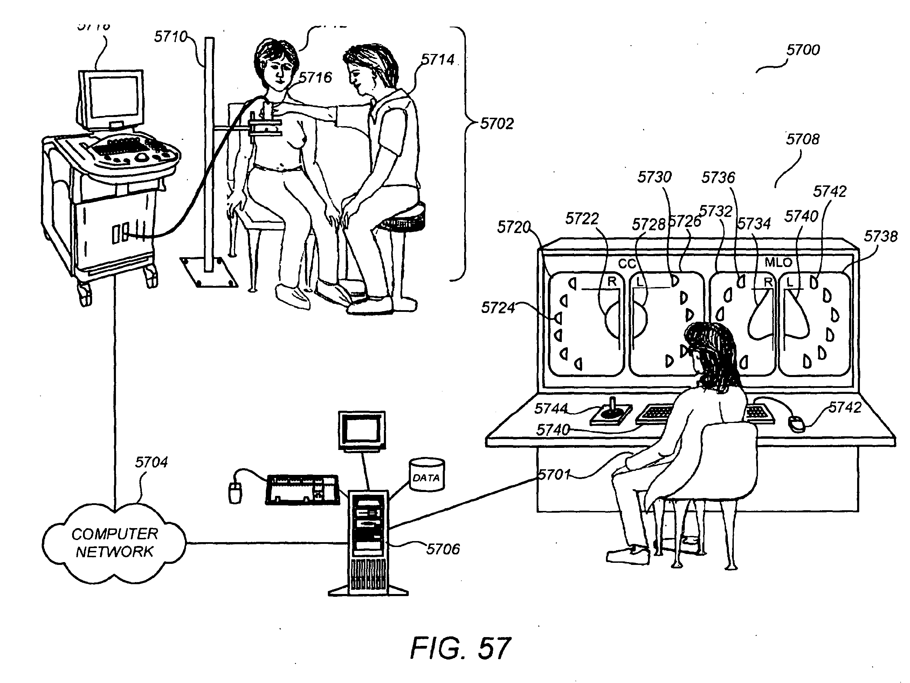

FIG. 57 illustrates a conceptual diagram of a system 5700 and associated methods for breast cancer screening using adjunctive ultrasound mammography according to a preferred embodiment. Many aspects of the system 5700 are also described in U.S. Ser. No. 10/160,836. Adjunctive ultrasound mammography refers to the acquisition and display of breast ultrasound information during the breast cancer screening process in a manner that supplements x-ray mammogram information. System 5700 comprises an ultrasound scanning station 5702, a computer network 5704, an adjunctive ultrasound server 5706, and an adjunctive ultrasound screening station 5708. Ultrasound scanning station 5702 comprises an ultrasound scanning apparatus 5710 for facilitating breast ultrasound scans of the patient 5712 by an ultrasound technician 5714. An ultrasound probe 5716 is used to scan a breast of the patient 5712, with reflected acoustic interrogation signals being processed by an ultrasound machine 5718.

Preferable to the ultrasound scanning station 5702 of FIG. 57 are ultrasound scanning unit described more fully in U.S. Ser. Nos. 60/415,385 and 10/305,936.

The ultrasound scanning apparatus 5710 supports and maintains the breast during the ultrasound scanning process. According to a preferred embodiment, the ultrasound scanning apparatus 5710 also flattens the breast along a plane parallel to a standard x-ray mammogram view plane such that resulting ultrasound images correspond more closely to standard x-ray mammogram images. In the example of FIG. 57, the standard x-ray mammogram view is the craniocaudal (CC) view. While described herein with respect to the CC view for simplicity and clarity of explanation, it is to be appreciated that the preferred embodiments are readily applied to the mediolateral oblique (MLO) view or to standard or custom x-ray mammogram views.

Although not shown in FIG. 57, the patient 5712 also undergoes a standard x-ray mammography procedure in addition to the ultrasound mammography procedure. The x-ray mammogram is usually taken during the same office visit as the ultrasonic mammography scans, although the scope of the preferred embodiments is not so limited. The ultrasound technician 5714 may be the same person or a different person as the x-ray technician who performs the x-ray mammography procedure.

If the ultrasound probe 5716 is manipulated by hand, as in the embodiment of FIG. 57, a position sensing system (not shown) is used to track the probe position such that the acquired ultrasound frames may be processed into a three-dimensional volumetric representation of the breast. It is generally preferable, however, that the ultrasound probe 5716 be machine-manipulated and controlled so as to provide reliable, consistent ultrasound scans. The ultrasound scans should be of sufficient resolution and taken at small enough intervals such that the three-dimensional volumetric representation has sufficient resolution to enable computer-aided diagnosis (CAD) algorithms to perform effectively, and such that both individual ultrasound slices and thick-slice images are of sufficient resolution to enable meaningful screening assistance to the radiologist.

As will be described further *infra,* the raw ultrasound scans may be taken directly in the standard x-ray mammogram view plane, or may alternatively be taken from a different orientation. When the raw ultrasound scans are taken directly in the standard x-ray mammogram view plane, each individual ultrasound slice is computed directly from an acquired two-dimensional ultrasound image or ultrasound frame. When the raw ultrasound scans are taken from a different orientation, each individual ultrasound slice corresponds to a plane of voxels (volume elements) in a three-dimensional volumetric representation of the breast, the plane of voxels being oriented in a direction parallel to the standard x-ray mammogram view plane. Most commonly, the three-dimensional volumetric representation of the breast is computed from the raw ultrasound scans, and then the individual ultrasound slice is extracted therefrom. However, in other preferred embodiments such as those described in Ser. No. 60/326,715, it is not always necessary to reconstruct the entire three-dimensional volumetric representation to compute the individual ultrasound slices. Stated more generally, if the raw ultrasound scans are taken in planes directly parallel to a plane of interest (CC, MLO, or a different "custom" plane of importance), each individual ultrasound slice is computed directly from an acquired two-dimensional ultrasound image or ultrasound frame, whereas if the raw ultrasound scans are taken from directions different than the plane of interest, each individual ultrasound slice corresponds to a plane of voxels in a three-dimensional volumetric representation of the breast in a direction parallel to the plane of interest.

Ultrasound machine 5718 may generally comprise any commercially available ultrasound machine having sufficient resolution, speed, and network connectivity to achieve the functionalities described herein. During or after the ultrasound scanning process, the raw ultrasound data is provided across the computer network 5704 to the adjunctive ultrasound server 5706, where the raw ultrasound data is processed into adjunctive ultrasound data that will be made available to the screening radiologist, the adjunctive ultrasound data including ultrasound slices, thick-slice images, vibrational Doppler imaging (VDI) images, CAD outputs, and other useful information. It is to be appreciated that the processing of the raw ultrasound data into the adjunctive ultrasound data may be performed by any of a variety of different computing devices coupled to the computer network 5704 and then transferred to the adjunctive ultrasound server 5706.

Although many different variations are within the scope of the preferred embodiments, in the example of FIG. 57 the adjunctive ultrasound screening station 5708 comprises four display monitors, each dedicated to a particular standard x-ray mammogram view for each breast. On a first display monitor 5720, a right CC x-ray mammogram image 5722 is displayed, with a plurality of ultrasound thick-slice thumbnail images 5724 being distributed in an arc-like pattern therearound as shown in FIG. 57. The thumbnail thick-slice images 5730 represent thick-slice portions of the left breast volume oriented parallel to the CC view plane. A second display monitor 5726 displays a left CC x-ray view 5728 and associated ultrasound thick-slice thumbnails 5730, a third display monitor 5732 displays a right MLO x-ray view 5734 and associated ultrasound thick-slice thumbnails 5736, and a fourth display monitor 5738 displays a left MLO x-ray view 5740 and associated ultrasound thick-slice thumbnails 5742. For simplicity and clarity of explanation, only the left CC view monitor 5726 is detailed herein, it being understood that similar descriptions apply to the other standard x-ray mammogram views.

Generally speaking, a thick-slice image is an integration of a plurality of substantially parallel individual ultrasound slices used to represent a slab-like or thick-slice volume of the breast. The thickness of the slab-like or thick-slice volume may lie, for example, in the range of 2 mm to 20 mm, although the scope of the preferred embodiments is not so limited. Techniques for integrating the component ultrasound slices into thickslice images according to the preferred embodiments include arithmetic averaging, geometric averaging, reciprocal averaging, exponential averaging, and other averaging methods, in each case including both weighted and unweighted averaging techniques. Other suitable integration methods may be based on statistical properties of the population of component ultrasound slices at common locations, such as maximum value, minimum value, mean, variance, or other statistical algorithms. Generally speaking, the ultrasound thick-slice images and thumbnails described herein are similar to those described in Ser. No. 10/160,836.

In the preferred embodiment of FIG. 57, at the outset of the display process, the ultrasound thick-slice thumbnails 5730 are of sufficient number and thickness to represent the entire breast volume. For example, if the compressed breast volume has a total elevation of 6 cm, there can be six individual thick-slice thumbnails each corresponding to 1 cm slab-like regions within the breast. The x-ray mammogram image 5728 is preferably displayed at full-scale. If the display monitor 5726 is sufficiently large, the thick-slice thumbnails can be replaced with full-scale thick-slice images if desired.

Adjunctive ultrasound screening station 5708 further comprises a control panel positioned near or integrated with each display monitor 5720, 5726, 5732, and 5738. In the simple example of FIG. 57 a keyboard 5740, a mouse 5742, and a joystick 5744 are provided through which user control and the manual image manipulations *infra* are achieved. It is to be appreciated that the user controls and manual image manipulations described herein are in addition to the user controls and other features described in Ser. No. 10/160,836.

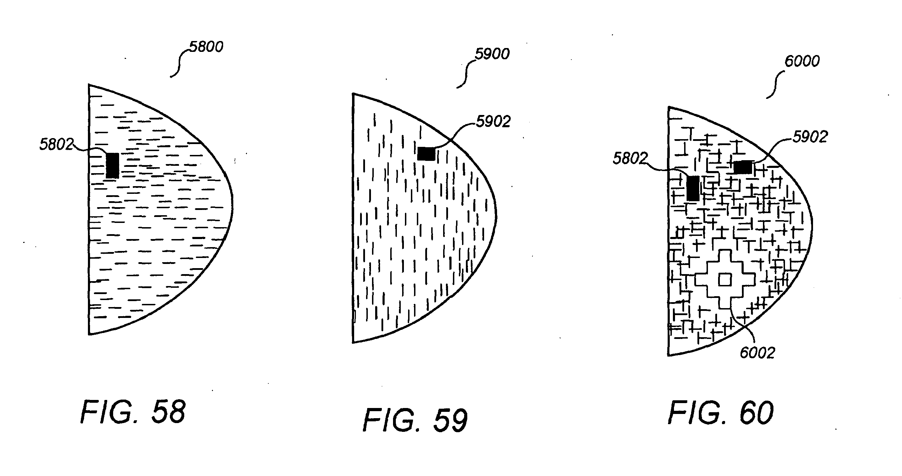

FIGS. 58-60 are conceptual diagrams intended to communicate, in a simplified hypothetical setting, the analytical assistance that an overlay of two medical images of two different modalities can provide. FIG. 58 illustrates a conceptual diagram of a first medical image 5800 of a breast according to a first imaging modality (e.g., x-ray mammogram). FIG. 59 illustrates a conceptual diagram of a second medical image 5900 of a breast according to a second imaging modality (e.g., ultrasound). FIG. 60 illustrates a conceptual diagram of a bimodal medical image 6000 formed by a superposition of the medical images 5800 and 5900. As indicated in FIG. 60, some of the features that are salient in one medical image (e.g., suspect regions 5802 and 5902) do not become enhanced or clarified by corresponding locations in the other image, and this information may be useful in determining a false positive or in further characterization of a suspect region. Conversely, some features that may not be particularly evident in either medical image may become apparent when the images are superimposed, as represented by the suspect region 6002. Once again, it is to be appreciated that the example of FIGS. 58-60 is hypothetical in nature for communicating one or more principles according to the preferred embodiments, and is not a literal portrayal of breast images. However, analogous advantages apparent to the trained eye can be enjoyed by overlay of ultrasound thick-slice images and x-ray mammogram images of a breast, or vice versa, in accordance with the preferred embodiments.

Thus, in one preferred embodiment, one or more ultrasound thick-slice images are superimposed onto a corresponding x-ray mammogram view, the thick-slice images representing a slab-like volume of the breast taken parallel to a standard x-ray mammogram view. In other preferred embodiment, one or more ultrasound thick-slice images are superimposed onto a corresponding x-ray mammogram view, the thick-slice images representing a slab-like portion of the breast that is substantially less thick than the entire breast volume. This provides the advantage, not offered by the summation ultrasound image of U.S. Pat. 5,938,613, *supra.*

In yet another preferred embodiment, one or more ultrasound images of the breast are superimposed onto a corresponding x-ray mammogram view in a manner that allows for manual vernier adjustments of the registration of the images. It has been found that the manual vernier registration adjustments of the ultrasound images with the corresponding x-ray mammogram image is of substantial benefit in image analysis. In particular, it rapidly increases the viewer's perception and appreciation of breast structures being displayed by both component images, as compared to when (i) the component images are presented side-by-side, and (ii) the component images are displayed in fixed registration. Although precise explanations might well be left to cognitive scientists, it is believed that the generally amorphous nature of the breast images makes it difficult, when placed side-by-side, to mentally carry across distance and proportion information from one image to the other. This problem is alleviated somewhat when the images are superimposed and displayed in fixed registration with each other. However, especially with the medical image modalities at hand, it is still often difficult to perceive which component image is displaying which localized patterns in the fixed-registration bimodal image. When manual, vernier registration adjustments are performed according to the preferred embodiments, the subtle shifts of entire localized patterns responsive to the user's own adjustments can substantially enhance comprehension of the different localized patterns in both component images and in the overall bimodal image.

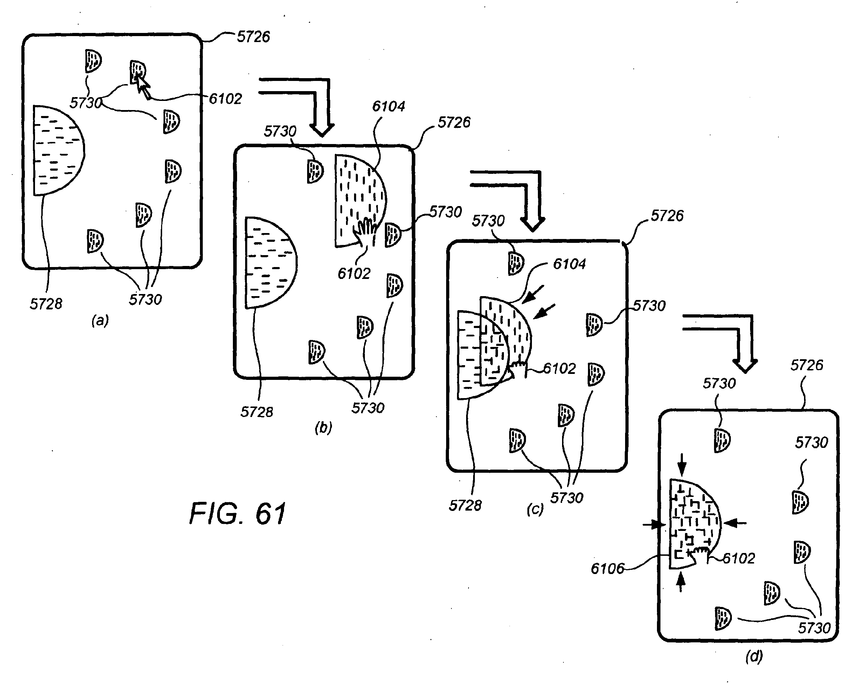

FIG. 61 illustrates the adjunct ultrasound display monitor 5726 at different intervals during an overlay of an ultrasound image onto an x-ray mammogram image 3728. In frame (a), the user first moves a cursor 6102 over a particular ultrasound thick-slice thumbnail of interest. In frame (b), upon clicking the thick-slice thumbnail, the thumbnail is expanded to a full ultrasound thick-slice image 6104 having the same spatial scale as the x-ray mammogram image 5728. At frame (c), the user clicks-and-drags the ultrasound thick-slice image 6104 over toward the x-ray mammogram image 5728. At frame (d), a bimodal image 6106 is displayed, with the user performing small, manual adjustments to the registration of the two component images forming the bimodal image. As the component images begin to overlap in frame (c), a mixing algorithm described further *infra* is used that approximates the visual effect of placing a conventional x-ray mammogram film on a light box, and superimposing a second transparency thereon containing a printed version of the thick-slice ultrasound image, although in general any of a variety of different mixing algorithms can be used to superimpose the component images.

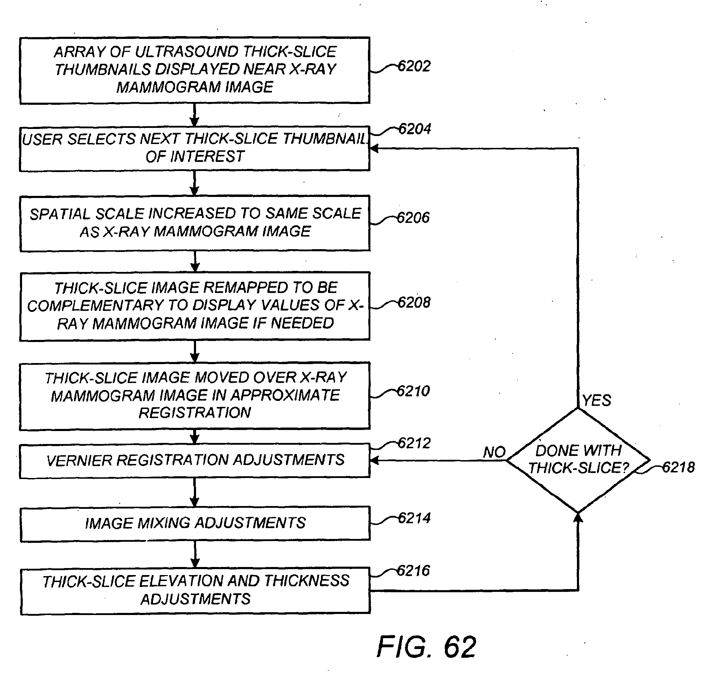

FIG. 62 illustrates steps for breast cancer screening using an x-ray mammogram with adjunct ultrasound overlays according to a preferred embodiment. The steps of FIG. 62 are described with respect to a single x-ray mammogram view/breast pair (e.g., CC view for left breast), it being understood that analogous steps for the other breast/view pairs are being carried out serially or in parallel with the steps of FIG. 62. At step 6202, an array of ultrasound thick-slice thumbnails is displayed near an x-ray mammogram image. At step 6204, the user selects an ultrasound thick-slice thumbnail of interest. At step 6206, the spatial scale of the ultrasound thick-slice thumbnail is increased to the same scale as the x-ray mammogram image. At step 6208, the thick-slice ultrasound image is remapped, if necessary, to be complementary to the display values of the x-ray mammogram image.

At step 6210, the thick-slice image is moved over the x-ray mammogram image in approximate or "starter" registration therewith. While in the embodiments of FIG. 61 this is described in terms of a manual click-and-drag process, the scope of the preferred embodiments is not so limited. In an alternative preferred embodiment, this step is performed automatically by the adjunctive ultrasound display system using methods known in the art, thereby saving radiologist time. Automated registration methods include those based on skin lines, nipple position, chest wall position, artificial external markers, natural internal feature markers such as visible microcalcifications, and/or other methods. Methods based on artificial external markers include those using a single "BB" or similar marker placed near the nipple, as well as those using three or more BBs. Where multiple artificial markers are used, the scaling of the component images may be contracted or expanded in one or both directions as necessary to get all of the markers to line up.

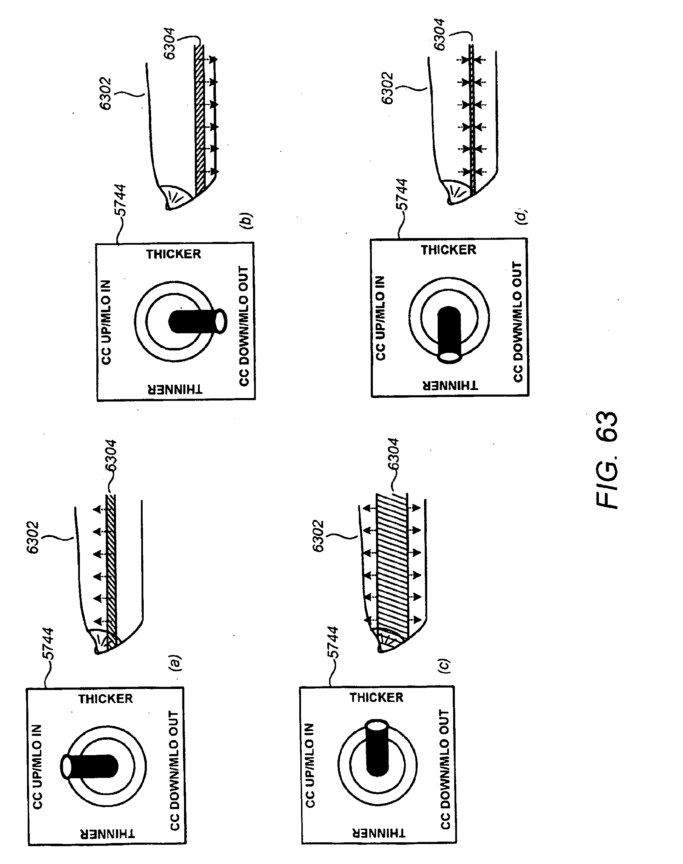

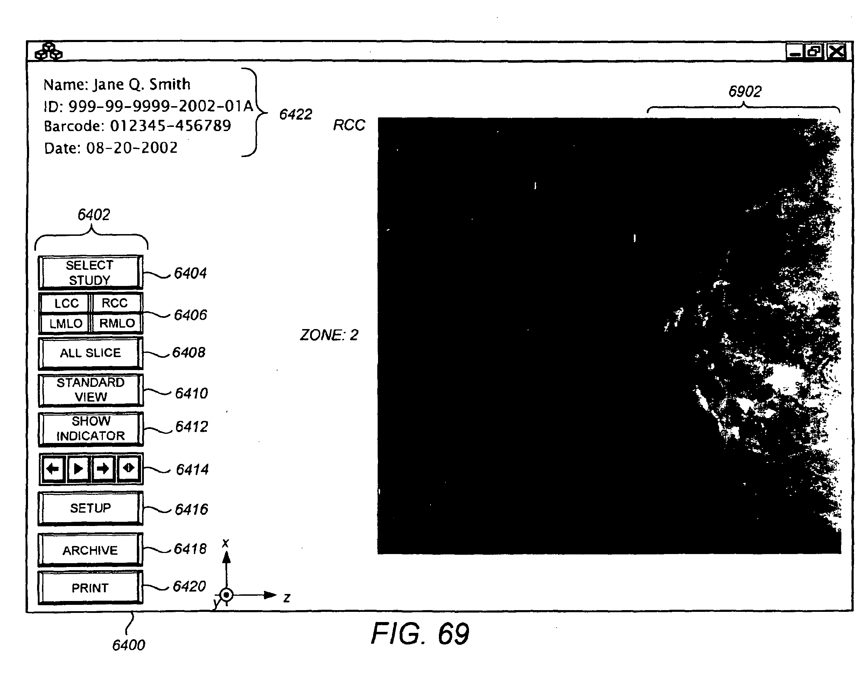

At step 6212 the user performs manual vernier registration adjustments. When the previous initial registration step 6210 is performed automatically rather than by manual manipulation, the vernier adjustments may be based on perceived registration differences in the initial registration. On the other hand, even if the initial registration is very good or perfect, the user still manipulates the component images to be slightly out-of-registration, and then moves them back into registration, in order to experience the benefits of the vernier image manipulations described *supra.* At step 6214 the user may optionally perform mixing parameter adjustments as described *infra* with respect to FIGS. 69-70. At step 6216 the user may optionally perform thick-slice elevation and thickness adjustments according to a "rolling thick-slice" method described *infra* with respect to FIG. 63. At step 6218 the user may choose to continue the adjustment and analysis process or may proceed to another image view or thick-slice image.

FIG. 63 illustrates a top view of the joystick control 5744 of the adjunctive ultrasound screening station 5708 of FIG. 57, along with a conceptual side view of a breast 6302 and a slab-like thick-slice region 6304 therein, for further describing the "rolling thick-slice" method of step 6216 of FIG. 62, *supra.* For the CC view, when the joystick 5744 is moved forward (frame (a)) or backward (frame (b)), the elevation of the thick-slice region 6304 relative to a bottom compression plate is adjusted upward or downward, respectively, within the breast volume. For the MLO view, this elevation metric corresponds to a distance from the vertically-oriented compression plane for that view. More generally, if a non-standard plane is used, the elevation corresponds to a distance from one of the compression plates used to compress the breast. When the joystick 5744 is moved right (frame (c)) or left (frame (d)), the thickness of the slab-like region whose data is used to form the thick-slice image is increased or decreased, respectively. Accordingly, the user may easily navigate throughout the breast volume, and may easily select between thinner and thicker-slice regions to compare to the x-ray mammogram. The thick-slice ultrasound image gently morphs along a continuum of two-dimensional representations in an intuitive manner that allows the radiologist to readily navigate the breast in both a positional sense (elevation) and abstractional sense (slab thickness).

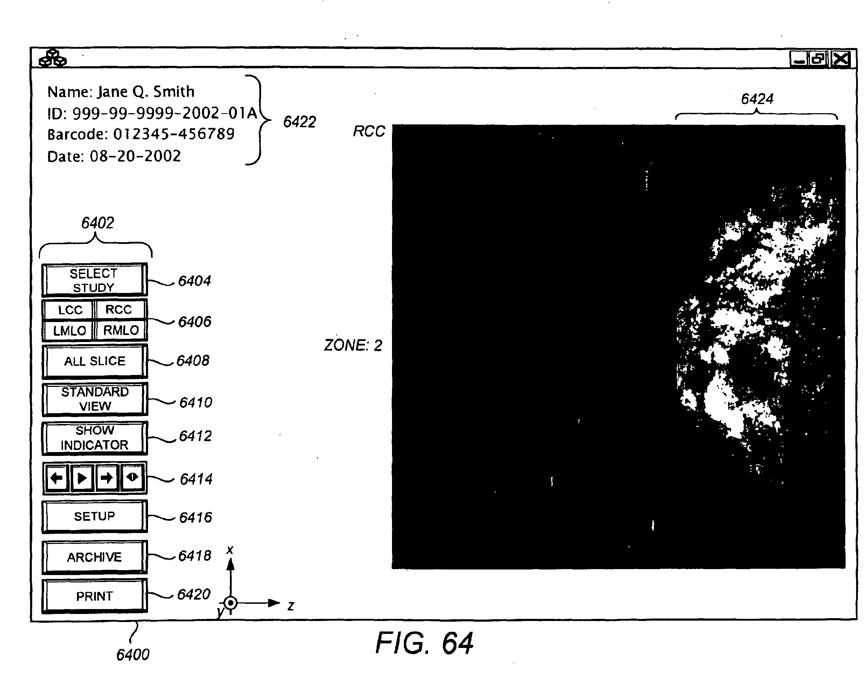

FIGS. 64-70 illustrate a user display 6400 according to a preferred embodiment at different points in a medical image superposition process according to a preferred embodiment. The user display 6400 comprises a set of selection buttons 6402, a patient identification display 6422, and one or more medical images as described herein. Generally speaking, in addition to the capabilities described herein, all of the capabilities of the user display of Ser. No. 10/160,836, are incorporated into the user display 6400, such as the ability to display multiple thumbnail thick-slice images, select and expand a given thumbnail into a full-scale image, analyze individual slices and cine-presentations thereof throughout the breast volume, etc., as facilitated by the selection buttons 6404-6420. In the preferred embodiment of FIGS. 64-70, it is an x-ray mammogram image that is manually superimposed over a static thick-slice ultrasound image.

FIG. 64 illustrates the user display 6400 after a particular thick-slice image from a particular zone for the RCC view has been selected and expanded into the ultrasound image 6424. The ultrasound image 6424 is an 8-bit grayscale image with the gray scale selected to be reminiscent of a film-based x-ray mammogram as displayed on a light box, *i.e.,* brightest = 255 = Dₘₐₓ = high acoustic echo and darkest = 0 = low acoustic echo.

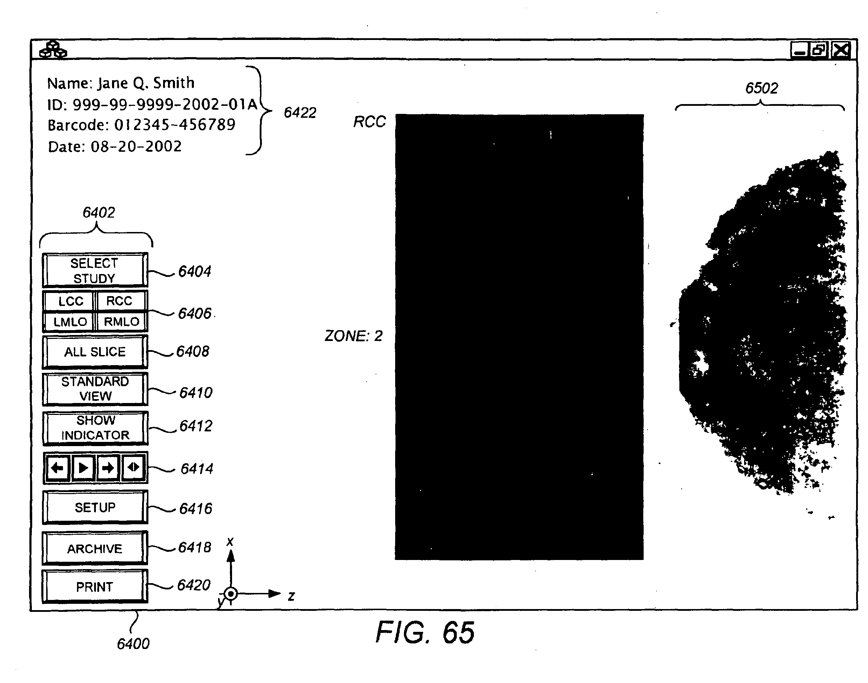

FIG. 65 illustrates the user display 6400 upon user pressing of a first superposition key, such as the keyboard letter "1." Responsive to this command, the ultrasound image 6424 is inverted such that brightest = 255 = Dₘₐₓ = low acoustic echo and darkest = 0 = high acoustic echo.

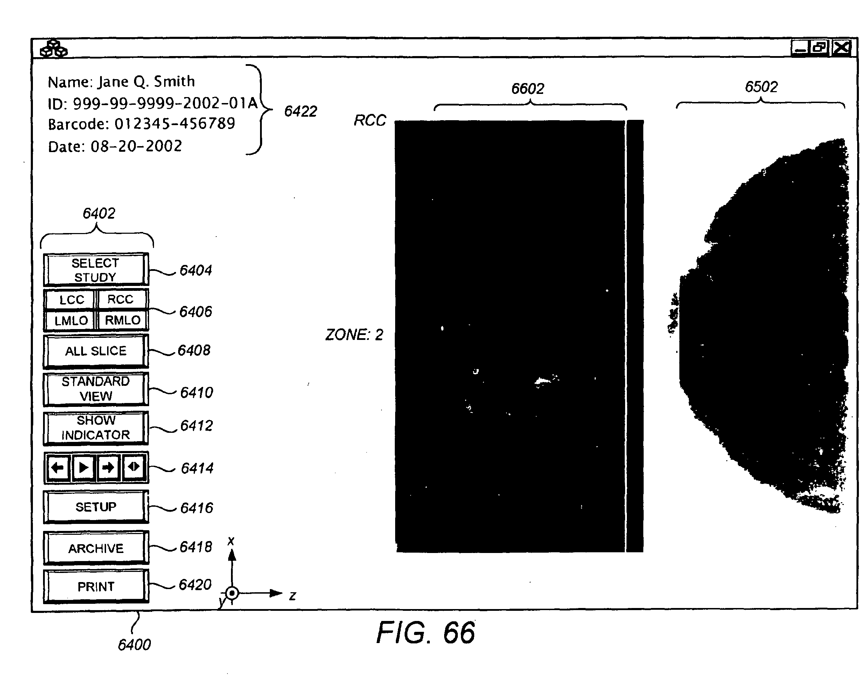

FIG.66 illustrates the user display 6400 upon user pressing of a second superposition key, such as the keyboard letter "M." Responsive to this command, an x-ray mammogram image 6602 is displayed near the ultrasound image 6502. The x-ray mammogram image 6602 is an 8-bit grayscale image with the gray scale set to mimic the appearance of a film-based x-ray mammogram as placed on a light box, i.e., brightest = 255 = Dₘₐₓ = highly radio-opaque and darkest = 0 = highly radio-transparent.

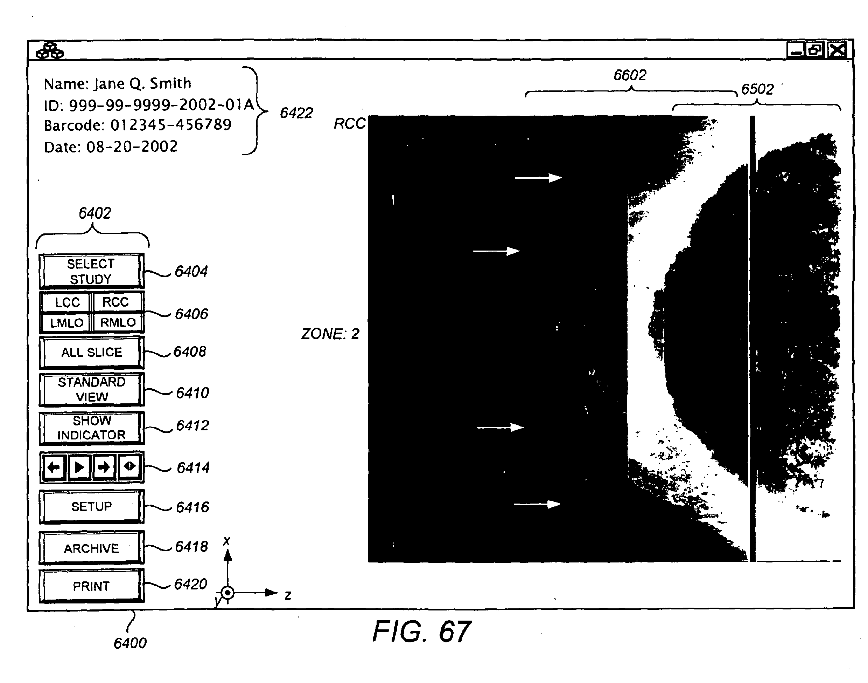

FIG. 67 illustrates the user display 6400 as the user manually moves the x-ray mammogram image 6602 over the ultrasound image 6502. In one preferred embodiment, the user manually causes lateral movement by pressing of the LEFT and RIGHT arrow keys of the keyboard and vertical movement by pressing the UP and DOWN arrows of the keyboard. At areas of image overlap, a pixelwise mixing algorithm is used to achieve superposition of the medical images. In one preferred embodiment illustrated in FIGS. 67-70, the mixing algorithm is given by Eq. (4) below, where (x,y) represents the coordinates of the pixel in question, u(x,y) is the value of the inverted ultrasound image 6502 at that location, m(x,y) is the value of the x-ray mammogram image 6602 at that location, d(x,y) is the ultimate output display value at that location, and Fₘᵢₓ is a scalar mixing factor valued between zero and unity: $d \left(x⁢y\right) = {F}_{mix} ⋅ m \left(x⁢y\right) + \left(1-{F}_{mix}\right) ⋅ u \left(x⁢y\right)$

Preferably, the mixing factor Fₘᵢₓ is dynamically user-adjustable, but will be assigned a default starting value of about 0.5. Any of a variety of mixing algorithms may be used in accordance with the preferred embodiments, and may be varied according to the specific display hardware used and the quality and dynamic range of the medical images used. In another preferred embodiment, the mixing algorithm is designed to closely emulate a film-based superposition scenario comprising (i) a light box, (ii) a standard film-based x-ray mammogram placed thereon (dark = radio-transparent, clear = radio-opaque), and (iii) an ultrasound image printed on a clear film placed thereon, the ultrasound image being printed such that dark = high acoustic echo and clear = low acoustic echo. This algorithm is given by Eq. (5) below, where Dₘₐₓ is the brightest value available on the display monitor: $d \left(x⁢y\right) = {D}_{max} * \left[m\left(x⁢y\right)/{D}_{max}\right] * \left[u\left(x⁢y\right)/{D}_{max}\right]$

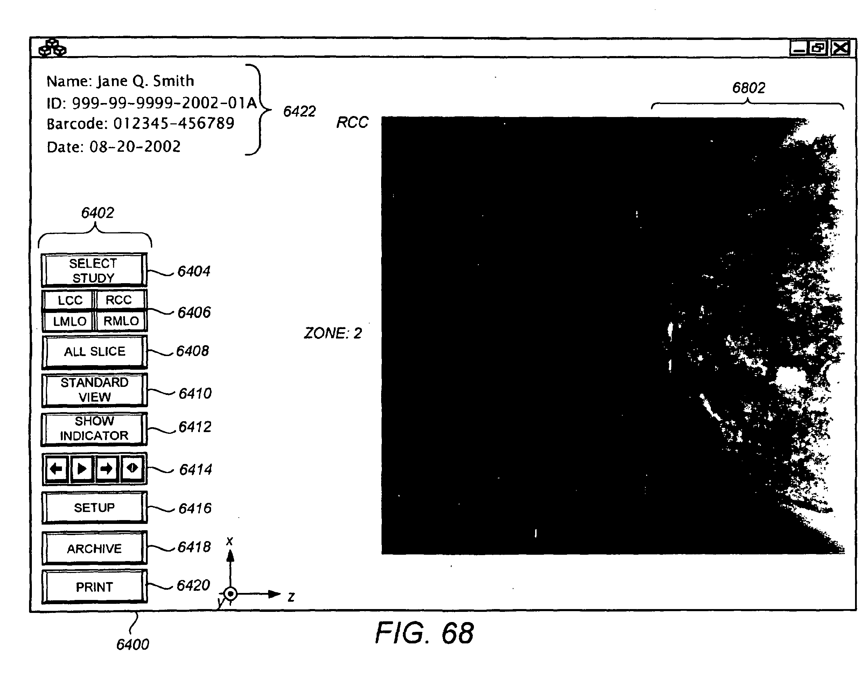

FIG. 68 illustrates the user display 6400 as registration of the component images is substantially achieved to form a bimodal image 6802. While viewing the bimodal image 6802, the user will usually be performing small manual adjustments of the registration of the component images by using the arrow keys, as described *supra.* During this process, the user may adjust the elevation and/or thickness of the thick-slice ultrasound image component in a "rolling thick slice" method described *supra* with respect to FIG. 63. Additionally, the mixing factor Fₘᵢₓ may be dynamically adjusted, for example by using the "+" and "-" keyboard keys, so as to let one or the other of the component medical images predominate.

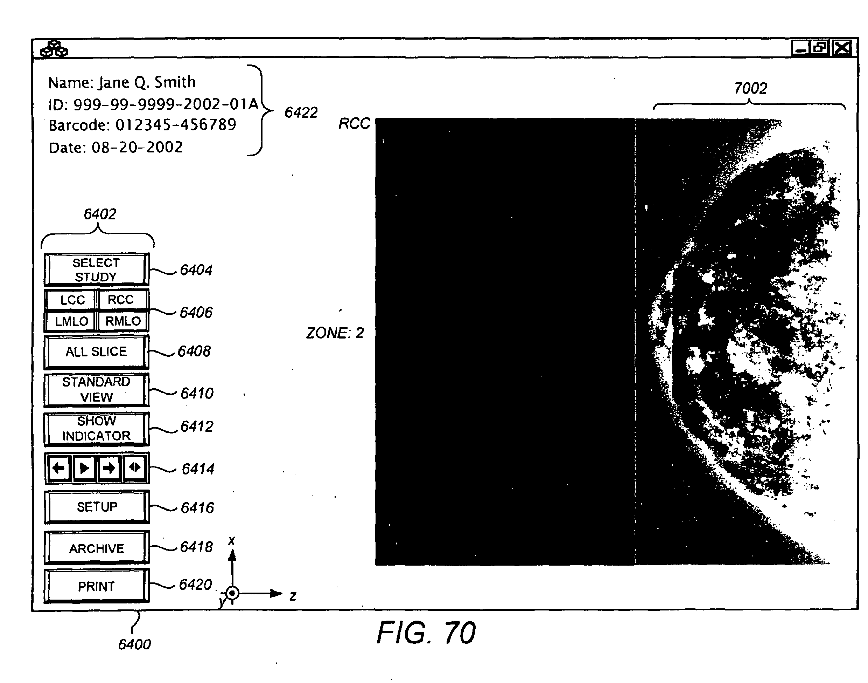

FIG. 69 illustrates the user display 6400 as the mixing factor Fₘᵢₓ is adjusted closer to zero to let the x-ray mammogram image component predominate. FIG. 70 illustrates the user display 6400 as the mixing factor Fₘᵢₓ is adjusted closer to unity to let the ultrasound image component predominate.

It is to be appreciated that those preferred embodiments described *supra* in which both component images are displayed in electronic format are presented by way of non-limiting example only, and any of a variety of other component medical image display and bimodal image formation methods is within the scope of the preferred embodiments. For example, in another preferred embodiment, the x-ray mammogram is provided on a conventional x-ray film and placed on a lightbox, the lightbox being capable of backprojecting an ultrasound image onto its display surface. After reviewing the x-ray film in a conventional fashion, the radiologist may then activate this backprojection feature and thereby view an overlay of the x-ray mammogram image on the ultrasound image. In another preferred embodiment, a high-brightness computer display is used to illuminate an x-ray film for conventional viewing in a first configuration, and to display an ultrasound image in a second configuration that projects through the x-ray film to achieve an overlay of the x-ray mammogram image on the ultrasound image. In still another preferred embodiment, the x-ray mammogram is displayed digitally on a monitor or backprojected onto a light box, while the ultrasound image is printed onto a translucent film and placed on the monitor or light box.

In still another preferred embodiment, a standard film-based x-ray mammogram is placed on a lightbox, and an ultrasound image printed on a clear film is placed thereon and manually manipulated by the user. In such preferred embodiment, several thick-slice ultrasound images are printed on separate transparent sheets, which can be interchanged by the user as needed to achieve elevation variations, and which can be doubled-up or tripled-up as needed to represent thicker thick-slice regions of the breast volume.

In each case, the overlying and underlying medical images should be complementary to each other, and should be amenable to manual vernier registration adjustment by the user. Any of a variety of manual vernier registration adjustment techniques may be employed between the overlaid image and the underlying image.

For those preferred embodiments in which a first medical image is provided in film or other hardcopy format while a second medical image is provided in electronic display format, a bar code reader is included in the viewing station hardware. The bar code reader reads a bar code that is placed on each hardcopy image, using that bar code information to locate and access the second medical image from the system database.

Any of a variety of manual vernier registration adjustment techniques may be employed between the overlaid image and the underlying image, including touch-screen control, mouse or joystick control, trackball control, mechanical control, other techniques, or a combination of these techniques. For example, where both the x-ray image and ultrasound image are electronically displayed on the same display monitor, a click-and-drag technique using a computer mouse or joystick may be used. If one or both of the component images is on film or other hardcopy format, the radiologist may slide the overlying image across the underlying image by hand. Alternatively or in conjunction therewith, where the underlying image is achieved by backprojection or computer display, the underlying image may be manually shifted using by computer mouse click-and-drag, joystick, or trackball control.

Whereas many alterations and modifications of the present invention will no doubt become apparent to a person of ordinary skill in the art after having read the foregoing description, it is to be understood that the particular embodiments shown and described by way of illustration are in no way intended to be considered limiting. By way of example, although described *supra* in terms of adjunctive ultrasound screening, in view of the present disclosure one skilled in the art would readily be able to apply the thick-slice display apparatus of the preferred embodiments in the context of computerized tomography (CT) and/or magnetic resonance imaging (MRI) environments. In each case, individual image slices generated from CT scans or MRI scans of the breast are compounded so as to form thick-slice images of slab-like portions of the breast along planes parallel to a standardized x-ray mammogram view plane, and the thick-slice images are displayed in close proximity to an x-ray mammogram of the breast in a way that allows them to be manually translated and superimposed thereon by the radiologist. The elevation and/or depth CT or MRI thick-slice images may be adjusted by manual joystick control or other control mechanism.

By way of further example, while described *supra* in terms of the superposition of only a single thick-slice ultrasound image over an x-ray mammogram (or vice versa), in other preferred embodiments two or more thick-slice ultrasound images are superimposed with the x-ray mammogram. Moreover, the two or more thick-slice images may correspond to non-adjacent portions of the breast volume. In still other preferred embodiments, it has been found that useful observations may be made by superimposing two ultrasound thick-slice images taken from the same region of the breast at different points in time, e.g., spaced 1 year apart, to assist in screening for changes in the breast over time. The two superimposed thick-slice images may be superimposed upon an x-ray mammogram image, or alternatively can be displayed without the x-ray mammogram image. In other preferred embodiments, three or more thick-slice ultrasound images are superimposed corresponding to three or more different points in time. In still other preferred embodiments, a plurality of x-ray mammogram images taken at different points in time can be superimposed with a plurality of thick-slice ultrasound images taken at different points in time. Methods for implementing systems according to these preferred embodiments would be readily apparent to those skilled in the art in view of the present disclosure.

By way of further example, while described *supra* in terms of the superposition of images from two different modalities, the features and advantages of the preferred embodiments are readily applied to the superposition of medical images from three different modalities, e.g., ultrasound, x-ray mammogram, and MRI. According to a preferred embodiment, for those modalities that yield three-dimensional information, thick-slice images are derived therefrom and used for overlay purposes. Exemplary combinations may include overlays of: (i) x-ray mammogram, ultrasound, and MRI; (ii) (ii) x-ray mammogram, ultrasound, and CT; (iii) CT, ultrasound, and MRI; (iv) CT, x-ray mammogram, and MRI; and others. The features and advantages of the preferred embodiments are also readily applied to the superposition of medical images from four or more different imaging modalities. For such multi-modality cases, in order to reduce the amount of clutter and to derive more utility from the overlays, the individual medical images are preferably enhanced prior to or during overlay so as to display the most salient features revealed by its respective imaging modality. By way of example, the ultrasound image will be spatially low-pass filtered to concentrate on larger features, it being understood that very small structures such as microcalcifications are not strongly revealed by the ultrasound modality. The reduced amount of speckle from the low-pass filtering will reduce the amount of clutter in the multi-modality overlay image.

Moreover, it is to be appreciated that the features and advantages of the preferred embodiments are applicable to medical imaging formats not currently contemplated for use in large-scale breast cancer screening programs. For example, phase information from holographically encoded medical images may be interferometrically combined to achieve the medical image superpositions, or other types of time- or space-based modulation methods may be used to encode and superimpose the medical images. Therefore, reference to the details of the preferred embodiments are not intended to limit their scope, which is limited only by the scope of the claims set forth below.

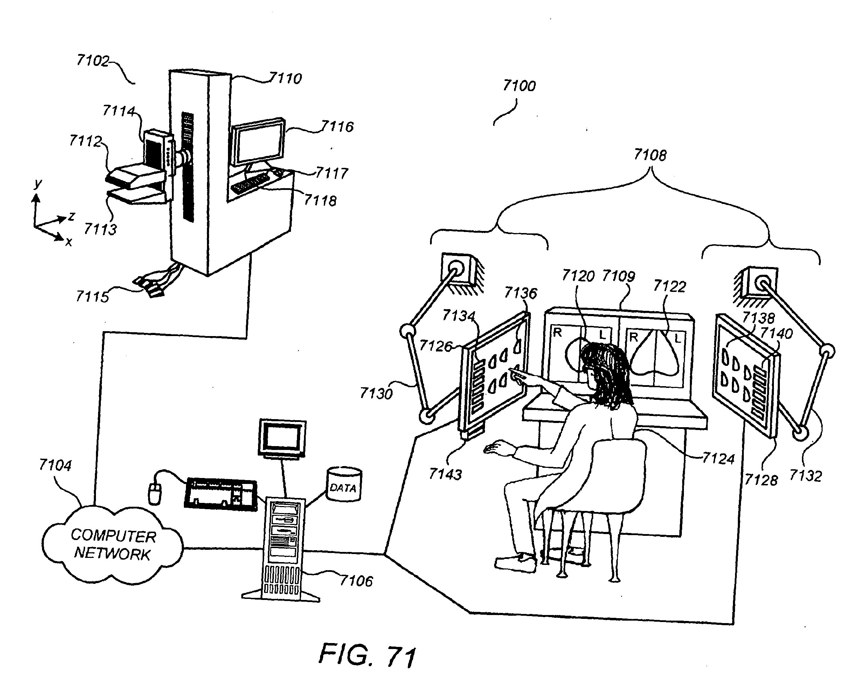

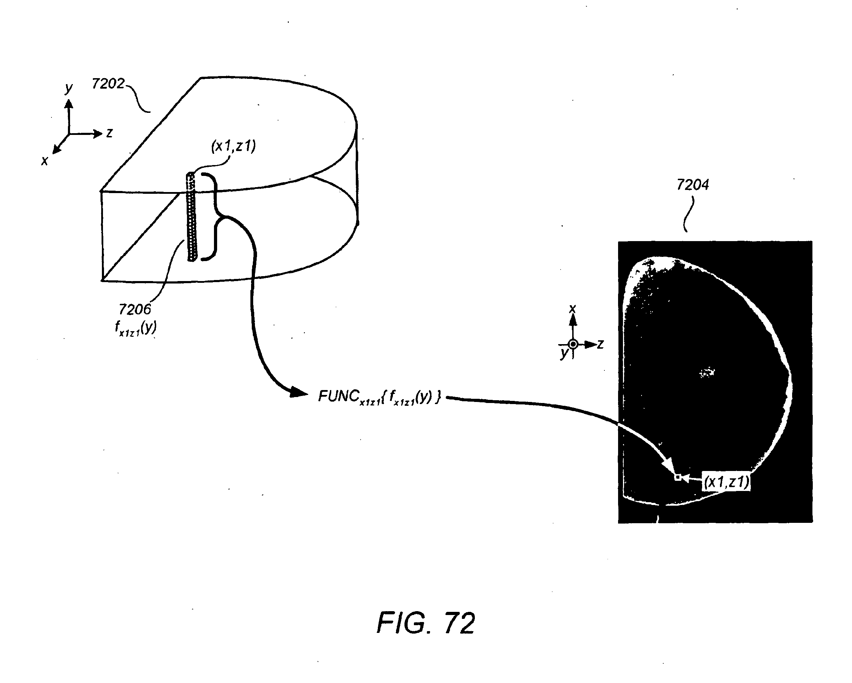

FIG. 71 illustrates a conceptual diagram of a system 7100 and associated methods for breast cancer screening using adjunctive ultrasound mammography into which the preferred embodiments described herein are advantageously incorporated. FIG. 72 illustrates a conceptual diagram of a three-dimensional array 7202 comprising ultrasound scan values for a particular thick-slice volume within a breast substantially parallel to the CC view plane, and a corresponding thick-slice image 7204 computed therefrom. A particular location (x1,z1) in the thick-slice image 7204 is computed from a voxel column 206 corresponding to the point (x1,z1). The voxel column can be represented by the function f_{x1z1}(y). The thick-slice pixel value at (x1,z1), denoted herein as tsval(x1,z1), is equal to an integration algorithm FUNC operating on the voxel column, that is, tsval(x1,z1) = FUNC{f_{x1z1}(y)}. According to a preferred embodiment, the integration algorithm FUNC is a neighborhood-dependent function that depends on the properties of a 3-D neighborhood surrounding the voxel column 7206. In FIG. 72 and the rest of the examples herein, the thick-slice image is presented using an inverted convention as explained further *infra.*

Once the viewer's attention is drawn to a particular location on the thick-slice image, the viewer can select that location and view planar ultrasound images passing through that location of interest, as described in U.S. Ser. No. 10/305,936, for a more detailed examination of the breast. The planar ultrasound images corresponding to the selected location are preferably displayed in real-time adjacent to the thick-slice image as the selected location is manipulated by a mouse, trackball, stylus, or the like.

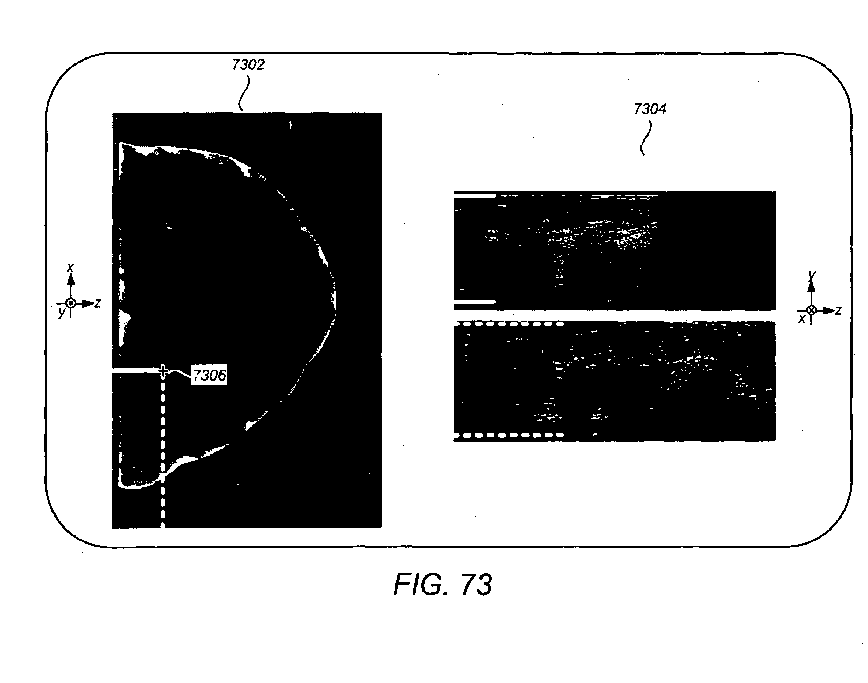

According to one preferred embodiment, the array of thick-slice images consists of a single thick-slice image representing the sonographic properties of substantially the entire breast volume. When this single thick-slice image is generated using the preferred neighborhood-dependent integration algorithm, the reduction in sensitivity brought about by using a lesser number of thick-slice regions is reduced. Advantageously, in this preferred embodiment the viewer can fix their attention on a single, static image representing the entire breast volume without being required to shift among several different thick-slice representations. This can increase viewer stamina and increase the number of cases reviewed per unit time, thereby reducing overall costs of the screening process. Also, where only one thick-slice image is displayed, there is more room on the display monitor and the image can be full-scale or even larger than full-scale. The single thick-slice image can serve as a useful "guide" or "road map" for viewing the planar ultrasound images. A conceptual example of a single thick-slice image 7302 is illustrated in FIG. 73, along with planar ultrasound images 7304 corresponding to the current position of the cursor 7306.

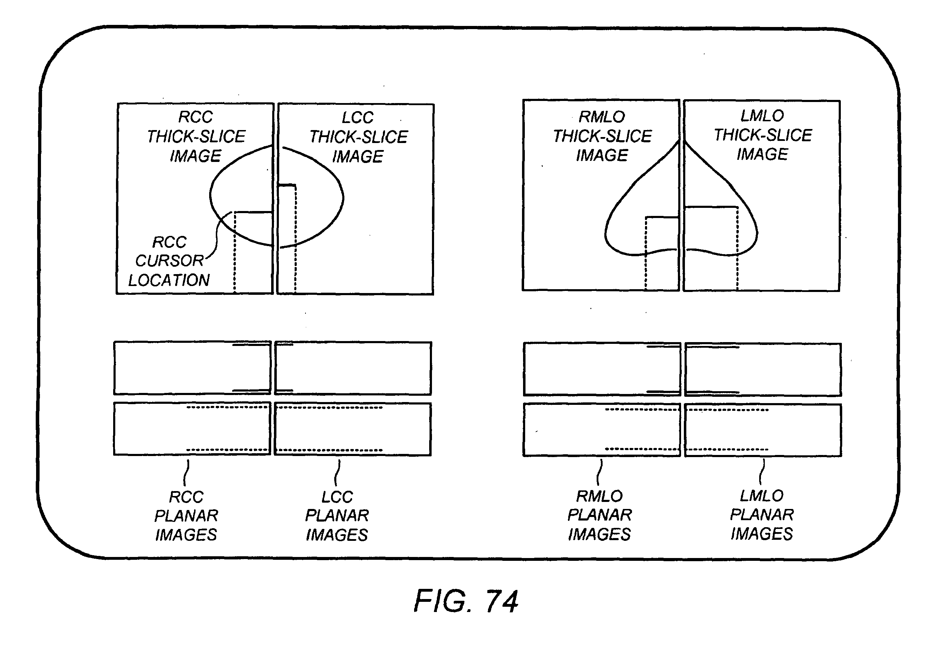

According to another preferred embodiment in which a single-member thickslice image array is used, the user display is configured to closely resemble a conventional x-ray mammogram viewing station layout. This configuration is made possible because, just as with x-ray mammogram views, there is now only a single ultrasound-based image for that breast and view. Thus, for example, thick-slice images for the LCC and RCC view can be placed on the left hand side of the monitor/view station, and thick-slice images for the LMLO and RMLO views can be placed on the right-hand side of the monitor/view station. The real-time planar ultrasound image displays for each view can be placed directly therebelow or toggled therewith. An example of this preferred embodiment is illustrated in FIG. 74.

In other preferred embodiments, the number of thick-slice images can be increased to two or more thick-slice images. In these preferred embodiments there are trade-offs incurred as the number of thick-slice images increases, wherein each increase in the number of thick-slice images brings about an increase in the viewing load and a decrease in the possible size of the images, but likewise increases sensitivity performance. Also, where more than one thick-slice image is display, mass shadowing effects can be more directly observed.

According to one preferred embodiment, the neighborhood-dependent integration algorithm FUNC comprises a fixed *method* for voxel-column integration, but varies the *parameters* for that algorithm according to neighborhood characteristics. In one such exemplary preferred embodiment, for every thick-slice pixel the integration method comprises weighting the voxels of the corresponding voxel column by a weighting vector and then summing the results, the weighting vector being computed according to neighborhood characteristics around that voxel column. This can be summarized by Eq. (6) below, where N is the number of elements of the voxel column f_{xz}(y) in the thick-slice volume at (x,z) and W_{xz}(n) is the weighting vector: $tsval \left(x⁢z\right) = FUNC \left\{{f}_{xz}\left(y\right)\right\} = {\sum }_{n = 1}^{N} {W}_{xz} \left(n\right) ⁢ {f}_{xz} \left({y}_{n}\right)$

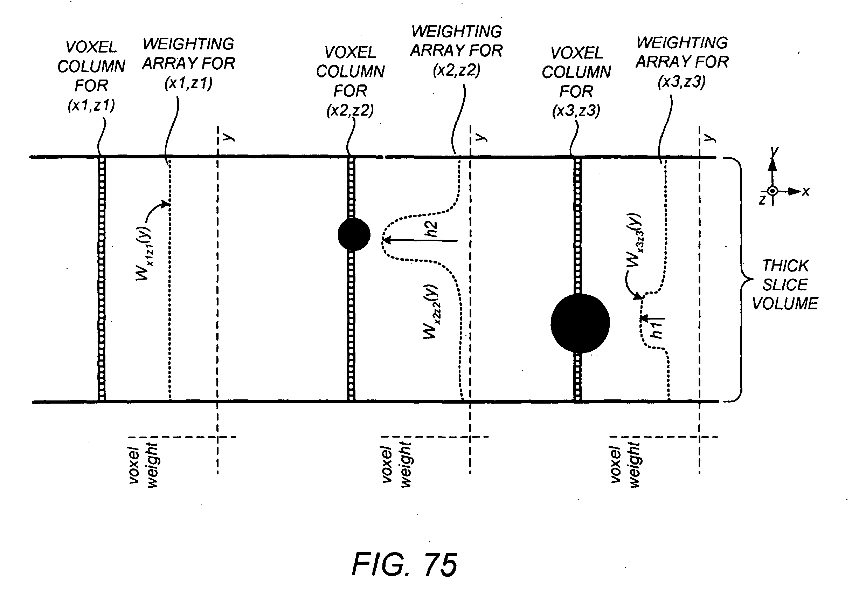

Using known three-dimensional segmentation techniques, the locations and sizes of masses in the thick-slice volume are identified. Any of a variety of three-dimensional segmentation algorithms can be used, including local thresholding algorithms, 3-D volume growing algorithms, or other algorithms as described, for example, in U.S. 6,317,617 to Gilhuijs, Giger, and Bick, which is incorporated by reference herein. For a given voxel column, the weighting vector comprises peaks at locations lying within a mass lesion and valleys elsewhere, thus causing the resulting thick-slice image to emphasize mass lesions in the output. Only masses in a predetermined size range are segmented. In one preferred embodiment, the peak magnitude of the weighting vector is proportional to the inverse of the size of the segmented lesion passing through that voxel column. This boosts the amplification of smaller mass lesions within the desired size range in the thickslice image which would otherwise be more easily "drowned out" by the rest of their voxel column data. FIG. 75 conceptually illustrates an example of the dependence of W_{xz}(n) on the neighborhood characteristics around the voxel columns, wherein (i) if no lesion is segmented, as with the voxel column at (x1,z1) the weighting vector is a constant, and (ii) if smaller segmented lesions (within the predetermined size range) are more heavily weighted (see (x2,z2)) than larger segmented lesions (see (x3,z3)).

According to another preferred embodiment, the thick-slice image generation algorithm can be run several times for different predetermined size ranges. While examining the thick-slice image(s), the viewer may optionally manipulate a user control, such as an onscreen sliding control (similar to a Windows volume control), to vary the predetermined size range. Accordingly, the viewer can first screen for large masses, for example, and then screen for smaller masses in the thick-slice image(s).

According to another preferred embodiment, the neighborhood-dependent integration algorithm FUNC varies the actual voxel-column integration *method* itself according to neighborhood characteristics. In this preferred embodiment, three-dimensional computer-assisted detection (CAD) algorithms are used to analyze and classify the segmented mass lesions and determine a likelihood of malignancy for each mass lesion. Any of a variety of three-dimensional CAD classification algorithms can be used, including artificial neural network algorithms or other algorithms as described, for example, in U.S. 6,317,617, *supra.* In computing the thick-slice image, different voxel-column integration algorithms are applied depending on the nature of the mass that intersects a given voxel column.

By way of example, where a segmented mass is classified as a probable liquid-filled cyst, an anechoic mass with a hyper-echoic "shadow", the voxel-column integration algorithm FUNC for the associated voxel columns is a straight column-averaging method because these masses are relatively harmless and do not warrant amplification. As a further example, where a segmented mass of size "S" is classified as a probable cancer, a hypo-echoic mass with a hypo-echoic shadow, the voxel-column integration algorithm FUNC for the associated voxel columns affirmatively discards all voxels lying "S/2" voxels above and "3S/2" below the mass boundary and averages the remaining voxels. This allows the nature and extent of the hypo-echoic shadow to have an influence on the mass visibility and normally results in a highly emphasized mass. Where a segmented mass of size "S" is classified as a probable fibroadenoma, a hyper-echoic mass with no "shadow" effect, FUNC comprises the weighted-sum algorithm of FIG. 75 having a weighting vector peak inversely proportional to the lesion size can be used.

In other preferred embodiments, the numerical suspiciousness metrics generated by the CAD algorithms themselves are incorporated into the voxel-column integration algorithms, e.g. to increase the visibility of higher-suspiciousness mass lesions in the output. Alternatively or in conjunction with the above-described thick-slice generation methods, CAD markers such as circles, triangles, stars, arrows, and the like can be superimposed on the thick-slice image display to direct the attention of the viewer to particular suspicious areas. These can optionally be accompanied by ASCII text indicators of suspiciousness levels or other annotations. Coloring techniques can also be used, such as by incorporating color aspects into the thick-slice pixel generation algorithms, by superimposing color highlighters on the output display, by using color-coded markers, and the like.

As described in U.S. Ser. No. 10/305,936, according to one preferred embodiment the displayed thick-slice images are inverted, i.e., they represent high acoustic reflections as "dark" and low acoustic reflections as "bright." This is in distinction to a standard ultrasound display convention in which low acoustic reflections are displayed as "dark" and high acoustic reflections are displayed as "bright." Preferably, the voxels of the three-dimensional ultrasound array are individually inverted prior to generation of the thick-slice images therefrom, although the scope of the preferred embodiments is not so limited. In other preferred embodiments, thick-slice images are computed from the original standard-convention volumetric representation, and then inverted prior to display.

A preferred embodiment relating to a cine display of a thick-slice image is now described. As described in U.S. Ser. No. 10/305,936, according to one preferred embodiment, a cine display or "moving" thick-slice image can be displayed to the viewer, either under framewise control of the user or as an automated sequence. To further assist the viewer, an iconic representation indicating the vertical depth within the breast of the current thick-slice region is presented. Superimposed on the icon are markers positioned at heights corresponding to planes containing mass lesions located by the three-dimensional CAD algorithm. The markers can be coded in shape, color, or with accompanying text to communicate the type of lesion and/or a degree of suspiciousness. In this manner the viewer's attention can be drawn to particular frames during the cine playback.

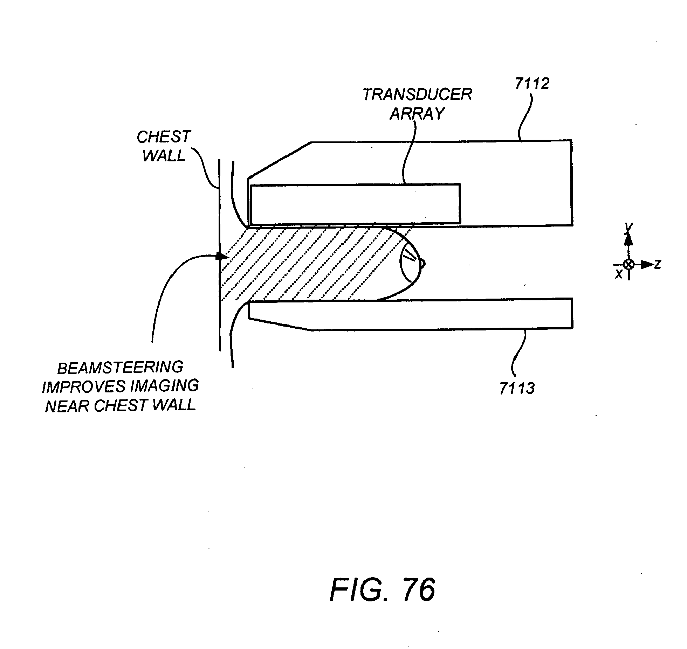

A preferred embodiment relating to a breast ultrasound scanning apparatus is now described. The breast ultrasound scanning apparatus is similar to that described in U.S. Ser. No. 10/305,936 and Ser. No. 60/415,385, and is further configured to improve breast imaging near the chest wall by steering the beam of the ultrasound probe in toward the chest wall. This can be done during a second, separate sweep of the breast, or alternatively the steered-beam frames can be interleaved with the non-steered frames. The steering angle can be up to a range between 30 and 45 degrees. FIG. 76 illustrates a cut-away side view of a scanning apparatus during a breast scan illustrating this principle according to the preferred embodiments. Advantageously, imaging near the chest wall is improved without affecting patient comfort. Ultrasonic beam steering for increasing visibility of a biopsy needle is described in U.S. 6,524,247, which is incorporated by reference herein.

Whereas many alterations and modifications of the present invention will no doubt become apparent to a person of ordinary skill in the art after having read the foregoing description, it is to be understood that the particular embodiments shown and described by way of illustration are in no way intended to be considered limiting. By way of example, although described *supra* in terms of adjunctive ultrasound screening, in view of the present disclosure one skilled in the art would readily be able to apply the thick-slice display apparatus of the preferred embodiments in the context of computerized tomography (CT) and/or magnetic resonance imaging (MRI) environments. By way of further example, the preferred embodiments described *supra* may also be used with different ultrasound modalities other than B-mode scans, including power or color Doppler modalities, and may also be used in conjunction with vibrational Doppler imaging (VDI) modalities. Therefore, reference to the details of the preferred embodiments are not intended to limit their scope, which is limited only by the scope of the claims set forth below.

The description includes the following numbered clauses detailing aspects of the invention:
1. A method for facilitating the detection of breast lesions, comprising:
   displaying an x-ray mammogram view of a breast captured in an x-ray mammogram view plane; and
   displaying near said x-ray mammogram view a plurality of two-dimensional thickslice images of the breast, each thick-slice image representing sonographic properties of a corresponding thick-slice volume of the breast substantially parallel to the x-ray mammogram view plane, said plurality of thick-slice images thereby providing additional information regarding said thick-slice volumes of the breast to a viewer of the x-ray mammogram in a way that facilitates ease of comparison therewith.
2. The method of clause 1, each thick-slice volume being slab-like in shape with substantially planar upper and lower surfaces parallel to said x-ray mammogram view plane, each thickslice volume having a thickness as measured between said upper and lower surfaces between about 2 mm and 20 mm.
3. The method of clause 2, each thick-slice image comprising a statistical combination of at least two individual component ultrasound slices derived from raw ultrasound scans of the breast, said component ultrasound slices being substantially parallel to said x-ray mammogram view plane.
4. The method of clause 3, said raw ultrasound scans being acquired by an ultrasound probe oriented substantially parallel to said x-ray mammogram view plane, said component ultrasound slices corresponding directly to said raw ultrasound scans.
5. The method of clause 4, said raw ultrasound scans being taken in planes spaced about 0.15 mm to 0.75 mm apart, said thick-slice volumes having thicknesses between about 7 mm to 12 mm, said thick-slice images being computed from between 9 and 80 component ultrasound slices.
6. The method of clause 4, said raw ultrasound scans being taken in planes spaced less than about 0.5 mm apart.
7. The method of clause 3, said raw ultrasound scans being acquired by an ultrasound probe oriented substantially perpendicular or non-parallel to said x-ray mammogram view plane, each component ultrasound slice corresponding to a plane of voxels extracted from a three-dimensional volumetric representation of the breast computed from said raw ultrasound scans, said plane of voxels being parallel to said x-ray mammogram view plane.
8. The method of clause 7, said raw ultrasound scans being obtained while the breast is compressed in a direction substantially perpendicular to said x-ray mammogram view plane.
9. The method of clause 1, wherein said x-ray mammogram view plane is a standardized x-ray mammogram view plane, whereby a viewer accustomed to analyzing x- ray mammograms captured in said standardized x-ray mammogram view plane can quickly and readily perceive the orientation and medical significance of said thick-slice images positioned near said x-ray mammogram view.
10. An apparatus for facilitating the detection of breast lesions in an breast cancer screening environment in which an x-ray mammogram view of a breast acquired in an x-ray mammogram view plane is displayed to a viewer on an x-ray mammogram viewing device, comprising:
   a processor configured to generate a plurality of thick-slice images of the breast, each thick-slice image being computed from at least two individual ultrasound slices of the breast substantially parallel to said x-ray mammogram view plane; and
   a display device for displaying said plurality of thick-slice images to the viewer, detection of breast lesions being facilitated by back-and-forth comparisons between the x- ray mammogram view and the thick-slice images when said display device is positioned near said x-ray mammogram viewing station.
11. The apparatus of clause 10, said at least two individual ultrasound slices being mutually adjacent in a direction perpendicular to said x-ray mammogram view plane and defining a slab-like thick-slice volume of the breast having a thickness between about 2 mm and 20 mm.
12. The apparatus of clause 11, each thick-slice image being computed from at least ten (10) individual ultrasound slices.
13. The apparatus of clause 12, thick-slice volume having a thickness between about 7 mm and 12 mm.
14. The apparatus of clause 12, said processor generating each thick-slice image by computing a pixelwise arithmetic average of said individual ultrasound slices.
15. The apparatus of clause 12, said processor generating each thick-slice image by computing a pixelwise statistical combination of said individual ultrasound slices selected from the group consisting of: weighted and unweighted arithmetic mean, weighted and unweighted geometric mean, weighted and unweighted reciprocal mean, weighted and unweighted exponential mean, maximum value, minimum value, standard deviation, and variance.
16. The apparatus of clause 15, each thick-slice volume having a thickness between about 5 mm and 15 mm, said thick-slice volumes collectively occupying a contiguous volume corresponding to at least 75 percent of the breast.
17. The apparatus of clause 16, said contiguous volume occupying substantially all of the breast volume, said plurality of thick-slices images being displayed simultaneously to the viewer.
18. The apparatus of clause 11, wherein said x-ray mammogram view plane is a standardized x-ray mammogram view plane, whereby a viewer accustomed to analyzing x-ray mammograms captured in said standardized x-ray mammogram view plane can quickly and readily perceive the orientation and medical significance of said thick-slice images positioned near said x-ray mammogram view.
19. The apparatus of clause 18, wherein said standardized x-ray mammogram view plane corresponds to a craniocaudal (CC) view or a mediolateral oblique (MLO) view.
20. A method, comprising:
   receiving a first plurality of substantially parallel individual ultrasound slices derived from a first set of raw ultrasound frames acquired by an ultrasound probe as it is swept across a breast;
   generating a plurality of two-dimensional thick-slices images from said individual ultrasound slices, each thick-slice image comprising an integration of at least two adjacent ones of said first plurality of individual ultrasound slices; and
   simultaneously displaying said thick-slice images on a first computerized display.
21. The method of clause 20, said at least two adjacent individual ultrasound slices defining a slab-like thick-slice volume of the breast having a thickness between about 2 mm and 20 mm.
22. The method of clause 21, further comprising:
   performing computer-assisted diagnosis (CAD) algorithms for each of said thick-slice images; and
   overlaying markers on said thick-slice images on said first computerized display at locations corresponding to suspected lesions identified by said CAD algorithms.
23. The method of clause 22, said performing CAD algorithms comprising:
   locating two-dimensional regions of interest (ROIs) in said thick-slice image according to a two-dimensional ROI location algorithm;
   segmenting two-dimensional borders of candidate lesions at each of said two-dimensional ROIs;
   extracting two-dimensional x-ray CAD features for each of said candidate lesions; and
   classifying said candidate lesions based on said two-dimensional x-ray CAD features.
24. The method of clause 23, said performing CAD algorithms further comprising: extracting two-dimensional acoustical CAD features for each of said candidate lesions; and classifying said candidate lesions based on (i) said two-dimensional x-ray CAD features, and (ii) said two-dimensional acoustical CAD features.
25. The method of clause 22, further comprising:
   locating, segmenting, and extracting two-dimensional features of one or more two-dimensional regions of interest (ROIs) from said thick-slice images;
   receiving a second plurality of substantially parallel individual ultrasound slices derived from a second set of raw ultrasound frames acquired by the ultrasound probe as it is swept across the breast, said second set of raw ultrasound frames being Doppler frames acquired while the breast is being vibrated at one or more audio frequencies;
   generating one or more vibrational Doppler images for each of said thick-slice images based on said second plurality of individual ultrasound slices;
   extracting a vibrational Doppler feature from said one or more vibrational Doppler images corresponding to each of said two-dimensional ROIs in said thick-slice images; and
   classifying each of said two-dimensional ROIs based on said two-dimensional features and said vibrational Doppler feature.
26. The method of clause 25, wherein said vibrational Doppler feature comprises a vibrational resonance feature.
27. An apparatus for facilitating acquisition of ultrasound scans of a breast of a patient by an ultrasound probe, comprising:
   a first compressive member;
   a second compressive member movably adjacent to said first compressive member to allow placement and compression of the breast therebetween, said second compressive member comprising a conformable, acoustically transparent membrane in a substantially taut state, said membrane having a first surface for contacting the breast and a second surface opposite said first surface; and
   a probe translation mechanism configured to hold the ultrasound probe against said second surface of said membrane while translating the ultrasound probe thereacross, the ultrasound probe being in acoustic communication with the breast through said membrane while being translated.
28. The apparatus of clause 27, wherein said membrane comprises a material similar to Mylar®, and wherein said first compressive member comprises a substantially rigid material.
29. The apparatus of clause 27, said apparatus having (i) an open configuration in which said membrane is sufficiently distant from said first compressive member such that the breast may be placed therebetween, and (ii) a compressed configuration in which the breast is compressed between the first compressive member and said membrane, the ultrasound probe scanning the breast when said apparatus is in said compressed configuration.
30. A method for facilitating breast cancer screening, comprising:
   acquiring raw ultrasound slices representing sonographic properties of a breast;
   forming a volumetric representation of said sonographic properties from said raw ultrasound slices;
   computing a two-dimensional thick-slice ultrasound image from said volumetric representation, said thick-slice ultrasound image representing said sonographic properties within a slab-like subvolume of the breast having a thickness greater than about 2 mm and less than about 20 mm;
   displaying said thick-slice ultrasound image to a user during a viewing session;
   computing a planar ultrasound image from said volumetric representation, said planar ultrasound image representing said sonographic properties along a substantially planar portion of the breast substantially nonparallel to said slab-like subvolume; and
   electronically displaying said planar ultrasound image to the user during the viewing session.
31. The method of clause 30, wherein said thick-slice ultrasound image and said planar ultrasound image are simultaneously displayed to the user near each other to facilitate back-and-forth viewing therebetween.
32. The method of clause 31, wherein said slab-like subvolume is substantially parallel to a standard x-ray mammogram view plane.
33. The method of clause 30, said slab-like subvolume being substantially parallel to a standard x-ray mammogram view plane, further comprising displaying an x-ray mammogram image of the breast taken along said standard x-ray mammogram view plane to said user during the viewing session.
34. The method of clause 33, wherein said thick-slice ultrasound image, said planar ultrasound image, and said x-ray mammogram image are simultaneously displayed to the user near each other to facilitate back-and-forth viewing thereamong.
35. The method of clause 34, wherein said x-ray mammogram is displayed as an x-ray film on a backlit display.
36. The method of clause 35, wherein said thick-slice ultrasound image is electronically displayed.
37. The method of clause 36, wherein said planar ultrasound image is displayed on a high-brightness CRT monitor positioned adjacent to said thick-slice ultrasound image.
38. The method of clause 37, wherein said thick-slice ultrasound image is displayed on an LCD monitor.
39. The method of clause 38, wherein said thick-slice ultrasound image is enlarged or reduced to have the same spatial dimensions as the x-ray mammogram image.
40. The method of clause 30, said raw ultrasound slices comprising values according to a conventional ultrasound display convention in which higher reflection readings are displayed as brighter and lower reflection readings displayed as darker, further comprising inverting said thick-slice image to an inverted ultrasound display convention in which higher reflection readings are displayed as darker and lower reflection readings displayed as brighter.
41. The method of clause 40, further comprising:
   segmenting said thick-slice image into a first region lying inside the breast and a second region lying outside the breast, said second region having bright pixels directly after said inverting; and
   resetting said second region pixels to dark values, the resulting thick-slice image thereby being more reminiscent of a conventional x-ray mammogram image in said second region.
42. A method for computing a two-dimensional thick-slice ultrasound image from a volumetric ultrasound representation of a breast, said volumetric ultrasound representation comprising voxels, said thick-slice ultrasound image comprising pixels and corresponding to a first slab-like subvolume of the breast lying between a first border plane and a second border plane thereof, comprising:
   identifying for each pixel location in the thick-slice ultrasound image a first voxel set corresponding to a voxel column in said volumetric ultrasound representation passing through that pixel location and extending from the first border plane to the second border plane;
   computing one or more statistical properties of said first voxel set; and
   computing an output value for that pixel location using said one or more statistical properties of said first voxel set.
43. The method of clause 42, said one or more statistical properties of said first voxel set incurring changes across different pixel locations in mass localities that are more significant for masses greater than a preselected size of interest and that are less significant for masses smaller than said preselected size of interest, mass lesions greater than said preselected size of interest being emphasized and mass lesions smaller than said preselected size of interest being de-emphasized in said thick-slice image.
44. The method of clause 15, said computing an output value comprising:
   computing a histogram of values of said first voxel set;
   computing at least a portion of a cumulative distribution function from said histogram;
   determining a first pixel level for which said cumulative distribution function is equal to a threshold, said threshold being a function of said preselected size of interest; and
   setting said output value equal to said first pixel level.
45. The method of clause 44, said threshold being a fixed value equal to a preselected constant K times a ratio of (i) said preselected size of interest, to (ii) a distance between said first and second border planes.
46. The method of clause 45, wherein K is between about 0.20 and about 0.45.
47. The method of clause 18, said threshold being variable for different pixel locations in said thick-slice ultrasound image, said threshold being equal to a variable multiplier K times a ratio of (i) said preselected size of interest, to (ii) a distance between said first and second border planes.
48. The method of clause 19, wherein values of K are maintained within a range of about 0.20 to about 0.45.
49. The method of clause 42, the breast further having a second slab-like subvolume immediately adjacent to the first slab-like volume, further comprising:
   identifying for each pixel location in the thick-slice ultrasound image a second voxel set corresponding to said voxel column extending from a first intermediate elevation in said first slab-like volume to a second intermediate location is said second slab-like volume, said first and second voxel sets having about the same number of voxels;
   computing said one or more statistical properties of said second voxel set;
   computing an alternative result for that pixel location using said one or more statistical properties of said second voxel set; and
   if said alternative result indicates an ultrasound echo intensity less than that indicated by said output value, resetting said output value to said alternative result.
50. The method of clause 49, said volumetric ultrasound representation being formed from scans taken during a sweep of an ultrasound probe across the breast, wherein said second slab-like volume is closer to a locus of said ultrasound probe sweep than said first slab-like volume.
51. An apparatus for facilitating analysis of a breast using an x-ray mammogram image thereof and ultrasound information derived from ultrasonic scans of the breast, comprising:
   a display device viewable by a user;
   an input device; and
   a processor coupled with said input device and with said display device, said processor generating and providing a display output to said display device, said display output comprising a superposition of the x-ray mammogram image and a two-dimensional ultrasound image derived from the ultrasound information, said superposition being characterized by an alignment amount, said processor adjusting said alignment amount according to direct manual manipulations of said input device by the user, user observation of said display output during said alignment adjustments facilitating user perception of breast structures.
52. The apparatus of clause 51, said input device being selected from the group consisting of : joystick, trackball, computer mouse, and touchscreen.
53. The apparatus of clause 51, said ultrasound image being a thick-slice ultrasound image representing sonographic properties of a slab-like region within the breast occupying less than the entire breast volume.
54. The apparatus of clause 53, said x-ray mammogram image corresponding to a standard x-ray mammogram view plane, said slab-like region being substantially parallel to said standard x-ray mammogram view plane.
55. The apparatus of clause 54, said processor dynamically varying a thickness of the slab-like region responsive to a first user input.
56. The apparatus of clause 54, said processor dynamically varying an elevation of the slab-like volume responsive to a second user input.
57. The apparatus of clause 54, said superposition being generated according to a predetermined pixelwise mixing algorithm.
58. The apparatus of clause 57, said mixing algorithm including a mixing factor that is dynamically user adjustable according to a third user input, said user adjustment of said mixing factor also facilitating user perception of breast structures.

## Claims

1. A method for processing a three-dimensional data volume of a sonographic property of a breast, said three-dimensional data volume having been acquired from ultrasonic scans of the breast while the breast was compressed along a plane of compression, the method comprising:
generating a plurality of two-dimensional thick-slice images (134, 3736, 5102) from the three-dimensional data volume, each thick-slice image (134, 3736, 5102) representing said sonographic property of the breast within a slab-like subvolume of the breast substantially parallel to said plane of compression (FIG. 9A: A, B, C, D, E; FIG. 49: 4902, 4904, 4906), said slab-like subvolume having a thickness between about 2 mm and 20 mm, wherein said generating comprises integrating the sonographic properties of at least two individual ultrasound slices lying within the slab-like subvolume; and
displaying said plurality of thick-slice images on a user display (126).

2. The method of claim 1, said slab-like subvolume (4902, 4904, 4906) encompassing N individual ultrasound slices V_{xz}(y₁)), ... V_{xz}(y_{N}), N ≥ 2, wherein said two-dimensional thickslice image is computed from said N individual ultrasound slices V_{xz}(y₁), ... V_{xz}(y_{N}) according to a neighborhood-dependent algorithm designed to emphasize lesions of a predetermined size range.

3. The method of any of the preceding claims, wherein said plane of compression is a standard x-ray mammogram view plane.

4. The method of any of the preceding claims, wherein said thick-slice images correspond to respective slab-like subvolumes that collectively occupy substantially all of the breast volume, and wherein said plurality of thick-slice images are displayed simultaneously on said user display.

5. The method of any of the preceding claims, further comprising:
receiving an x-ray mammogram image (120, 122) of the breast corresponding to said standard x-ray mammogram view plane; and
displaying said x-ray mammogram image (120, 122) on said user display (109, 126) concurrently with said plurality of thick-slice images.

6. A data processing apparatus (106, 3706) programmed and configured to carry out a method for processing a three-dimensional data volume of a sonographic property of a breast according to any of claims 1-5.

7. A computer program comprising processor-implementable instructions for programming a programmable data processing apparatus to carry out a method for processing a three-dimensional data volume of a sonographic property of a breast according to any of claims 1-5.

8. A data processing apparatus (106, 3706) arranged to process a three-dimensional data volume of a sonographic property of a breast, said three-dimensional data volume having been acquired from ultrasonic scans of the breast while the breast was compressed along a plane of compression,
wherein the data processing apparatus is operable to generate image data corresponding to a plurality of two-dimensional thick-slice images (134, 3736, 5102) from the three-dimensional data volume, each thick-slice image (134, 3736, 5102) representing said sonographic property of the breast within a slab-like subvolume of the breast substantially parallel to said plane of compression (FIG. 9A: A, B, C, D, E; FIG. 49: 4902, 4904, 4906), said slab-like subvolume having a thickness between about 2 mm and 20 mm,
wherein the data processing apparatus is operable to generate the image data by integrating the sonographic properties of at least two individual ultrasound slices lying within the slab-like subvolume, and
wherein the data processing apparatus is operable to cause said plurality of thick-slice images to be displayed on a user display (126).

9. The data processing apparatus of claim 8, said slab-like subvolume (4902, 4904, 4906) encompassing N individual ultrasound slices V_{xz}(y₁), ... V_{xz}(y_{N}), N ≥ 2, wherein the data processing apparatus is operable to compute said two-dimensional thick-slice images from said N individual ultrasound slices V_{xz}(yₗ), ... V_{xz}(y_{N}) according to a neighborhood-dependent algorithm designed to emphasize lesions of a predetermined size range.

10. The data processing apparatus of claim any of claims 8-9, wherein said plane of compression is a standard x-ray mammogram view plane.

11. The data processing apparatus of any of claims 8-10, wherein said thick-slice images correspond to respective slab-like subvolumes that collectively occupy substantially all of the breast volume, and wherein said data processing apparatus is operable to cause said plurality of thick-slice images to be displayed simultaneously on said user display.

12. The data processing apparatus of any of claims 8-11, wherein said data processing apparatus is further operable to:
receive an x-ray mammogram image (120, 122) of the breast corresponding to said standard x-ray mammogram view plane; and
cause said x-ray mammogram image (120, 122) to be displayed on said user display (109, 126) concurrently with said plurality of thick-slice images.

13. A computer program comprising processor-implementable instructions for programming a programmable data processing apparatus to be a data processing apparatus according to any of claims 8 to 12.
